(19) Europäisches Patentamt
European Patent Office
Office européen des brevets

(11) EP 3 900 739 A1

(12) EUROPEAN PATENT APPLICATION

(43) Date of publication:
**27.10.2021 Bulletin 2021/43**

(21) Application number: **20170605.8**

(22) Date of filing: **21.04.2020**

(51) Int Cl.:
*A61K 39/09* (2006.01) *A61P 11/00* (2006.01)
*A61P 31/04* (2006.01) *C07K 14/315* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Max-Planck-Gesellschaft zur Förderung der Wissenschaften e.V.
80539 München (DE)**

(72) Inventor: **The designation of the inventor has not yet been filed**

(74) Representative: **Arth, Hans-Lothar
ABK Patent Attorneys
Jasminweg 9
14052 Berlin (DE)**

# (54) SYNTHETIC STREPTOCOCCUS PNEUMONIAE SACCHARIDE CONJUGATES TO CONSERVED MEMBRANE PROTEIN

(57) The present invention relates to conjugates of synthetic *Streptococcus pneumoniae* oligosaccharides with pneumococcal carrier proteins such as detoxified pneumolysin or pneumococcal surface protein A, and to immunogenic compositions comprising the same. The disclosed conjugates are useful for the prevention and/or treatment of diseases caused by *Streptococcus pneumoniae,* including *Streptococcus pneumoniae* serotypes 2 and 3.

**Figure 5**

a)

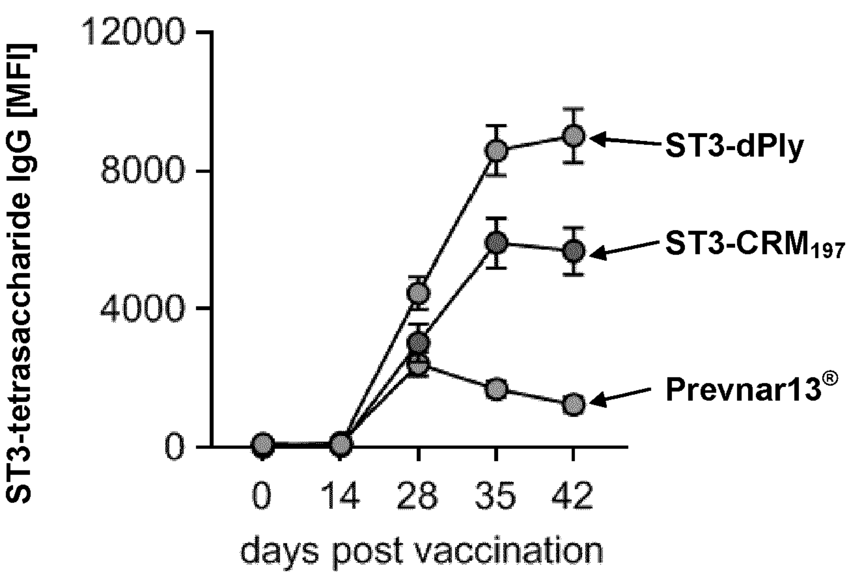


EP 3 900 739 A1

## Description

The present invention relates to conjugates of synthetic *Streptococcus pneumoniae* oligosaccharides with pneumococcal carrier proteins such as detoxified pneumolysin or pneumococcal surface protein A, and to immunogenic compositions comprising the same. The disclosed conjugates are useful for the prevention and/or treatment of diseases caused by *Streptococcus pneumoniae,* including *Streptococcus pneumoniae* serotypes 2 and 3.

## Background of the invention

The Gram-positive bacterium *Streptococcus pneumoniae* is one of the main pathogens and causes severe invasive diseases like meningitis, pneumonia or bacteremia. Particularly infants, the elderly and immunocompromised patients are at high risk towards pneumococcal infections. About more than 90 serotypes of *Streptococcus pneumoniae* have been identified throughout the world, with a small number of these serotypes accounting for most diseases. The serotypes are identified by their different core capsular polysaccharide (CPS) structure. The CPS consists of polymers of repeating oligosaccharide units, which represent the epitope of the bacteria. These capsular polysaccharides or parts of them are often immunogenic and constitute potential candidates for pneumococcal vaccines.

The resistance of *Streptococcus pneumoniae* to antibiotics is a rapidly increasing problem. Thus, pneumococcal vaccines providing protection against pneumococcal infections are becoming increasingly important. Two classes of pneumococcal vaccines are currently available, one based on polysaccharides and the other based on polysaccharides conjugated to a carrier protein. The polysaccharides are thymus-independent type 2 antigens and thus induce low-affinity antibodies. In addition, they evoke no B-cell memory. Pneumococcal conjugate vaccines can circumvent these disadvantages with an increased serotype-specific antibody response to capsular polysaccharides. The conjugate vaccine PCV-13 contains immunogenic conjugates comprising the purified polysaccharides of 13 different *S. pneumoniae* serotypes covalently linked to a protein, such as $CRM_{197}$.

At least 98 S. pneumoniae serotypes can be distinguished based on their CPS. Currently available CPS-based pneumococcal vaccines contain the serotypes most frequently associated with invasive pneumococcal diseases (IPDs). Although the licensed 23-valent polysaccharide vaccine (Pneumovax 23®) is not effective in younger children, the conjugate vaccines Prevnar13® and Synflorix® cover thirteen and ten serotypes respectively and are highly successful in all age groups. Nevertheless, the plasticity of the pneumococcal genome means that the pathogen has the potential to adapt to the selective pressure of vaccines. The serotype replacement due to vaccination and regional differences in dominant serotypes necessitate the expansion of existing vaccines to include additional serotypes. New strains, e.g., serotype 19A and 22F, arise and replace previous colonizing strains under this selective pressure.

*Streptococcus pneumoniae* type 3 (ST3) is part of the current pneumococcal vaccines. The capsular polysaccharide of ST3 consists of [→3)-β-D-Glc*p*A-(1→4)-β-D-Glc*p*-(1→] repeating units. Immunization experiments with ST3 oligosaccharides conjugated to $CRM_{197}$ showed that increasing IgG antibody titers were found with increasing chain length (from monosaccharide to tetrasaccharide) and no influence on the immunogenicity by the oligosaccharide/$CRM_{197}$ ratio (see Benaissa-Trouw et al., Infection and Immunity, 2001, 69, 4698 - 4701).

*S. pneumoniae* serotype 3 (ST3) is an important cause of invasive pneumococcal disease, particularly pneumonia, in both children and adults. Serotype 3 conjugate was added to the formulation of 13-valent pneumococcal vaccine (PCV13) and is now included in the routine immunization schedule. However, this particular serotype remains a prominent cause of the invasive pneumococcal disease (IPD) in all age groups in most countries using PCV13. Many studies show no change in the incidence rate of serotype 3 after the introduction of PCV13 (Moore MR, et al. 2015, The Lancet. Infectious diseases 15(3): 301-309). *S. pneumoniae* serotype 3 produces a very thick mucoid capsule which protects the bacteria from phagocytosis, inhibits opsonization by complement, helps to escape the neutrophil extracellular traps and prevents macrophage killing. Lack of clinical efficacy against serotype 3 after pneumococcal conjugate vaccination may be a result of reduced induction of immune memory. It was found that the levels of pre-existing ST3-specific antibody are negatively correlated with the B cell memory response to a booster dose of PCV13 containing ST3 glycoconjugate. Therefore, improvement by alternative approaches is needed to advance vaccines for this highly invasive pneumococcal serotype.

Synthetic oligosaccharides based on ST3 capsular polysaccharide repeating units have been shown to protect mice against lethal systemic challenge with ST3 pneumococci. A highly immunogenic tetrasaccharide glycotope based on the disaccharide repeating unit of *S. pneumoniae* serotype 3 was synthesized (below). The semi-synthetic oligosaccharide-based glycoconjugate vaccine candidate shows an immunoprotective effect against experimental pneumonia caused by transnasal infection with ST3 strain PN36. However, the vaccine needs further enhancement to achieve long-lived immune memory (Parameswarappa SG, et al. 2016, Cell chemical biology 23(11):1407-1416).

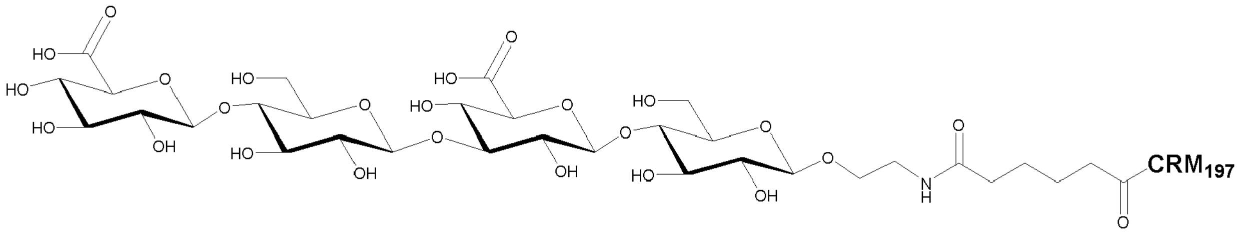

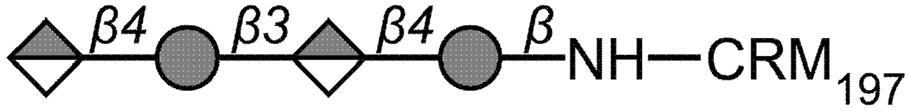

Recent studies show that *S. pneumoniae* type 2, a serotype not covered by the currently commercially available vaccines, has emerged in the SAARC (The South Asian Association for Regional Cooperation) countries. *S. pneumoniae* type 2 is responsible for 4.54% of invasive pneumococcal disease in children in Nepal and 8.9% of invasive pneumococcal disease in children in Banglagesh (hospital based study) (Distribution of Serotypes, Vaccine Coverage, and Antimicrobial Susceptibility Pattern of Streptococcus Pneumoniae in Children Living in SAARC Countries: A Systematic Review Jaiswal, N. et al. PLOS ONE 2014, 9). Further population based studies on pneumococcal serotype distribution attest that *S. pneumoniae* type 2 is the most prevalent serotype in Bangladesh causing 12.2% of invasive pneumococcal diseases (Current Trend in Pneumococcal Serotype Distribution in Asia, Le C. et al. J Vaccines Vaccin 2011). Hence, there is a high need to provide a vaccine protecting against *S. pneumoniae* type 2, a serotype not covered by the currently commercialized vaccines.

Replacement of specific serotypes included in the vaccines by the serotypes not included occurs frequently and is called **serotype replacement.** Development of non-vaccine serotypes is a major challenge in controlling of pneumococcal diseases. This problem has been a major reason for intensive research in the direction of developing a 'universal' pneumococcal vaccine immunogenic in all age groups and broadly cross-protective against all serotypes. Several studies reported a protein-based serotype-independent vaccine to prevent pneumococcal infections. Targeting multiple pneumococcal membrane proteins based on their roles in bacterial pathogenicity and physiology seems to be a promising approach to achieve additive protection against pneumococci in mice. Indeed, membrane proteins originating from the same pathogen can induce an additional response, so-called "**additional valency**". Among them, pneumolysin and PspA are conserved proteins among all *S.pneumoniae* serotypes that could broaden the spectrum of protection of the pneumococcal vaccine.

The nature of the carrier protein is critical in defining the magnitude and quality of vaccine-induced immune responses, and the duration of protection. Different aspects, such as competition with anti-carrier antibodies elicited by related vaccination against tetanus and diphtheria, an overload of carrier protein or carrier-mediated epitope suppression, may influence both T- and B- cell responses to glycoconjugate vaccines. Additionally, differences in extent and persistence of protective antibodies triggered by primary vaccination with bacterial polysaccharides conjugated to various carrier proteins have been observed in infants.

**It is the objective of the present invention to provide conjugates of synthetic *Streptococcus pneumoniae* oligosaccharides with pneumococcal carrier proteins such as detoxified pneumolysin or pneumococcal surface protein A, and immunogenic compositions comprising the same.**

The objective of the present invention is solved by the teaching of the independent claims. Further advantageous features, aspects and details of the invention are evident from the dependent claims, the description, the figures, and the examples of the present application.

The disclosed conjugates are useful for the prevention and/or treatment of diseases caused by *Streptococcus pneumoniae,* including *Streptococcus pneumoniae* serotype 2 and 3. Moreover, the inventive conjugates can be useful to raise cross-reactive immunity through induction of systemic and local antibodies specific for the pneumococcal virulence factors used as carrier proteins.

## Brief description of the invention

The present invention is directed to a conjugate of a synthetic *Streptococcus pneumoniae* oligosaccharide with a carrier protein selected from detoxified pneumolysin (dPly) or an immunogenic fragment thereof, and pneumococcal surface protein A, or an immunogenic fragment thereof.

In accordance with one embodiment of the invention, the synthetic *Streptococcus pneumoniae* oligosaccharide is selected from the group of serotypes comprising serotype 1, 2, 3, 4, 5, 8.

In accordance with one particular embodiment of the invention, the synthetic *Streptococcus pneumoniae* oligosaccharide is of *Streptococcus pneumoniae* serotype 3.

In accordance with one further particular embodiment of the invention, the synthetic *Streptococcus pneumoniae* oligosaccharide is of *Streptococcus pneumoniae* serotype 3 and the conjugate has the general formula (**I**)

$$[H-(C)_{c3}-(D-C)_{c2}-(D)_{c1}-O-L^3-NH-W^3]_{m3}\text{-carrier protein} \qquad (\mathbf{I})$$

wherein

C represents: 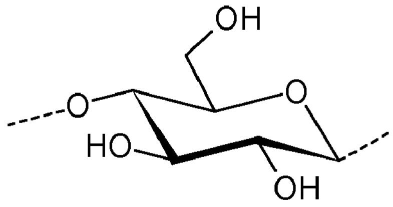

D represents: 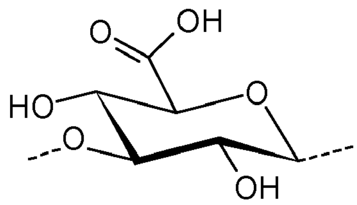

c2 is an integer selected from 1, 2, 3, 4, 5, 6, 7, 8, 9 and 10;
c1 and c3 represent independently of each other an integer which is 0 or 1 ;
$L^3$ represents a linker;
m3 is comprised between about 2 and about 18;
$-W^3-$ is selected from:

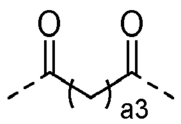 , 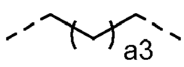 , and 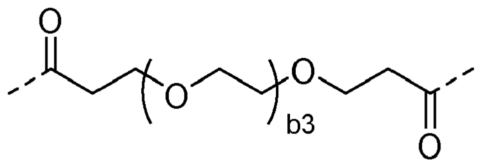 ;

a3 represents an integer from 1 to 10;
b3 represents an integer from 1 to 4; and
the carrier protein has the meaning as defined in claim 1.

In accordance with another particular embodiment of the invention, the synthetic *Streptococcus pneumoniae* oligosaccharide is of *Streptococcus pneumoniae* serotype 2.

In accordance with another particular embodiment of the invention, the synthetic *Streptococcus pneumoniae* oligosaccharide is of *Streptococcus pneumoniae* serotype 2, and the conjugate has the general formula (**II**)

$$[B^*-A_{y+3}-A_{y+2}-A_{y+1}-A_y-O-L^2-NH-W^2]_{m2}\text{-carrier protein} \qquad (\mathbf{II})$$

wherein y is an integer selected from 1, 2, 3 and 4,

$A_1 = A_5 =$ 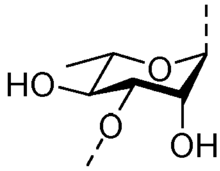 ,

$A_2 = A_6 =$ 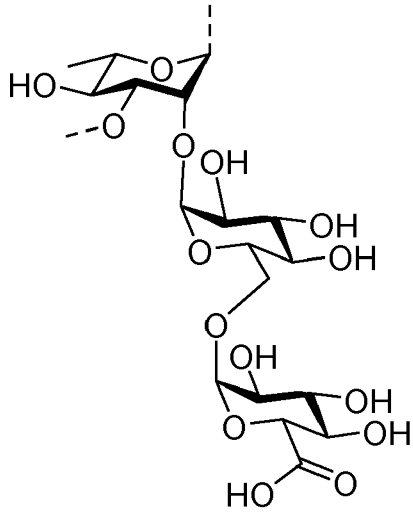 ,

$A_3 = A_7 =$ 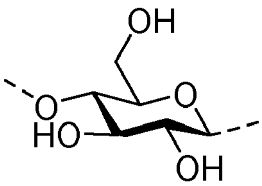 ;

$A_4 =$ 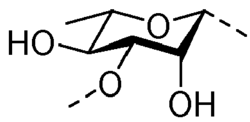 ;

B*- represents H-, H-$A_y$-, H-$A_{y+1}$-$A_y$-, H-$A_{y+2}$-$A_{y+1}$-$A_y$- or H-$A_{y+3}$-$A_{y+2}$-$A_{y+1}$-$A_y$-;
$L^2$ represents a linker;
m2 is comprised between about 2 and about 18,
-$W^2$- is selected from:

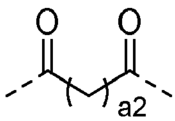 , 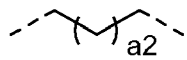 , and 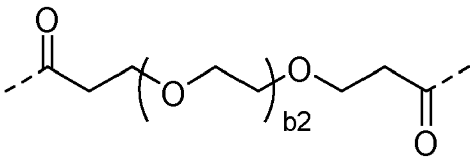 ;

a2 represents an integer from 1 to 10;
b2 represents an integer from 1 to 4; and
the carrier protein has the meaning as defined in claim 1.

In accordance with another embodiment, the present invention is directed to an immunogenic composition comprising one or more conjugates as described above.

In accordance with another particular embodiment of the invention, the immunogenic composition comprises (i) a conjugate of a synthetic *Streptococcus pneumoniae* oligosaccharide with detoxified pneumolysin or an immunogenic fragment thereof, and (ii) a conjugate of a synthetic *Streptococcus pneumoniae* oligosaccharide with pneumococcal surface protein A or an immunogenic fragment thereof.

In accordance with a further particular embodiment of the invention, the immunogenic composition comprises a conjugate of *Streptococcus pneumoniae* serotype 3 as described above (ST3), and /
a mixture consisting of capsular polysaccharides from *Streptococcus pneumoniae* serotypes 1, 4, 5, 6A, 6B, 7F, 9V, 14, 18C, 19A, 19F and 23F individually conjugated to $CRM_{197}$ carrier protein. PREVNAR 13 - ST3

In accordance with a more particular embodiment of the invention, the immunogenic composition described above comprises one or more conjugates are selected from a conjugate of a synthetic *Streptococcus pneumoniae* serotype 1 oligosaccharide, a conjugate of a synthetic *Streptococcus pneumoniae* serotype 2 oligosaccharide, a conjugate of a synthetic *Streptococcus pneumoniae* serotype 3 oligosaccharide, a conjugate of a synthetic *Streptococcus pneumoniae* serotype 4 oligosaccharide, a conjugate of a synthetic *Streptococcus pneumoniae* serotype 5 oligosaccharide, a conjugate of a synthetic *Streptococcus pneumoniae* serotype 8 oligosaccharide.

In accordance with a more particular embodiment of the invention, the immunogenic composition described above further comprises at least one unconjugated *Streptococcus pneumoniae* protein selected from detoxified pneumolysin or an immunogenic fragment thereof, and pneumococcal surface protein A, or an immunogenic fragment thereof.

One embodiment of the invention also refers to the conjugate as described above for use or the immunogenic composition as described above for use in raising a protective immune response in a human and/or animal host.

One particular embodiment of the invention also refers to the conjugate as described above for use or the immunogenic composition as described above for use in the prevention and/or treatment of a disease caused by *Streptococcus pneumoniae.*

Another particular embodiment of the invention further refers to the conjugate as described above for use or the immunogenic composition as described above for use in the prevention and/or treatment of a disease caused by *Streptococcus pneumoniae* serotype 2 and/or serotype 3.

The diseases caused by *Streptococcus pneumoniae,* or in particular by *Streptococcus pneumoniae* serotype 2 and/or serotype 3, include pneumonia, meningitis, otitis media, bacteremia and acute exacerbation of chronic bronchitis, invasive pneumococcal disease, exacerbation of chronic obstructive pulmonary disease, sinusitis, arthritis and conjunctivitis.

A further embodiment of the invention is directed to a vaccine comprising the conjugate as described above and/or the immunogenic composition as described above, together with at least one pharmaceutically acceptable adjuvant and/or excipient.

Said vaccine includes a nasal formulation, preferably nasal drops, nasal powder, a nasal gel or a nasal spray.

In a further embodiment, said vaccine comprises the adjuvant Cholera Toxin subunit B or the adjuvant Aluminum hydroxide.

## Description of the invention

The present invention refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof.

In other words, the present invention refers to a conjugate of a synthetic *Streptococcus pneumoniae* oligosaccharide with a **pneumococcal protein** selected from detoxified pneumolysin or an immunogenic fragment thereof, and pneumococcal surface protein A, or an immunogenic fragment thereof.

Slightly reworded, the present invention refers to a conjugate of a synthetic *Streptococcus pneumoniae* oligosaccharide with a **pneumococcal virulence factor** selected from detoxified pneumolysin or an immunogenic fragment thereof, and pneumococcal surface protein A, or an immunogenic fragment thereof.

Further slightly reworded, the present invention refers to a conjugate of a synthetic *Streptococcus pneumoniae* oligosaccharide with a **pneumococcal conserved protein** selected from detoxified pneumolysin or an immunogenic fragment thereof, and pneumococcal surface protein A, or an immunogenic fragment thereof.

More in particular, the present invention refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the synthetic Streptococcus pneumoniae oligosaccharide is selected from the group of serotypes comprising serotype 1, 2, 3, 4, 5, 8.

Still more in particular, the present invention refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the synthetic *Streptococcus pneumoniae* oligosaccharide is of *Streptococcus pneumoniae* serotypes 3.

Still more in particular, the present invention refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the synthetic *Streptococcus pneumoniae* oligosaccharide is of *Streptococcus pneumoniae* serotypes 3, and wherein the conjugate has the general formula (I)

$$\mathbf{[H\text{-}(C)_{c3}\text{-}(D\text{-}C)_{c2}\text{-}(D)_{c1}\text{-}O\text{-}L^3\text{-}NH\text{-}W^3]_{m3}\text{-}carrier\ protein} \qquad \mathbf{(I)}$$

wherein

C represents: 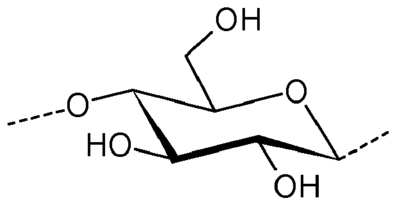

D represents: 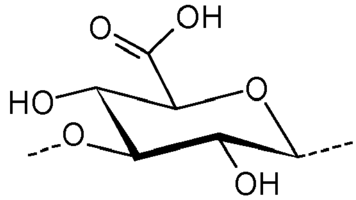

c2 is an integer selected from 1, 2, 3, 4, 5, 6, 7, 8, 9 and 10;
c1 and c3 represent independently of each other an integer which is 0 or 1;
$L^3$ represents a linker;
m3 is comprised between about 2 and about 18;
$-W^3-$ is selected from:

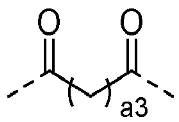 , 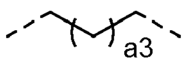 , and 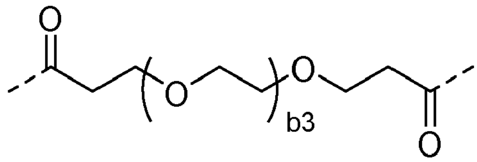 ;

a3 represents an integer from 1 to 10;
b3 represents an integer from 1 to 4; and
carrier protein is selected from detoxified pneumolysin or an immunogenic fragment thereof, and pneumococcal surface protein A, or an immunogenic fragment thereof.

A particular embodiment of the present invention refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the synthetic *Streptococcus pneumoniae* oligosaccharide is of *Streptococcus pneumoniae* serotypes 2.

A more particular embodiment of the present invention refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the synthetic *Streptococcus pneumoniae* oligosaccharide is of *Streptococcus pneumoniae* serotypes 2, and wherein the conjugate has the general formula (**II**)

$$[B^*\text{-}A_{y+3}\text{-}A_{y+2}\text{-}A_{y+1}\text{-}A_y\text{-O-}L^2\text{-NH-}W^2]_{m2}\text{-carrier protein} \qquad \textbf{(II)}$$

wherein

y is an integer selected from 1, 2, 3 and 4,

A3 = A7 = 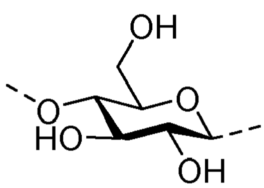 ; A4 = 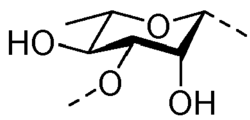 ;

$A_1 = A_5 =$ 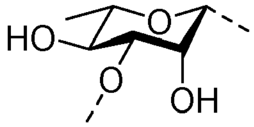 ; $A_2 = A_6 =$ 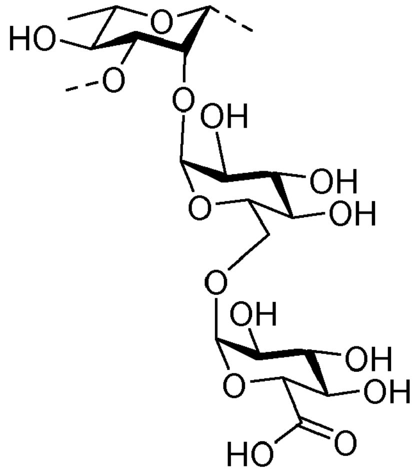 ,

B*- represents H-, H-$A_y$-, H-$A_{y+1}$-$A_y$-, H-$A_{y+2}$-$A_{y+1}$-$A_y$- or H-$A_{y+3}$-$A_{y+2}$-$A_{y+1}$-$A_y$-;
$L^2$ represents a linker;
m2 is comprised between about 2 and about 18,
-$W^2$- is selected from:

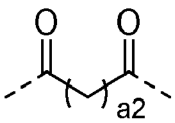 , 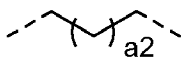 , and 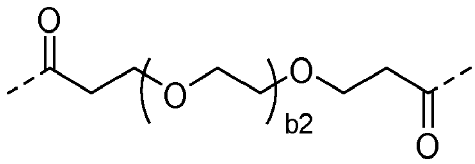 ;

a2 represents an integer from 1 to 10;
b2 represents an integer from 1 to 4; and
carrier protein is selected from detoxified pneumolysin or an immunogenic fragment thereof, and pneumococcal surface protein A, or an immunogenic fragment thereof.

Saccharides are known by the person skilled in the art as generally TI-2 (T cell independent-2) antigens and poor immunogens. TI-2 antigens are antigens, which are recognized only by mature B cells through the cross linking of surface exposed immunoglobulin receptors. Without T cell help, no immunological memory is generated and neither isotype switching from IgM to other IgG subclasses, nor B cells affinity maturation occurs. Moreover, saccharides are known poor immunogens in humans due to the structural homology to human glycolipids and glycoproteins. Due to their poor immunogenic properties, saccharides manifest poor ability to produce both antibody production by B cells, as well as the formation of memory cells, features which are essential for the production of potent vaccines.

Therefore, to produce a potent saccharide-based vaccine, the saccharides are conjugated to a carrier protein to provide glycoconjugates, which present increased immunogenicity in comparison with the saccharide.

As used herein, the term "**conjugate**" or "**glycoconjugate**" refers to a conjugate between a protein, i.e. a carrier protein, and an antigen, i.e. a synthetic *Streptococcus pneumoniae* oligosaccharide.

The present invention refers to conjugates, also referred to as glycoconjugate, obtained by reacting a synthetic *Streptococcus pneumoniae* oligosaccharide with a carrier protein selected from detoxified pneumolysin or an immunogenic fragment thereof, and pneumococcal surface protein A, or an immunogenic fragment thereof. Said glycoconjugates proved to be efficient as a vaccine for immunization against diseases associated with *Streptococcus pneumoniae.* Hence, said glycoconjugates are useful for raising a protective immune response in a human and/or animal host and therefore are useful for the prevention and/or treatment of diseases associated with *Streptococcus pneumoniae.*

The term "**carrier protein**" as used herein refers to a protein covalently attached to an antigen (e.g. saccharide antigen) to create a conjugate (e.g. glycoconjugate or bioconjugate). A carrier protein activates T-cell mediated immunity in relation to the antigen to which it is conjugated. A "**carrier protein**" is preferably non-toxic and non-reactogenic and obtainable in sufficient amount and purity. A carrier protein is amenable to standard conjugation procedures. Carrier proteins known in the prior art include inactivated bacterial toxins such as tetanus toxoid (TT), pertussis toxoid (PT), cholera toxoid (CT), *E. coli* LT, *E. coli* ST, and exotoxin A from *Pseudomonas aeruginosa.* Bacterial outer membrane proteins such as outer membrane complex c (OMPC), porins, transferrin binding proteins, detoxified **pneumolysin,**

**pneumococcal surface protein A (PspA)**, pneumococcal adhesin protein (PsaA), C5a peptidase from Group A or Group B streptococcus, *Haemophilus influenzae* protein D (PD), or a member of the polyhistidine triad family (Pht) proteins, fragments or fusion proteins thereof can also be used. Other proteins, such as ovalbumin, keyhole limpet hemocyanin (KLH), bovine serum albumin (BSA) or purified protein derivative of tuberculin (PPD) can also be used as carrier proteins.

As used herein, the term "**immunogenic fragment**" is a portion of an antigen smaller than the whole that is capable of eliciting a humoral and/or cellular immune response in a host animal, e.g. human, specific for that fragment. Fragments of a protein can be produced using techniques known in the art, e.g. recombinantly, by proteolytic digestion, or by chemical synthesis. Internal or terminal fragments of a polypeptide can be generated by removing one or more nucleotides from one end (for a terminal fragment) or both ends (for an internal fragment) of a nucleic acid which encodes the polypeptide. Typically, fragments comprise at least 10, 20, 30, 40 or 50 contiguous amino acids of the full length sequence. Fragments may be readily modified by adding or removing 1, 2, 3, 4, 5, 6, 7, 8, 9, 10, 20, 30, 40 or 50 amino acids from either or both of the N and C termini.

As used herein the term protein or fragment thereof also include "**variant**" of a protein or of a fragment thereof, and refers to a protein or fragment that shares a certain amino acid sequence identity with the reference protein or fragment upon alignment by a method known in the art. A variant of a protein or of a fragment thereof can include a substitution, insertion, deletion, frameshift or rearrangement in another protein. In some embodiments variants share at least 70%, 80%, 85%, 90%, 95% or 99% sequence identity with the native protein or with the fragment thereof.

Recitation of any protein provided herein encompasses a functional variant of the protein. The term "functional variant" of a protein refers to a variant of the protein that retains the ability to be integrated in or specifically targeted to the giant unilamellar vesicle.

The term "**mutated**" is used herein to mean a molecule which has undergone deletion, addition or substitution of one or more amino acids using known techniques for site directed mutagenesis or any other conventional method.

As used herein, the term "**deletion**" is the removal of one or more amino acid residues from the protein sequence. Typically, no more than about from 1 to 6 residues (e.g. 1 to 4 residues) are deleted at any one site within the protein molecule.

As used herein, the term "**addition**" or "**insertion**" is the addition of one or more non-native amino acid residues in the protein sequence. Typically, no more than about from 1 to 10 residues, (e.g. 1 to 7 residues, 1 to 6 residues, or 1 to 4 residues) are inserted at any one site within the protein molecule

As used herein, the term "**conservative amino acid substitution**" involves substitution of a native amino acid residue with a non-native residue such that there is little or no effect on the size, polarity, charge, hydrophobicity, or hydrophilicity of the amino acid residue at that position, and without resulting in decreased immunogenicity. For example, these may be substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. Conservative amino acid modifications to the sequence of a polypeptide (and the corresponding modifications to the encoding nucleotides) may produce polypeptides having functional and chemical characteristics similar to those of a parental polypeptide.

The diphtheria toxoid **$CRM_{197}$** which is a non-toxic variant (i.e. toxoid) of diphtheria toxin isolated from cultures of *Corynebacterium diphteria strain* C7 (β197) grown on casamino acids and yeast extract-based medium, may also be used as carrier protein. $CRM_{197}$ is purified through ultra-filtration, ammonium sulfate precipitation, and ion-exchange chromatography. Alternatively, $CRM_{197}$ is prepared recombinantly in accordance with U.S. Patent No. 5,614,382.

The term "**conserved protein**" refer to proteins having identical or similar sequences in nucleic acids (DNA and RNA) or proteins across species (orthologous sequences), or within a genome (paralogous sequences). Conservation indicates that a sequence has been maintained by natural selection.

Virulence factors of *S. pneumoniae*

In an embodiment of the present invention, the carrier protein is detoxified pneumolysin (dPly). Pneumolysin, Uniprot ID: Q04IN8, is a 53 kDa protein that belongs to the family of cholesterol-dependent cytolysin. Pneumolysin is not actively secreted from the bacterium as it lacks a typical signal secretion leader sequence but escapes from the cell by either autolysis or the action of lytic antibiotics. Pneumolysin forms pores in the cell membrane by oligomerization and conformational change in the structure. The pores formed can be up to 350 Å in diameter with each pore up to 50 pneumolysin monomers. The formation of pores results in a cell membrane disintegration which helps the bacteria to spread in the body as well as increase the host cell death and disease manifestation. Pneumolysin is a relatively conserved protein across all serotypes of *S. pneumoniae.* However, at least 16 different naturally-occurring variants of pneumolysin in specific strains of serotypes 1, and 8 pneumococci have been identified.

Pneumolysin is a multifunctional toxin with distinct activities such as complement activation, lysis of red blood cells (haemolysis) causing haemoglobin release, production of immune regulatory molecules, including cytokines as

TNF$\alpha$, IL-1, and IL-6, influencing neutrophil activity and neutrophil extracellular traps formation; favour of bacteria spread in the blood, influence of cell-signalling, cytoskeletal rearrangement and DNA damage induction; colonization of the host; causing endothelial hyper-permeability (pulmonary permeability edema), a major complication of pneumonia.

The evidence from animal infection studies points clearly to an integral role of pneumolysin in invasive pneumococcal diseases. Neutralization of the toxin seems to be a potentially valuable approach to treat pneumococcal diseases as well as an exciting vaccine candidate.

Detoxified pneumolysin (dPly) refers to a pneumolysin variant or mutant exhibiting significantly impaired lytic activity on human cells, such as red blood cells, so that it is not toxic anymore for human or other animals.

Detoxification of pneumolysin can be conducted by chemical means, e.g., subject to formalin or glutaraldehyde treatment or a combination of both. Such methods are known in the art for various toxins. Alternatively, pneumolysin can be genetically detoxified. Thus, the invention encompasses variants of pneumococcal proteins which may be, for example, mutated proteins. For example, a mutant or mutated pneumolysin protein may be altered so that it is biologically inactive, i.e. not toxic, whilst still maintaining its immunogenic epitopes.

Mutant detoxified pneumolysin proteins suitable for the embodiments of the present invention includes PlyA370G (Ala370 $\rightarrow$ Gly), PlyA370E (Ala370 $\rightarrow$ Glu), PlyA406G (Ala406 $\rightarrow$ Gly), PlyA406E (Ala406 $\rightarrow$ Glu), PlyW433G (Trp433 $\rightarrow$ Gly), PlyW433E (Trp433 $\rightarrow$ Glu), PlyW433F (Trp433 $\rightarrow$ Phe), PlyL460G (Leu460 $\rightarrow$ Gly), and PlyL460E (Leu460 $\rightarrow$ Glu) (Taylor et al., PlosOne 2013, 8(4), e61300), PlyC428G (Cys428 $\rightarrow$ Gly), PlyC428S (Cys428 $\rightarrow$Ser), PlyE434D (Glu434 $\rightarrow$ Asp), Trp435 $\rightarrow$ Phe (WO1990006951 A1).

As used herein, it is understood that the term "**Ply**" encompasses **mutated pneumolysin** and **detoxified pneumolysin** (**dPly**), which are suitable for medical use (i.e., non toxic).

**Pneumococcal surface protein A** - **PspA**

PspA is described to be an important pneumococcal virulence factor for inhibiting complement deposition and for protecting pneumococci from killing by apolactoferrin. This cross-reactive protein is widely known to be immunogenic and protective and is present in all pneumococcal strains.

PspA is composed of four different distinct regions (1) the C-terminal anchoring the protein to the pneumococcal surface, (2) a stretch of ten highly conserved 20-aminoacid repeats, (3) a proline-rich region which acts as a tether and allows greater flexibility and movement of the amino-terminus, and (4) a highly charged amino-terminus. Based on amino acid sequence ahead of the proline-rich region, PspA is classified into three families and six clades (Family 1, clades 1 and 2; Family 2, clades 3, 4 and 5; and the rarely isolated Family 3, clade 6). The interaction of PspA with the iron-saturated lactoferrin helps bacteria to adhere on the surface of the host. A highly polar electrostatic charge of PspA increase the capsule charge stabilization and the predominant part of protein prevents C3-mediated binding of the host complement to pneumococci by competing with the C-reactive protein.

The multifunctional immune evasive properties of PspA are essential for pneumococcal nasopharyngeal colonization and invasion of *S.pneumoniae.* PsaA is immunogenic and induces both the humoral (antibody production) and cellular (activation of phagocytes, cytotoxic T-lymphocytes, and the release of various cytokine) immune response.

A fragment or immunogenic fragment of pneumococcal surface protein A suitable for the present invention comprises the sequence between amino acids 32 - 318 of the protein sequence ID AAA27018.1.

Immunogenic fragments of pneumococcal surface protein A comprise immunogenic fragments of at least 50, 75, 100, 125, 150, 175, 200, 250, or 286 contiguous amino acids of sequence between amino acids 32 - 318 of the protein sequence ID AAA27018.1. In one embodiment the fragment is less than 50, 75, 100, 125, 150, 175, 200, 250, or 286 contiguous amino acids of sequence between amino acids 32 - 318 of the protein sequence ID AAA27018.1. The immunogenic fragments may elicit antibodies which can bind to the sequence between amino acids 32 - 318 of the protein sequence ID AAA27018.1. The immunogenic fragment may comprise a B and/or T cell epitope of the sequence between amino acids 32 - 318 of the protein sequence ID AAA27018.1.

Immunogenic fragments of pneumococcal surface protein A comprise immunogenic fragments having at least 70%, 80%, 85%, 90%, 95% or 99% sequence identity with the sequence between amino acids 32 - 318 of the protein sequence ID AAA27018.1

As used herein, the term "**synthetic *Streptococcus pneumoniae* oligosaccharide"** refers to saccharides produced synthetically to have the same or similar structure and/or composition of the *Streptococcus pneumoniae* saccharides disclosed herein, that are not produced in the native host cell (i.e., *S. pneumoniae*). The term "**synthetic**" includes for example, saccharides produced by chemical synthesis, but not those obtained by recombinant expression in host cells, such as gram negative bacteria.

As used herein the "**size**" of, for example, a conjugate or pneumococcal saccharide means the Molecular weight (Mw) of the conjugate or pneumococcal saccharide, respectively. The Mw may be provided in kilodaltons (kDa). As used herein "in the range" and "between" are inclusive of the range's upper and lower limits (for example, "in the range 30-300kDa" includes 30 kDa and 300kDa).

As used herein, the term "**conjugation**" is the coupling of carrier protein to saccharide.

The term "***linker***" as used herein encompasses molecular fragments capable of connecting the reducing-end monosaccharide of a saccharide with an immunogenic carrier or a solid support, optionally by binding to at least one interconnecting molecule. Thus, the function of the linker *per se* or together with the interconnecting molecule is to establish, keep and/or bridge a special distance between the reducing-end monosaccharide and an immunogenic carrier or a solid support. More specifically, one extremity of the linker is connected to the exocyclic oxygen atom at the anomeric center of the reducing-end monosaccharide and the other extremity is connected *via* the nitrogen atom with the interconnecting molecule, or directly with the immunogenic carrier or the solid support.

The **linker** or spacer group herein disclosed may be any moiety that enables to couple the oligosaccharide to a carrier molecule, i.e. a carrier protein or a glycosphingolipid. A large variety of such linker groups are known in the art and a suitable linker group can be selected in dependence from the respective carrier molecule. For example, the linker may be an aliphatic or aromatic residue, e.g. an alkyl(en) group or phenyl(en) group, comprising a reactive functional group, such as an amino group, preferably a primary amino group, (activated) carboxy group, aldehyde, azide, alkenyl or alkynyl group. In specific embodiments the linker may comprise a polyether or polyester chain. In particular, the linker is selected from the group comprising primary alkylamines, alkyl or aralkyl residues with a terminal aldehyde, azide, alkyne or alkene group or (activated) carboxy group, and alkylaryl and aryl residues, e.g. phenyl residues, comprising a reactive amine, an aldehyde or an azide group, or (activated) carboxy group.

<u>Synthetic saccharide ST3</u>

More preferably, the conjugate of a saccharide from *S. pneumoniae* serotype 3 and a carrier protein has the following general formula (**I**)

$$\mathbf{[H\text{-}(C)_{c3}\text{-}(D\text{-}C)_{c2}\text{-}(D)_{c1}\text{-}O\text{-}L^3\text{-}NH\text{-}W^3]_{m3}\text{-carrier protein}} \qquad \text{(I)}$$

wherein

C represents:

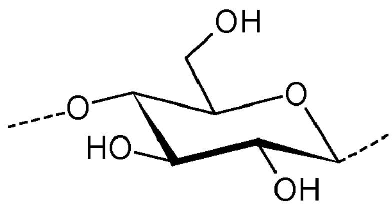

D represents

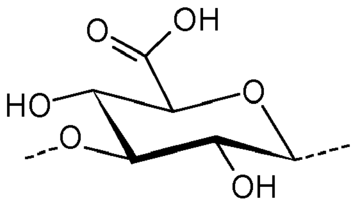

c2 is an integer selected from 1, 2, 3, 4, 5, 6, 7, 8, 9 and 10;
c1 and c3 represent independently of each other an integer selected from 0 and 1;
$L^3$ represents a linker;
m3 is comprised between about 2 and about 18;
$-W^3-$ is selected from:

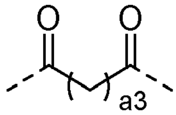
,
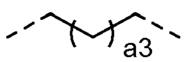
, and
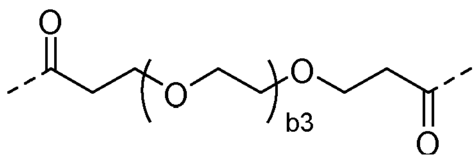
.

a3 represents an integer from 1 to 10;
b3 represents an integer from 1 to 4; and
carrier protein is selected from detoxified pneumolysin, or an immunogenic fragment thereof, and pneumococcal

surface protein A, or an immunogenic fragment thereof.

$-L^3-$ is defined as a linker and is part of the fragment $-O-L^3-NH-$. Thus, the linker - $L^3-$ is bound to an oxygen atom and to the nitrogen atom of the $-NH-W^3-$ fragment. It is preferred that at least two carbon atoms of the linker are between the oxygen atom and the $-NH-W^3-$ fragment, like $-O-C-C-NH-W^3-$. The linker $-L^3-$ can be an aliphatic chain, wherein the aliphatic chain can optionally include an aromatic chain inserted in it, or a number of heteroatoms oscillating from 0 to 10. The linker $-L^3-$ preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms. The shortest atom chain between the oxygen atom (i.e. the oxygen of $-O-L^3-NH-$) and the $-NH-W^3-$ fragment consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the $-NH-W^3-$ fragment) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker $-L^3-$, or the shortest chain is fully or partially fluorinated. The linker $-L^3-$ may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker $-L^3-$ may also contain amide (-NH-CO-, -CO-NH-) and/or urea (-NH-CO-NH-) residues and preferably only one amide or urea residue. The linker may also contain substituents and preferably two substituents, such as $R^{10**}$ and $R^{11**}$, or four substituents such as $R^{10**}$, $R^{11**}$, $R^{15**}$ and $R^{14**}$, which have the meanings as defined herein and which are preferably selected from: -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$, and $-N(C_2H_5)_2$;

In case the linker $-L^3-$ is fluorinated, more than two substituents -F are preferred. Linker $-L^3-$ is preferably selected from: $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-CF_2-$, $-(CF_2)_2-$, $-(CF_2)_3-$, $-(CF_2)_4-$, $-(CF_2)_5-$, $-(CF_2)_6-$, $-(CF_2)_7-$, $-(CF_2)_8-$, $-(CF_2)_9-$, $-(CF_2)_{10}-$, $-(CH_2)_2-O-(CH_2)_2-$, $-CH_2-O-(CH_2)_3-$ $-(CH_2)_3-O-CH_2-$, $-CH_2-O-(CH_2)_2-$, $-(CH_2)_2-O-CH_2-$, $-(CH_2)_3-O-(CH_2)_2-$, $-(CH_2)_2-O-(CH_2)_3-$, $-(CH_2)_4-O-CH_2-$, $-CH_2-O-(CH_2)_4-$, $-L^{a**}-$, $-L^{a**}-L^{e**}-$, $-L^{a**}-L^{b**}-L^{e**}-$, $-L^{a**}-L^{b**}-L^{d**}-L^{c**}-L^{e**}-$, $-L^{a**}-L^{d**}-L^{e**}-$;

wherein $-L^{a**}-$ is selected from: $-(CH_2)_{o**}-$, $-(CF_2)_{o**}-$, $-(CH_2-CH_2-O)_{o**}-C_2H_4-$, $-(CH_2-CH_2-O)_{o**}-CH_2-$, $-(CR^{10*}R^{11*})_{o**}-$,

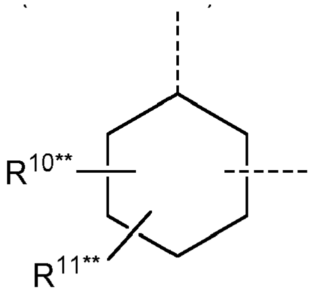,
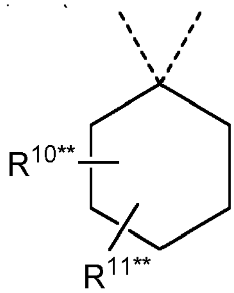,
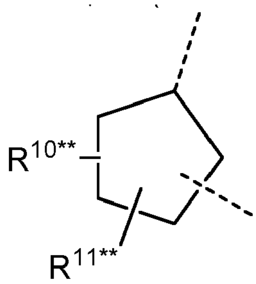,
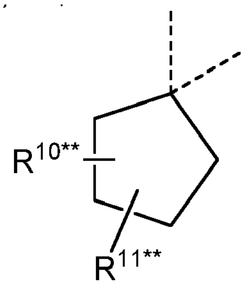,

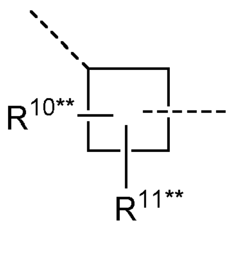, and 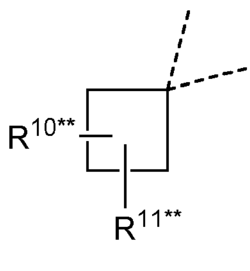;

$-L^{b**}-$ and $-L^{c**}-$ are independently of each other selected from: -O-, -NH-C(O)-NH-, -NH-C(S)-NH- -NH-C(O)-, -C(O)-NH-, -NH-C(O)-O-, $-NR^{9**}-$, $-NR^{18**}-$, $-SO_2-$,

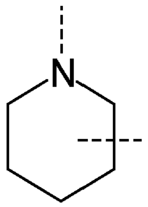,
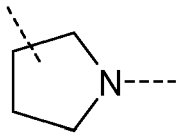,
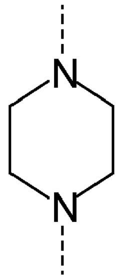,
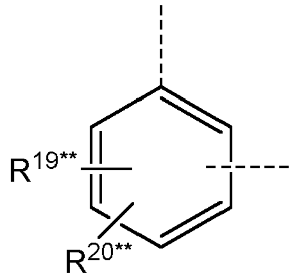,

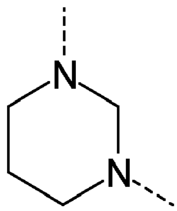,
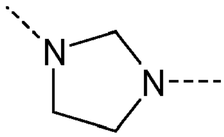,
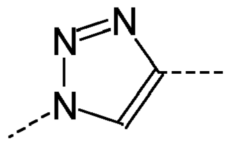,
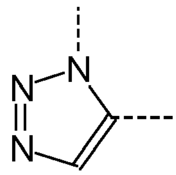,

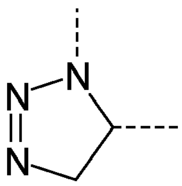,
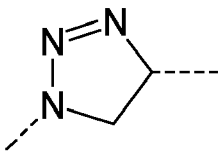,
and
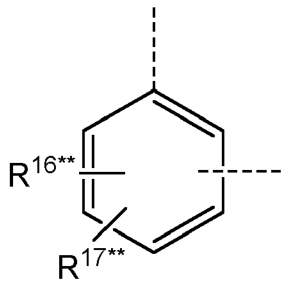;

$-L^{d**}-$ represents: $-(CH_2)_{q**}-$, $-(CF_2)_{q**}-$, $-(CR^{12**}R^{13**})_{q**}-$, $-(CH_2\text{-}CH_2\text{-}O)_{q**}\text{-}C_2H_4-$, $-(CH_2\text{-}CH_2\text{-}O)_{q**}\text{-}CH_2-$,

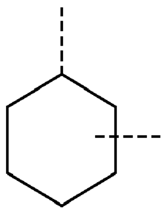,
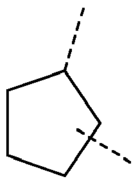,
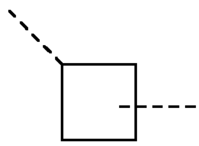,
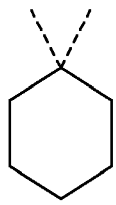,

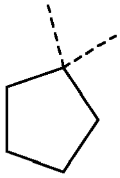,
and
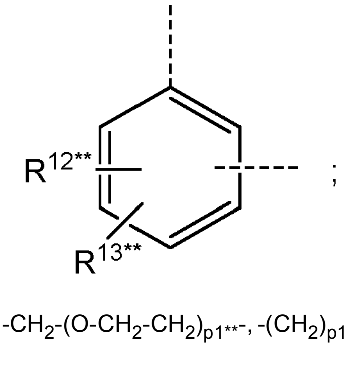;

$-L^{e**}-$ is selected from: $-(CH_2)_{p1**}-$, $-(CF_2)_{p1**}-$, $-C_2H_4\text{-}(O\text{-}CH_2\text{-}CH_2)_{p1**}-$, $-CH_2\text{-}(O\text{-}CH_2\text{-}CH_2)_{p1**}-$, $-(CH_2)_{p1**}\text{-}O\text{-}(CH_2)_{p2**}-$, $-(CR^{14**}R^{15**})_{p1**}-$, $-(CR^{14**}R^{15**})_{p1**}\text{-}O\text{-}(CR^{21**}R^{22**})_{p2**}-$,

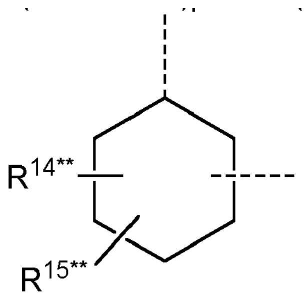 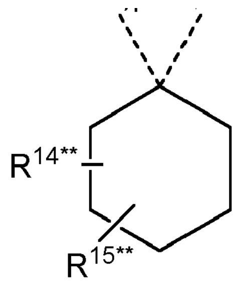, 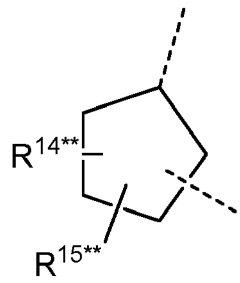, 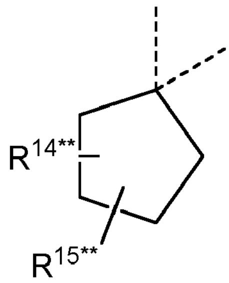,

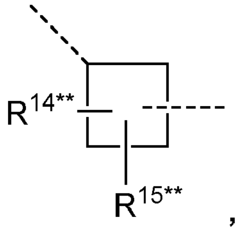, and 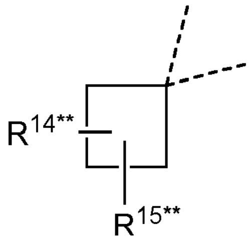;

$R^{9**}$ and $R^{18**}$ are independently of each other selected from: $-CH_3$, $-C_2H_5$, $-C_3H_7$, and $-C(O)CH_3$;
$R^{10**}$, $R^{11**}$, $R^{12**}$, $R^{13**}$, $R^{14**}$, $R^{15**}$, $R^{16**}$, $R^{17**}$, $R^{19**}$, $R^{20**}$, $R^{21**}$ and $R^{22**}$ are independently of each other selected from: -H, -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$ and $-N(C_2H_5)_2$;
o** q** p1** and p2** are independently of each other an integer selected from 1, 2, 3, 4, 5, 6, 7, 8, 9, and 10.

In general formula (**I**), c1 and c3 represent preferably 0. Thus, *S. pneumoniae* serotype 3 conjugate has general formula (**I-A**):

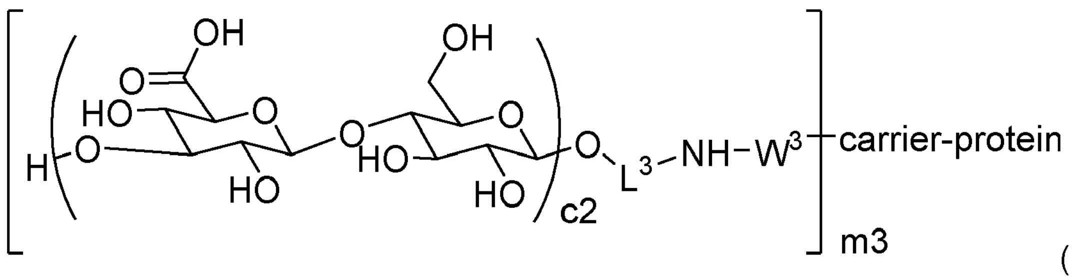

(**I-A**)

wherein c2, $L^3$, $-W^3-$, m3 and carrier protein have the meanings defined herein.

Preferably the linker $-L^3-$ is selected from: $-L^{a**}-$, $-L^{a**}-L^{e**}-$, $-L^{a**}-L^{b**}-L^{e**}-$, $-L^{a**}-L^{d**}-L^{e**}-$; wherein
$-L^{a**}-$ is selected from: $-(CH_2)_{o**}-$, $-(CH_2-CH_2-O)_{o**}-C_2H_4-$, $-(CH_2-CH_2-O)_{o**}-CH_2-$;
$-L^{b**}-$ represents -O-;
$-L^{d**}-$ is selected from: $-(CH_2)_{q**}-$, $-(CF_2)_{q**}-$, $-(CH_2-CH_2-O)_{q**}-C_2H_4-$, and $-(CH_2-CH_2-O)_{q**}-CH_2-$;
$-L^{e**}-$ is selected from: $-(CH_2)_{p1**}-$, $-(CF_2)_{p1**}-$, $-C_2H_4-(O-CH_2-CH_2)_{p1**}-$, $-CH_2-(O-CH_2-CH_2)_{p1**}-$ and $-(CH_2)_{p1**}-O-(CH_2)_{p2**}-$;
and o**, q**, p1** and p2** are independently of each other an integer selected from 1, 2, 3, 4, 5, and 6.

More preferably, the linker $-L^3-$ represents $-(CH_2)_{o**}-$ and o** is an integer selected from 2, 3, 4, 5, 6, 7 and 8. It is also preferred that $-W^3-$ represents

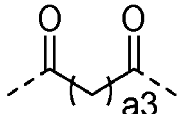

and a3 is an integer selected from 2, 3, 4, 5 and 6.

Synthetic saccharide ST2

The *Streptococcus pneumoniae* serotype 2 conjugate has preferably the following general formula (**II**):

$$[B^{*}\text{-}A_{y+3}\text{-}A_{y+2}\text{-}A_{y+1}\text{-}A_{y}\text{-}O\text{-}L^{2}\text{-}NH\text{-}W^{2}]_{m2}\text{-carrier protein} \qquad (\textbf{II})$$

wherein

y is an integer selected from 1, 2, 3 and 4;

$A_1 = A_5 =$ 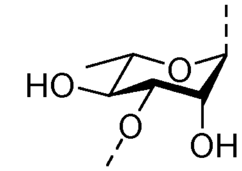, $A_2 = A_6 =$ 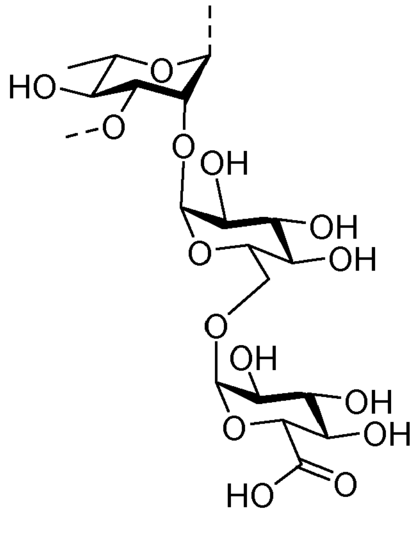,

$A_3 = A_7 =$ 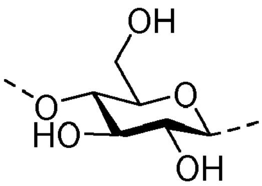; $A_4 =$ 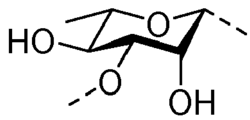;

B*- represents: H-, H-$A_y$-, H-$A_{y+1}$-$A_y$-, H-$A_{y+2}$-$A_{y+1}$-$A_y$- or H-$A_{y+3}$-$A_{y+2}$-$A_{y+1}$-$A_y$-;
$L^2$ represents a linker;
m2 is comprised between about 2 and about 18;
-$W^2$- is selected from:

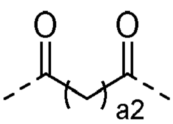, 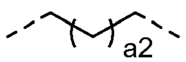, and 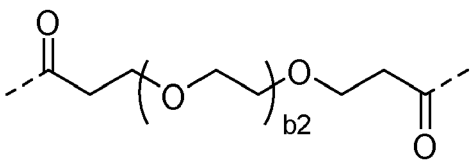;

a2 represents an integer from 1 to 10;
b2 represents an integer from 1 to 4; and
**carrier protein is selected from detoxified pneumolysin, or an immunogenic fragment thereof, and pneumococcal surface protein A, or an immunogenic fragment thereof.**

-$L^2$- is defined as a linker and is part of the fragment -O-$L^2$-NH-. Thus, the linker - $L^2$- is bound to an oxygen atom and to the nitrogen atom of the -NH-$W^2$- fragment. It is preferred that at least two carbon atoms of the linker are between the oxygen atom and the -NH-$W^2$- fragment, like -O-C-C-NH-$W^2$-. The linker -$L^2$- can be an aliphatic chain, wherein the aliphatic chain can optionally include an aromatic chain inserted in it, or a number of heteroatoms oscillating from 0 to 10.

The linker $-L^2-$ preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms.

The shortest atom chain between the oxygen atom (i.e. the oxygen of $-O-L^2-NH-$) and the $-NH-W^2-$ fragment consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the $-NH-W^2-$ fragment) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker $-L^2-$, or the shortest chain is fully or partially fluorinated. The linker $-L^2-$ may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker $-L^2-$ may also contain amide (-NH-CO-, -CO-NH-) and/or urea (-NH-CO-NH-) residues and preferably only one amide or urea residue. The linker may also contain substituents and preferably two substituents, such as $R^{10*}$ and $R^{11*}$, or four substituents such as $R^{10*}$, $R^{11*}$, $R^{15*}$ and $R^{14*}$, which have the meanings as defined herein and which are preferably selected from: -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$, and $-N(C_2H_5)_2$.

In case the linker $-L^2-$ is fluorinated, more than two substituents -F are preferred. Linker $-L^2-$ is preferably selected from: $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$ $-(CH_2)_9-$, $-(CH_2)_{10}-$, $-CF_2-$, $-(CF_2)_2-$, $-(CF_2)_3-$, $-(CF_2)_4-$, $-(CF_2)_5-$, $-(CF_2)_6-$, $-(CF_2)_7-$, $-(CF_2)_8-$, $-(CF_2)_9-$, $-(CF_2)_{10}-$, $-(CH_2)_2-O-(CH_2)_2-$, $-CH_2-O-(CH_2)_3-$, $-(CH_2)_3-O-CH_2-$, $-CH_2-O-(CH_2)_2-$, $-(CH_2)_2-O-CH_2-$, $-(CH_2)_3-O-(CH_2)_2-$, $-(CH_2)_2-O-(CH_2)_3-$, $-(CH_2)_4-O-CH_2-$, $-CH_2-O-(CH_2)_4-$, $-L^{a*}-$, $-L^{a*}-L^{e*}-$, $-L^{a*}-L^{b*}-L^{e*}-$, $-L^{a*}-L^{b*}-L^{d*}-L^{c*}-L^{e*}-$, $-L^{a*}-L^{d*}-L^{e*}-$;
wherein
$-L^{a*}-$ is selected from: $-(CH_2)_{o*}-$, $-(CF_2)_{o*}-$, $-(CH_2-CH_2-O)_{o*}-C_2H_4-$, $-(CH_2-CH_2-O)_{o*}-CH_2-$, $-(CR^{10*}R^{11*})_{o*}-$,

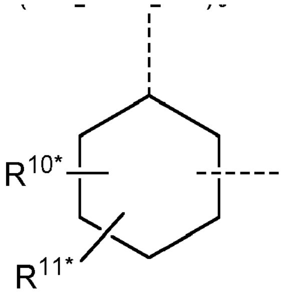,
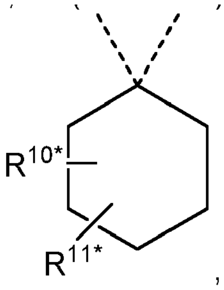,
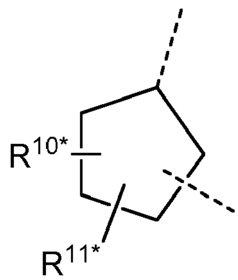,
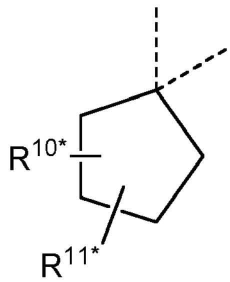,

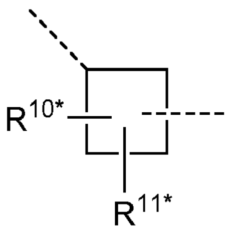, and 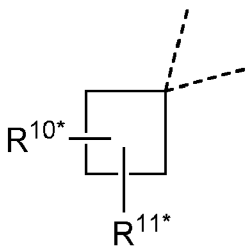;

$-L^{b*}-$ and $-L^{c*}-$ are independently of each other selected from: -O-, -NH-C(O)-NH-, -NH-C(S)-NH- -NH-C(O)-, -C(O)-NH-, -NH-C(O)-O-, $-NR^{9*}-$, $-NR^{18*}-$, $-SO_2-$,

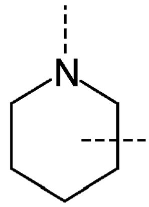 , 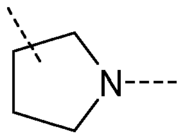 , 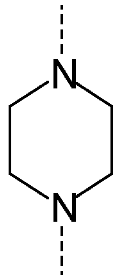 , 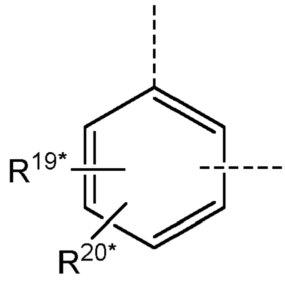

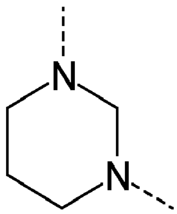 , 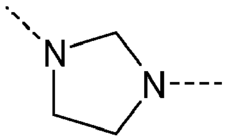 , 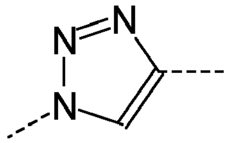 , 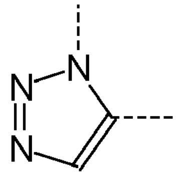

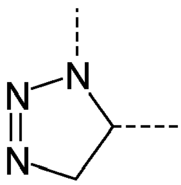 , 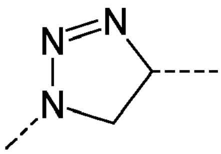 , and 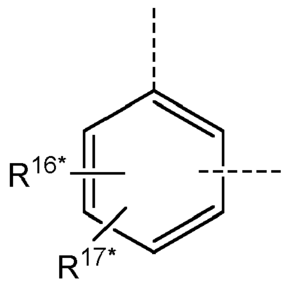 ;

-$L^{d*}$- represents $-(CH_2)_{q*}-$, $-(CF_2)_{q*}-$, $-(CR^{12*}R^{13*})_{q*}-$, $-(CH_2-CH_2-O)_{q*}-C_2H_4-$, $-(CH_2-CH_2-O)_{q*}-CH_2-$,

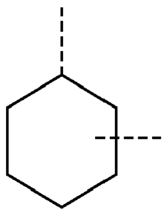 , 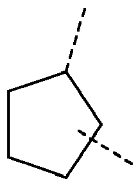 , 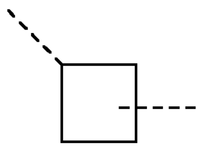 , 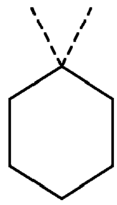 ,

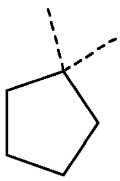 , and 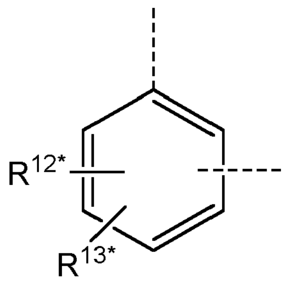 ;

-$L^{e*}$- is selected from: $-(CH_2)_{p1*}-$, $-(CF_2)_{p1*}-$, $-C_2H_4-(O-CH_2-CH_2)_{p1*}-$, $-CH_2-(O-CH_2-CH_2)_{p1*}-$, $-(CH_2)_{p1*}-O-(CH_2)_{p2*}-$, $-(CR^{14*}R^{15*})_{p1*}-$, $-(CR^{14*}R^{15*})_{p1*}-O-(CR^{21*}R^{22*})_{p2*}-$,

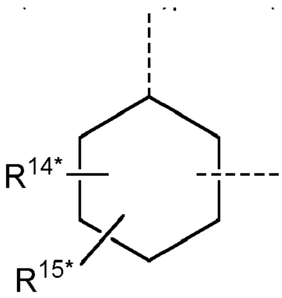
,
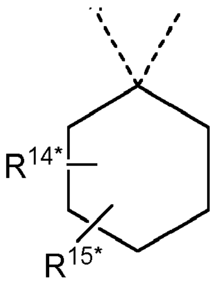
,
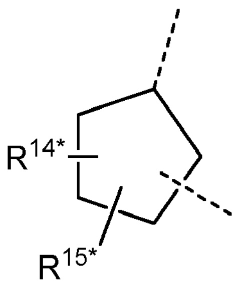
,
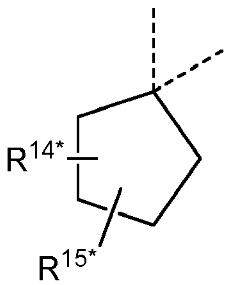
,

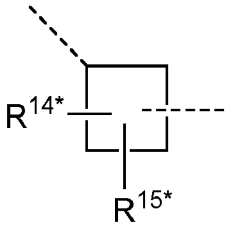
,
and
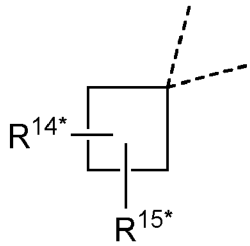
;

$R^{9*}$ and $R^{18*}$ are independently of each other selected from: $-CH_3$, $-C_2H_5$, $-C_3H_7$, and $-C(O)CH_3$;
$R^{10*}$, $R^{11*}$, $R^{12*}$, $R^{13*}$, $R^{14*}$, $R^{15*}$, $R^{16*}$, $R^{17*}$, $R^{19*}$, $R^{20*}$, $R^{21*}$ and $R^{22*}$ are independently of each other selected from: -H, -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$ and $-N(C_2H_5)_2$;
o*, q*, p1* and p2* are independently of each other an integer selected from 1, 2, 3, 4,5,6,7,8,9, and 10.

Synthetic saccharide of *S. pneumoniae* ST 1

*Streptococcus pneumoniae* type 1 (SP1) is one of the most prevalent *S. pneumoniae* serotypes. *Streptococcus pneumoniae* type 1 capsular polysaccharide is a linear polymer having as a repeating unit: [→3)-α-2,4,6-trideoxy-4-amino-D-GalNAc-(1→4)-α-D-GalAp-(1→3)-α-D-GalAp-(1→].

Synthetic saccharide structures derived from [→3)-α-2,4,6-trideoxy-4-amino-D-GalNAc-(1→4)-α-D-GalAp-(1→3)-α-D-GalAp-(1→] trisaccharide repeating unit of *Streptococcus pneumoniae* type 1 capsular polysaccharide were already reported. However, the method developed by Bundle (Chem. Eur. J. 2010, 16, 3476.) provides α-methoxy saccharides, which are not suitable for conjugation to an immunogenic carrier.

The *Streptococcus pneumoniae* serotype 1 oligosaccharide has preferably the following general formula (**III**):

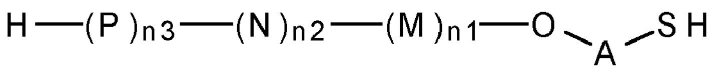

**(III)**

wherein A is a linker;

M, N and P represent independently of each other one of the following sugar fragments:

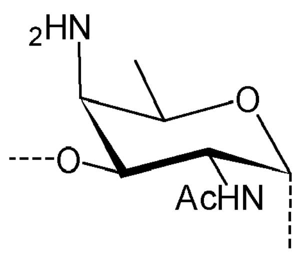
S1

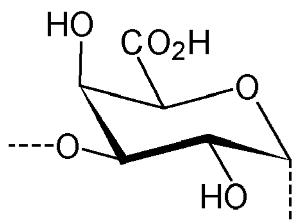
S2

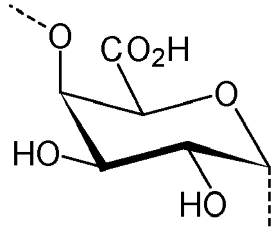
S3

wherein the sugar fragments S1, S2, S3 are connected to each other and to -O-A-SH fragment *via* O-glycosidic bonds, each sugar fragment S1, S2, and S3 is not more than once present in the general formula (**I**), sugar fragment S1 cannot be simultaneously connected to -O-A-SH and sugar fragment S3, sugar fragment S3 cannot be simultaneously connected to -O-A-SH and sugar fragment S2, and sugar fragment S2 cannot be simultaneously connected to -O-A-SH and sugar fragment S1, and n1, n2 and n3 are integers selected from 0 and 1, wherein at least one of the integers n1, n2 and n3 is 1.

**A is defined as a linker** and is part of the fragment -O-A-SH. Thus, the linker A is bound to an oxygen atom and to an SH-group, while the oxygen atom and the SH-group are bound to different carbon atoms of the linker A. It is preferred that at least two carbon atoms of the linker are between the oxygen atom and the SH-group, like -O-C-C-SH.

The linker A preferably contains between 2 and 20 carbon atoms (including the carbon atoms of optional side chains), more preferably between 2 and 18, more preferably between 2 and 16, more preferably between 2 and 14, more preferably between 2 and 12, and still more preferably between 2 and 10 carbon atoms.

The shortest atom chain between the oxygen (i.e. the oxygen of -**O**-A-SH) and the SH-group consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen and the SH-group) consists of 2 to 5 atoms, these are preferably carbon atoms. In case the shortest chain consists of 4 to 8 atoms, the chain may contain 1, 2, 3, or 4 heteroatoms selected from O, N and S. In case the shortest chain consists of 9 to 14 atoms, the chain may contain 1, 2, 3, 4, 5, or 6 heteroatoms selected from O, N and S. It is preferred that the shortest chain contains 0, 1, or 2 sulphur atoms and/or 0, 1, or 2 nitrogen atoms and/or 0, 1, 2, or 3 oxygen atoms. In case more than 4 oxygen atoms are present, preferably no other heteroatoms are present.

It is also preferred that the linker A, or the shortest chain is fully or partially fluorinated. The linker A may contain a 4-membered or a 5-membered or a 6-membered saturated carbocycle or a 6-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle.

The linker A may also contain amide (-NH-CO-, -CO-NH-) and/or urea (-NH-CO-NH-) residues and preferably only one amide or urea residue. The linker may also contain substituents and preferably one substituent such as $R^1$ or two substituents such as $R^1$ and $R^2$, which have the meanings as defined herein and which are preferably selected from $-OCH_3$, $-OC_2H_5$, $-CH_3$, $-C_2H_5$, $-CH_2F$, $-CF_2H$, $-CF_3$, $-C(O)-NH_2$, -NHAc, $-NH(CH_3)$, $-NH(C_2H_5)$, $-N(CH_3)_2$, $-N(C_2H_5)_2$, $-NH-C(O)-CH_3$, and $-C(O)-CH_3$.

In case the linker is fluorinated, more than two substituents -F are preferred.

In case an oxygen heterocycle is present each carbon atom of the oxygen heterocycle may be substituted by a hydroxy group (-OH). Thus, a 5-membered oxygen heterocycle may contain one or two hydroxy groups and a 6-membered oxygen heterocycle may contain one or two or preferably 3 hydroxy groups.

The linker A, also defined herein as $-A^a-A^b-A^c-A^d-$ or $-A^a-A^b-A^d-$ contains preferably 1, 2, 3 or 4 and more preferably 1, 2, or 3 of the following fragments: $-(CH_2)_{o1}-$, $-(CR^1R^2)_{o1}-$, $-(CH_2)_{o3}-(CH_2-CH_2-O)_{o2}-(CH_2)_{o1}-$, $-(CH_2)_{o1}-S-(CH_2)_{o4}-$, $-(CH_2)_{o1}-0-(CH_2)_{o4}-$, $-(CH_2)_{o1}-NH-(CH_2)_{o4}-$, $-(CH_2)_{o1}-NAC-(CH_2)_{o4}-$, $-(CH_2)_{o1}-C(O)-(CH_2)_{o4}-$, $-(CH_2)_{p1}-$, $-(CR^7R^8)_{p1}-$, $-(CH_2-CH_2-O)_{p1}-$, -O-, -S-, -NH-, -C(O)-, -NH-C(O)-NH-, $-NH-C(O)-(CH_2)_{p2}-$, $-C(O)-NH-(CH_2)_{p2}-$, $-NH-C(O)-C_2H_4-C(O)-NH-$, -C(O)-NH-, $-C(O)-NH-(CH_2-CH_2-O)_{p1}-C_2H_4-$, $-C(O)-NH-(CH_2-CH_2-O)_{p1}-$, $-(CH_2)_{q1}-$, $-(CR^{16}R^{17})_{q1}-$, $-(CH_2)_{q1}-NH-C(O)-$, $-(CH_2)_{q1}-C(O)-NH-$,

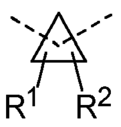
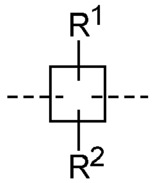
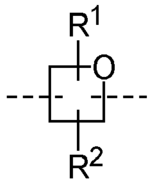
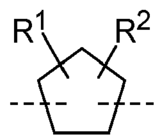
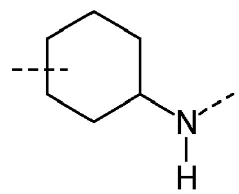

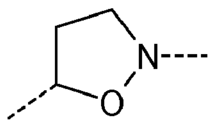
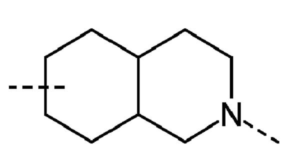
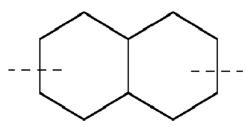
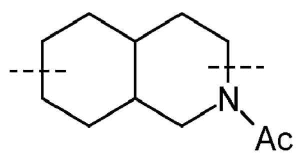

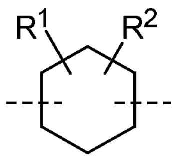
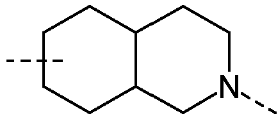
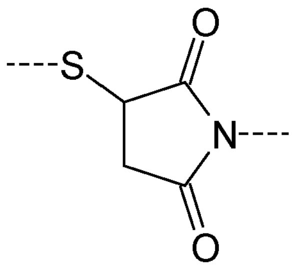
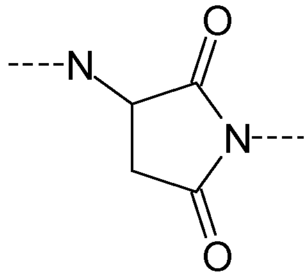

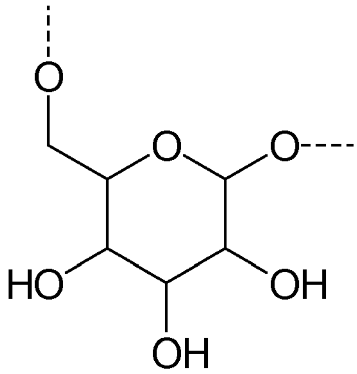
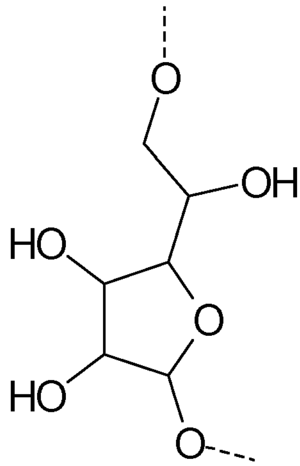
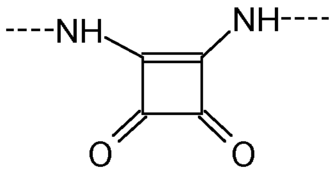

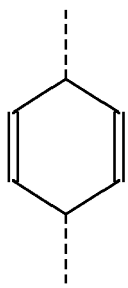
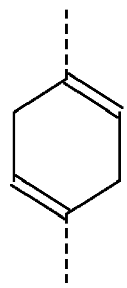

wherein not two heteroatoms of the above-mentioned residues are linked together, such as a group -S- is not linked to a group -NH-C(O)-NH-;
p2, o2, o3 are integers selected independently of each other from: 0, 1,2, 3, 4, 5, 6, 7, 8, 9, 10;
p1, q1, o1, o4 are integers selected independently of each other from: 1,2, 3, 4, 5, 6, 7, 8, 9, 10;
and
$R^1$, $R^2$, $R^7$, $R^8$, $R^{16}$, and $R^{17}$ represent independently of each other -H, $-OCH_3$, $-OC_2H_5$, $-CH_3$, $-C_2H_5$, $-C_3H_7$, $-CH(CH_3)_2$, -F, $-CH_2F$, $-CF_2H$, $-CF_3$, $-C(O)-NH_2$, $-SCH_3$, $-SC_2H_5$, -NHAc, $-NH(CH_3)$, $-NH(C_2H_5)$, $-N(CH_3)_2$, $-N(C_2H_5)_2$, $-NH-C(O)-CH_3$, and $-C(O)-CH_3$.

The linker -A- according to the present invention represents:

$$-A^a-A^b-A^c-A^d- \text{ or } -A^a-A^b-A^d- \text{ or } -A^a-A^d- \text{ or } -A^a-;$$

wherein
$A^a$ represents $-(CH_2)_{o1}-$, $-(CR^1R^2)_{o1}-$, $-(CR^1R^2)_{o1}-(CR^3R^4)_{o2}-$, $-(CR^1R^2)_{o1}-(CR^3R^4)_{o2}-(CR^5R^6)_{o3}$, $-(CR^1R^2)_{o3}-(CH_2-CH_2-O)_{o2}-(CR^3R^4)_{o1}-$, $-(CH_2-CH_2-O)_{o2}-(CR^1R^2)_{o3}-(CR^3R^4)_{o1}-$, $-(CR^1R^2)_{o1}-(CR^3R^4)_{o2}-S-(CR^5R^6)_{o4}-$, $-(CR^1R^2)_{o1}-(CR^3R^4)_{o2}-O-(CR^5R^6)_{o4}-$, $-(CR^1R^2)_{o1}-(CR^3R^4)_{o2}-NH-(CR^5R^6)_{o4}-$, $-(CR^1R^2)_{o1}-S-(CR^3R^4)_{o4}-$, $-(CR^1R^2)_{o1}-S-(CR^5R^6)_{o3}-(CR^3R^4)_{o4}-$, $-(CR^1R^2)_{o1}-O-(CR^3R^4)_{o4}-$, $-(CR^1R^2)_{o1}-O-(CR^5R^6)_{o3}-(CR^3R^4)_{o4}-$, $-(CR^1R^2)_{o1}-NH-(CR^3R^4)_{o4}-$, $-(CR^1R^2)_{o1}-NH-(CR^5R^6)_{o3}-(CR^3R^4)_{o4}-$, $-(CR^1R^2)_{o1}-C(O)-(CR^3R^4)_{o4}-$, $-(CR^1R^2)_{o1}-C(O)-(CR^5R^6)_{o3}-(CR^3R^4)_{o4}-$,

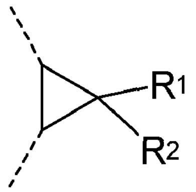
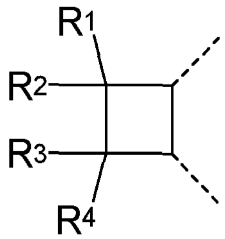
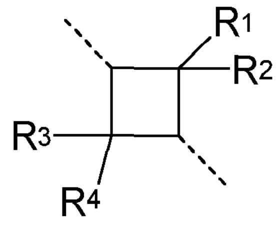
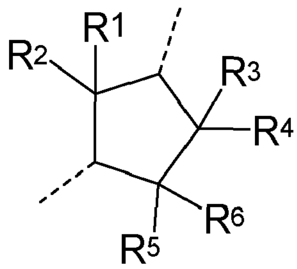
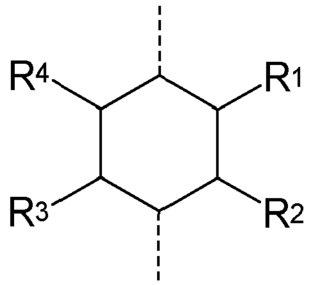
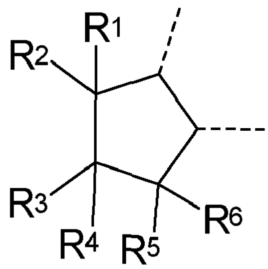
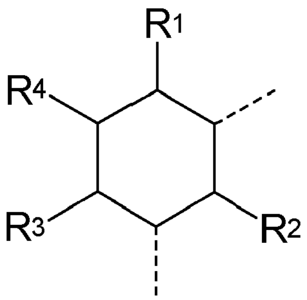
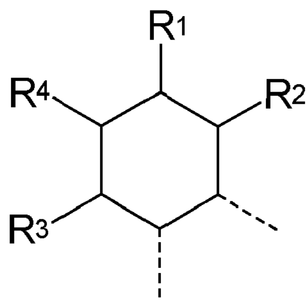
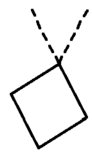
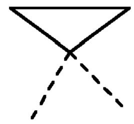
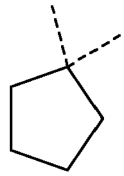
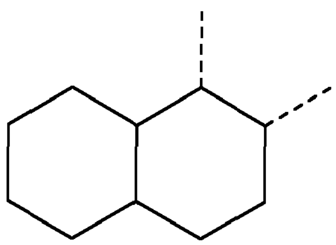
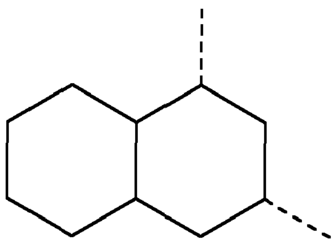
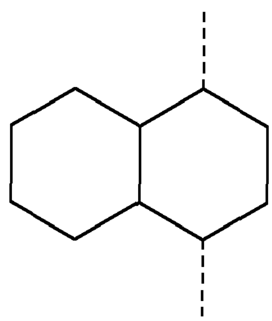
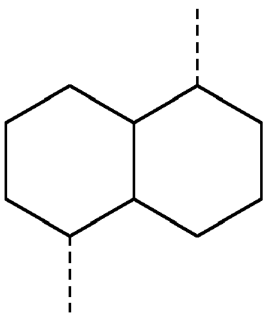
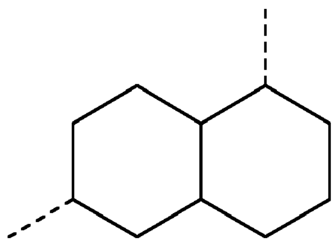
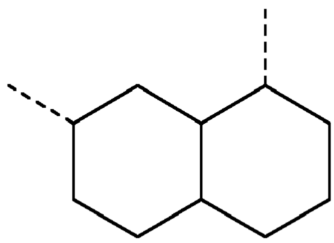

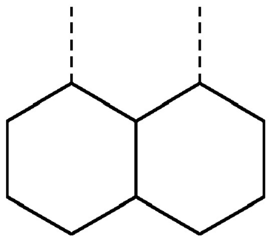 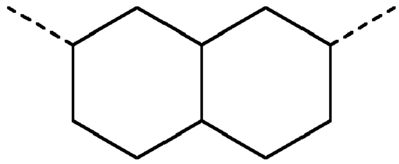 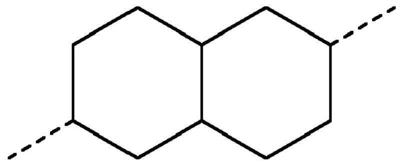

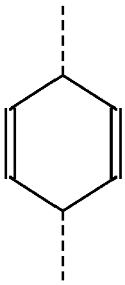 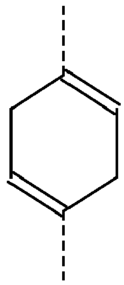

$A^b$ represents $-(CH_2)_{p1}-$, $-(CR^7R^8)_{p1}-$, $-(CH_2\text{-}CH_2\text{-}O)_{p1}-$, $-(CR^7R^8)_{p1}\text{-}S\text{-}(CR^9R^{10})_{p2}-$, $-(CR^7R^8)_{p1}\text{-}O\text{-}(CR^9R^{10})_{p2}-$, $-(CR^7R^8)_{p1}\text{-}NH\text{-}(CR^9R^{10})_{p2}-$, -O-, -S-, -NH-, -C(O)-, -NH-C(O)-NH-, $-NH\text{-}C(O)\text{-}(CH_2)_{p2}-$, $-C(O)\text{-}NH\text{-}(CH_2)_{p2}-$, $-NH\text{-}C(O)\text{-}C_2H_4\text{-}C(O)\text{-}NH-$, $-(CR^7R^8)_{p1}\text{-}(CR^9R^{10})_{p2}\text{-}(CH_2\text{-}CH_2\text{-}O)_{p3}-$, $-(CR^7R^8)_{p1}\text{-}(CH_2\text{-}CH_2\text{-}O)_{p2}\text{-}(CR^9R^{10})_{p3}-$, $-(CH_2\text{-}CH_2\text{-}O)_{p1}\text{-}(CR^7R^8)_{p2}\text{-}(CR^9R^{10})_{p3}-$, $-C(O)\text{-}NR^{15}-$, $-(CR^7R^8)_{p1}\text{-}(CR^9R^{10})_{p2}\text{-}(CR^{11}R^{12})_{p3}$, $-C(O)\text{-}NH\text{-}CH(R^{18})-$, $-C(O)\text{-}NH\text{-}CH(R^{18})\text{-}C(O)\text{-}NH\text{-}CH(R^{19})-$, $-C(O)\text{-}NH\text{-}(CH_2\text{-}CH_2\text{-}O)_{p1}\text{-}C_2H_4-$, $-C(O)\text{-}NH\text{-}(CH_2\text{-}CH_2\text{-}O)_{p1}-$,

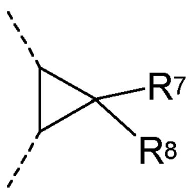 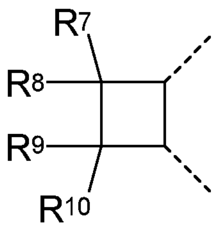 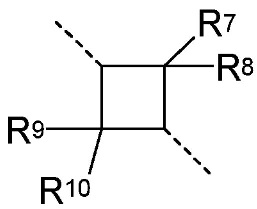

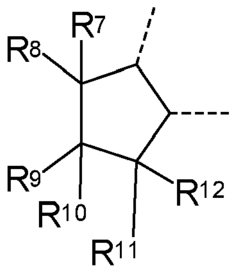 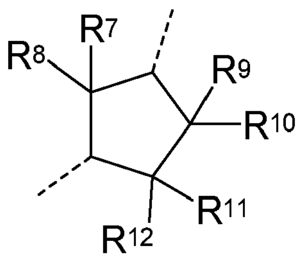 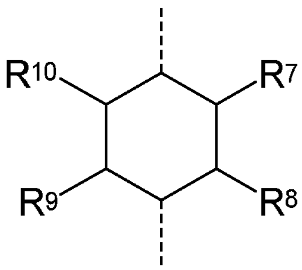

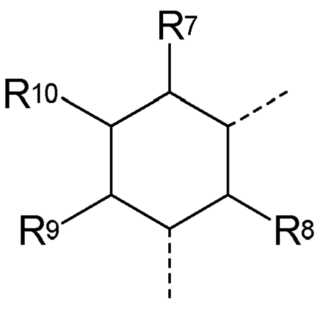 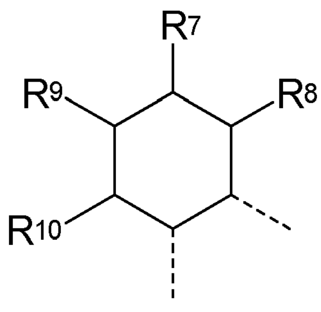 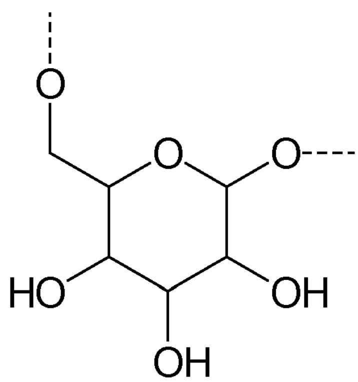

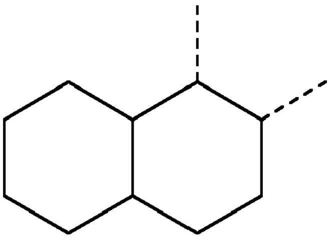
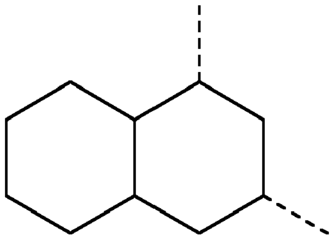
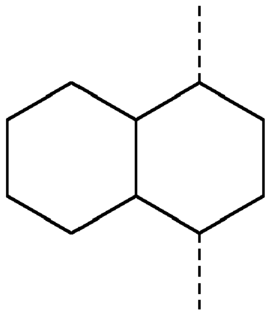
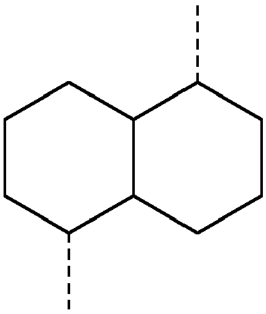
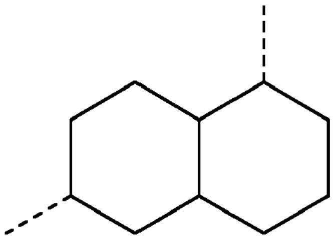
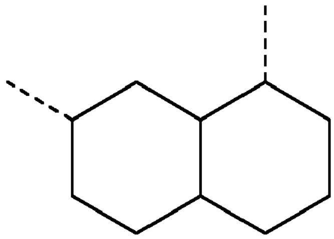
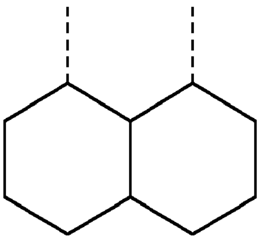
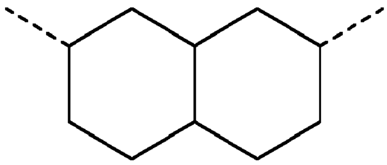
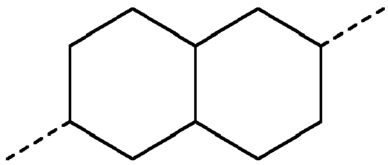
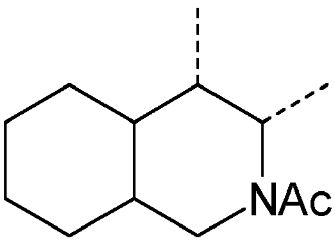
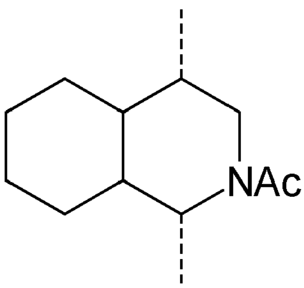
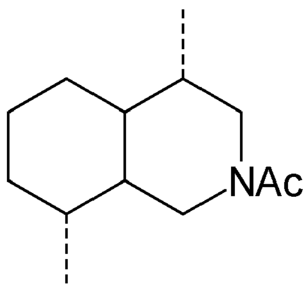
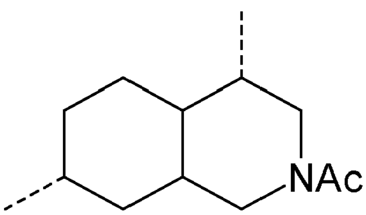
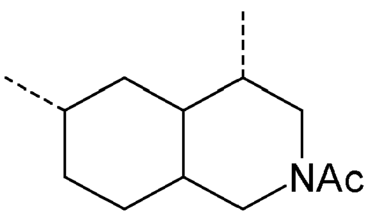
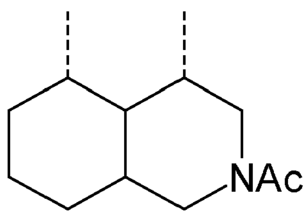

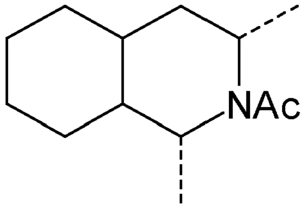
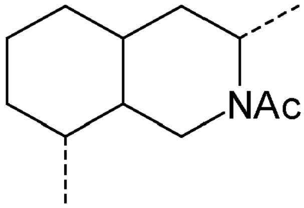
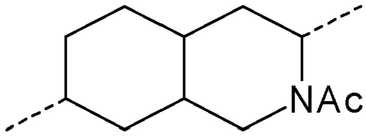

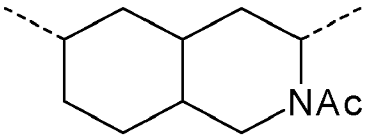
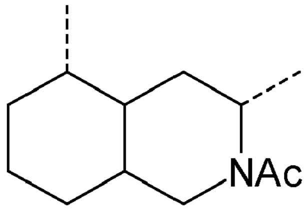
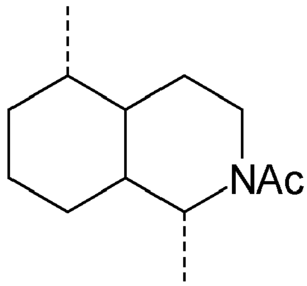

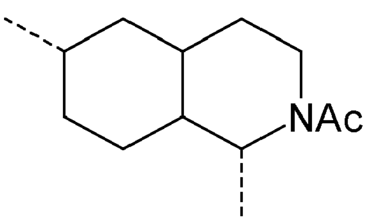
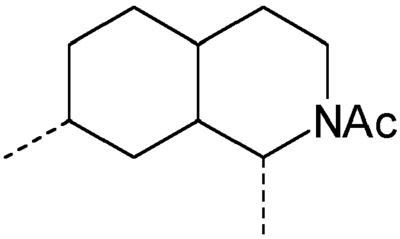
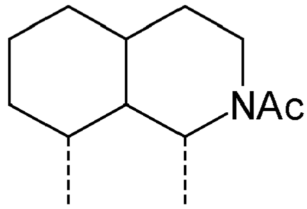

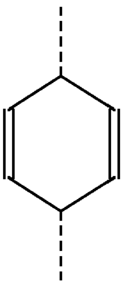
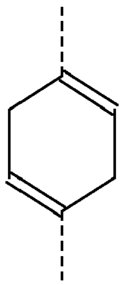
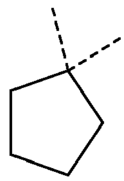

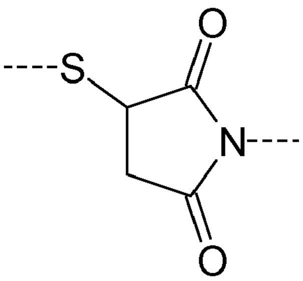
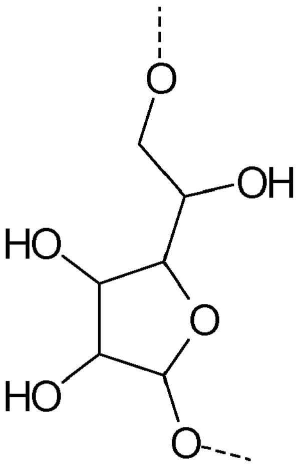
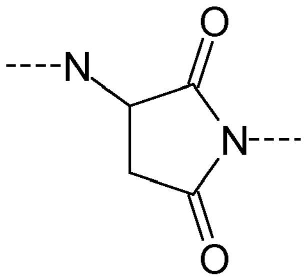

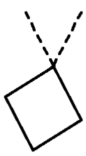
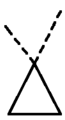

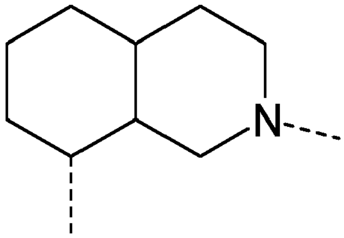 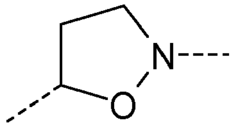 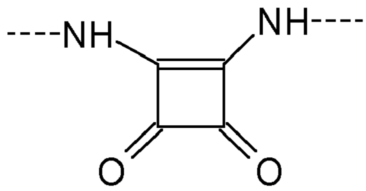

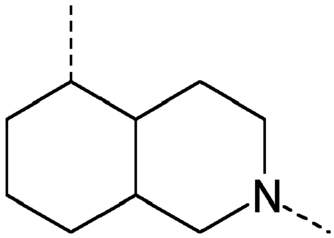 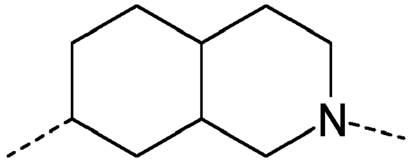 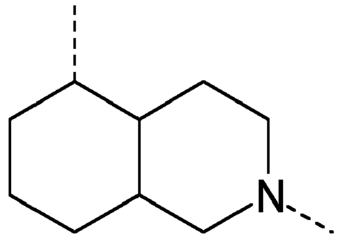

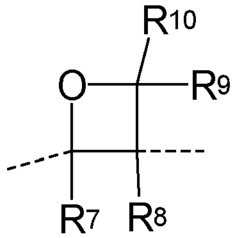 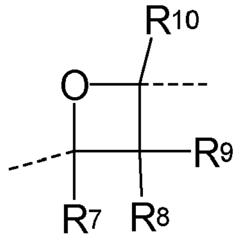 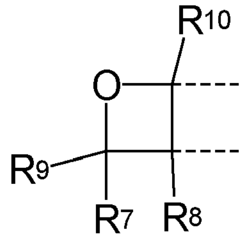

$A^c$ represents $-(CH_2)_{q1}-$, $-(CR^{16}R^{17})_{q1}-$, $-(CH_2)_{q1}-NH-C(O)-$, $-(CH_2)_{q1}-C(O)-NH-$,

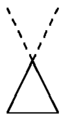 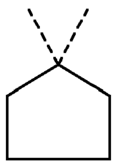 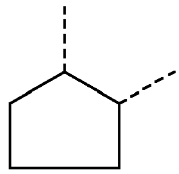 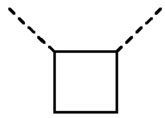 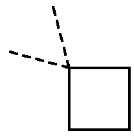

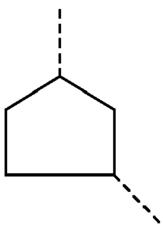 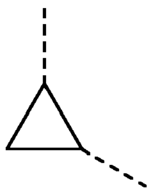 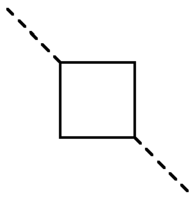 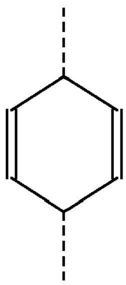 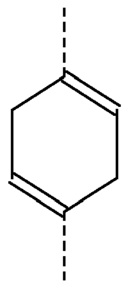

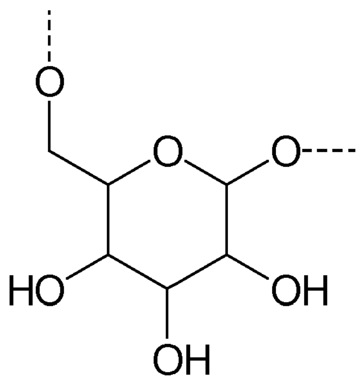
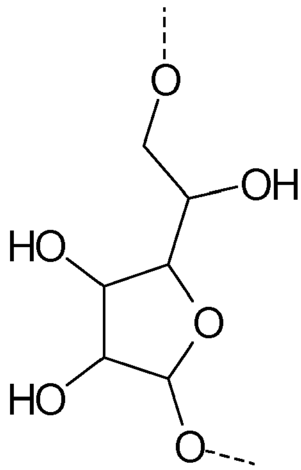
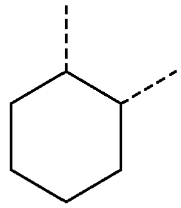
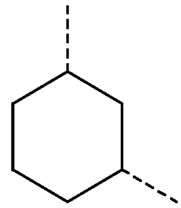
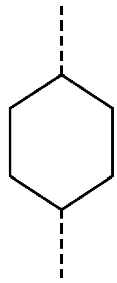
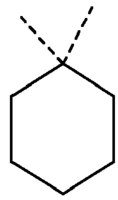

$A^d$ represents $-(CH_2)_{m1}-$, $-(CR^{13}R^{14})_{m1}-$, $-CH_2-S-(CH_2)_{m1}-$, $-CH_2-O-(CH_2)_{m1}-$, $-CH_2-C(O)-(CH_2)_{m1}-$, $-(CH_2-CH_2-O)_{m1}-(CR^{13}R^{14})_{m2}-$, $-(CH_2)_{m1}-(CR^{13}R^{14})_{m2}-$, $-(CR^{13}R^{14})_{m2}-(CH_2)_{m1}-$ $-(CR^{13}R^{14})_{m1}-(CH_2)_{m2}-$, $-(CH_2)_{m2}-(O-CH_2-CH_2)_{m1}-$,

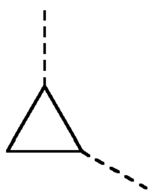
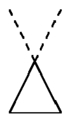
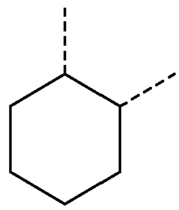
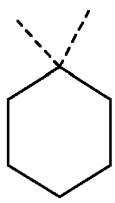
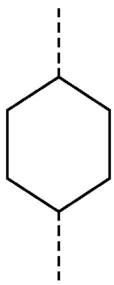
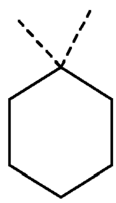
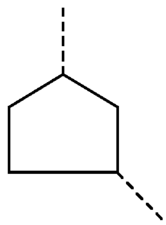
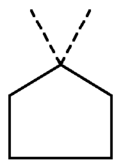
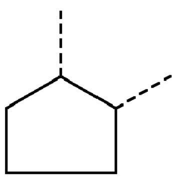
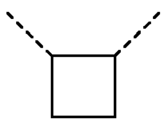
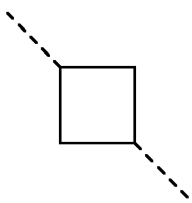
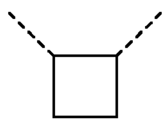

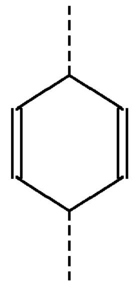 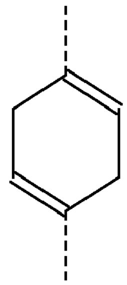

$R^1$-$R^{14}$, $R^{16}$ and $R^{17}$ represent independently of each other -H, $-OCH_3$, $-OC_2H_5$, $-OC_3H_7$, -cyclo-$C_3H_5$, -cyclo-$C_4H_7$, -cyclo-$C_5H_9$, -cyclo-$C_6H_{11}$, -cyclo-$C_7H_{13}$, -cyclo-$C_8H_{15}$, $-CH_2$-cyclo-$C_6H_{11}$, -C(cyclo-$C_6H_{11}$)$_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-C_7H_{15}$, $-C_8H_{17}$, $-C_6H_4-OCH_3$, $-CH_2-CH_2-OCH_3$, $-CH_2-OCH_3$, $-CH_2-C_6H_4-OCH_3$, -F, $-CH_2F$, $-CF_2H$, $-CF_3$, -C(O)-NH$R^{15}$, -C(O)-NH$R^{22}$, -C(O)-NH$R^{23}$, -C(O)-NH$R^{24}$, -C(O)-NH$R^{25}$, $-SCH_3$, $-SC_2H_5$, -N$R^{15}R^{22}$, -NH$R^{15}$, -NH$R^{22}$, -NH$R^{23}$, -NH$R^{24}$, -NH$R^{25}$, -NH-C(O)-$R^{15}$, -NH-C(O)-$R^{22}$, -NH-C(O)-$R^{23}$, -NH-C(O)-$R^{24}$, -NH-C(O)-$R^{25}$, -C(O)-$R^{15}$, -C(O)-$R^{22}$ -C(O)-$R^{23}$, -C(O)-$R^{24}$ -C(O)-$R^{25}$.

$R^{15}$, $R^{22}$, $R^{23}$, $R^{24}$ and $R^{25}$ represents : $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$;

$R^{18}$, $R^{19}$, $R^{20}$ and $R^{21}$ represent independently of each other $-CH_2-OCH_3$, $-CH_2-SCH_3$, $-CH_2-SeCH_3$, $-C_2H_4-OCH_3$, $-C_2H_4-SCH_3$, $-C_2H_4-SeCH_3$, -H $-CH_3$, $-C_2H_5$, $-C_3H_7$, $-CH(CH_3)(OCH_3)$, $-CH(CH_3)_2$, $-CH_2-CH(CH_3)_2$, $-CH_2$-Ph, $-CH_2-CH(CH_3)(C_2H_5)$, $-CH_2-C(O)-NH_2$, $-C_2H_4-C(O)-NH_2$, $-CH_2$-*p*-$C_6H_4$-$OCH_3$;

p2, p3, o2, o3 are integers selected independently of each other from: 0, 1, 2, 3, 4, 5, 6, 7, 8, 9, 10;

p1, q1, o1, o4, m1, m2 are integers selected independently of each other from: 1,2, 3, 4, 5, 6, 7, 8, 9, 10.

Preferably the linker A represents the residue -$A^a$-.

-$A^a$- is preferably a linear carbon chain or a saturated carbocycle selected from

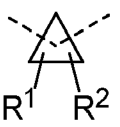 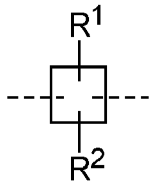 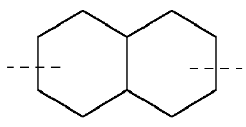 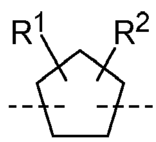 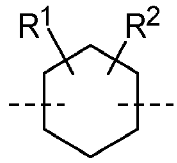

Also preferably the linker A represents $-(CH_2)_{o1}$-$A^b$-$A^c$-$A^d$-, $-(CH_2)_{o1}$-$A^b$-$A^d$-, $-(CH_2)_{o1}$-$A^d$-, $-(CR^1R^2)_{o1}$-$A^b$-$A^c$-$A^d$-, $-(CR^1R^2)_{o1}$-$A^b$-$A^d$-, or $-(CR^1R^2)_{o1}$-$A^d$-, wherein $A^b$, $A^c$ and $A^d$ have the meanings as defined herein and $R^1$ and $R^2$ have the meanings as defined herein and preferably represent independently of each other -H, $-OCH_3$, $-OC_2H_5$, $-CH_3$, $-C_2H_5$, $-C_3H_7$, $-CH(CH_3)_2$, -F, $-CH_2F$, $-CF_2H$, $-CF_3$, $-C(O)-NH_2$, $-SCH_3$, $-SC_2H_5$, -NHAc, $-NH(CH_3)$, $-NH(C_2H_5)$, $-N(CH_3)_2$, $-N(C_2H_5)_2$, $-NH-C(O)-CH_3$, and $-C(O)-CH_3$.

$A^b$ represents in general and especially in the afore-mentioned general formula $-(CH_2)_{o1}$ -$A^b$-$A^c$-$A^d$-, $-(CH_2)_{o1}$-$A^b$-$A^d$-, $-(CR^1R^2)_{o1}$-$A^b$-$A^c$-$A^d$-, $-(CR^1R^2)_{o1}$-$A^b$-$A^d$- preferably one of the following residues: -O-, -S-, -NH-, -C(O)-, -NH-C(O)-NH-, $-NH-C(O)-(CH_2)_{p2}$-, $-C(O)-NH-(CH_2)_{p2}$-, $-NH-C(O)-C_2H_4-C(O)-NH$-, -C(O)-N$R^{15}$-, -C(O)-NH-CH($R^{18}$)-, $-C(O)-NH-(CH_2-CH_2-O)_{p1}-C_2H_4$-, $-C(O)-NH-(CH_2-CH_2-O)_{p1}$-, wherein the substituents $R^{15}$ and $R^{18}$ have the meanings as defined herein and preferably $-CH_3$, $-C_2H_5$, or $-C_3H_7$.

$A^c$ represents in general and especially in the afore-mentioned general formula $-(CH_2)_{o1}$-$A^b$-$A^c$-$A^d$- and $-(CR^1R^2)_{o1}$-$A^b$-$A^c$-$A^d$- preferably one of the following residues: $-(CH_2)_{q1}$-,

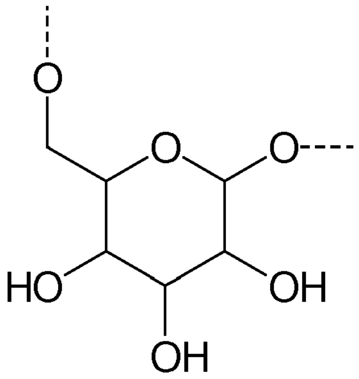
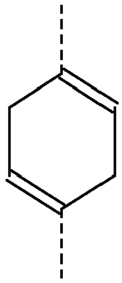
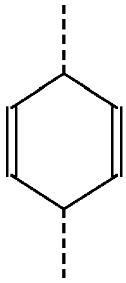
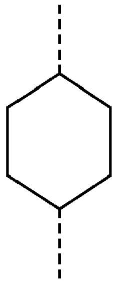
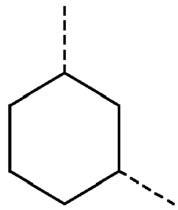
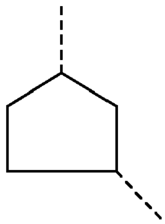
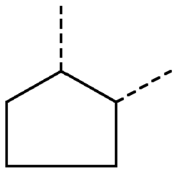
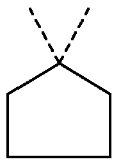
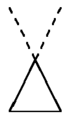

$A^d$ represents in general and especially in the afore-mentioned general formula $-(CH_2)_{o1}-A^b-A^c-A^d-$, $-(CH_2)_{o1}-A^b-A^d-$, $-(CH_2)_{o1}-A^d-$, $-(CR^1R^2)_{o1}-A^b-A^c-A^d-$, $-(CR^1R^2)_{o1}-A^b-A^d-$ and $-(CR^1R^2)_{o1}-A^d-$ preferably one of the following residues: $-(CH_2)_{m1}-$,

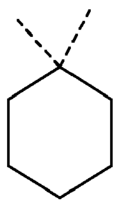
,
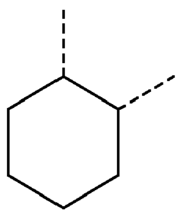
,
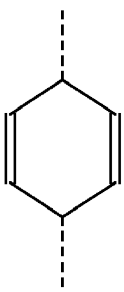
,
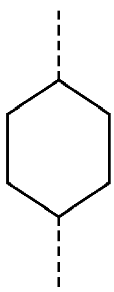
,
or
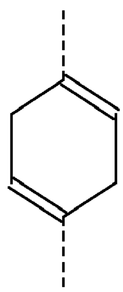

The function of the linker alone or together with the interconnecting molecule is to covalently connect the reducing-end of the saccharides to an immunogenic carrier or to a solid support. An interconnecting molecule according to the present invention refers to a bifunctional molecule containing functional group X and functional group Y, wherein functional group X is capable of reacting with the terminal thiol group on the linker A and the functional group Y is capable of binding to an immunogenic carrier or to a solid support. Thus, the present invention refers to saccharides of the formula (**I**), wherein the linker *per se* or together with the interconnecting molecule is capable of establishing, keeping and/or bridging a special distance between the reducing-end monosaccharide and an immunogenic carrier or a solid support.

In a preferred embodiment according to the present invention the linker -A-represents $-A^a-A^b-A^c-A^d-$. Preferably, the fragment $-A^a-A^b-A^c-A^d-$ is selected from the following fragments:

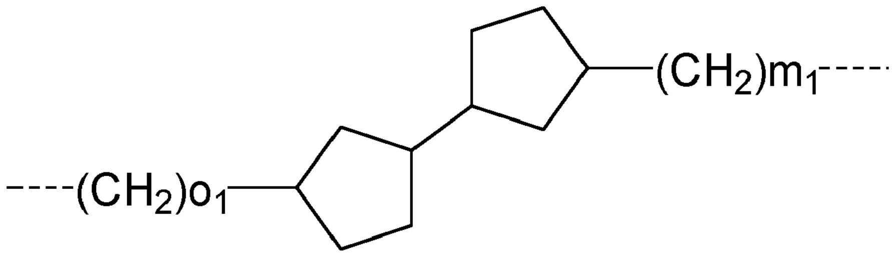

,

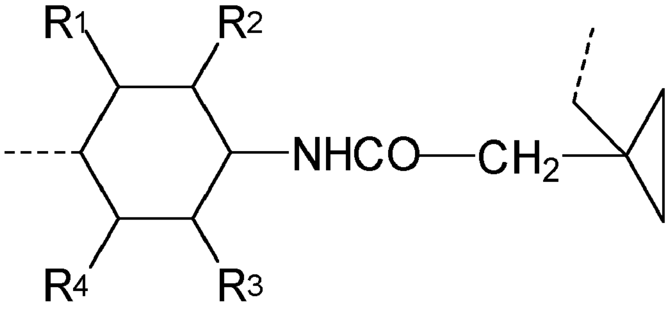

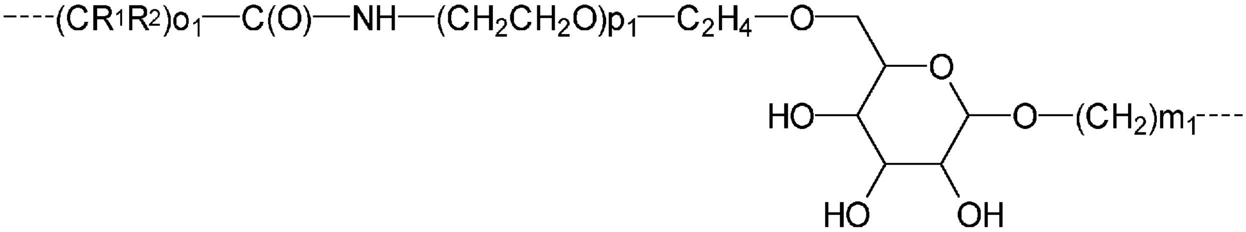

Another preferred embodiment is directed to saccharides of general formula (I), wherein the linker -A- represents $-A^a-A^b-A^d-$. Preferably, fragment $-A^a-A^b-A^d-$ is selected from:

$-(CH_2)o_1-S-(CH_2)o_4-NH-C(O)-NH-(CH_2)m_1-$

$-(CH_2)o_1-NH-C(O)-NH-(CH_2)m_1-$.

$-(CH_2)o_1-CR^3R^4-O-(CH_2)o_4-C(O)-NR^{15}-CH(C(O)NHR^{22})-(CH_2)m_1-$.

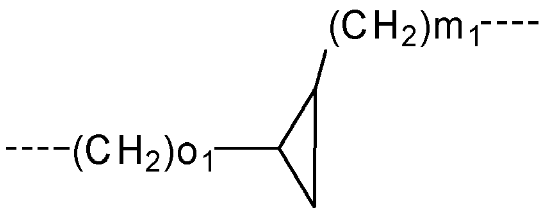

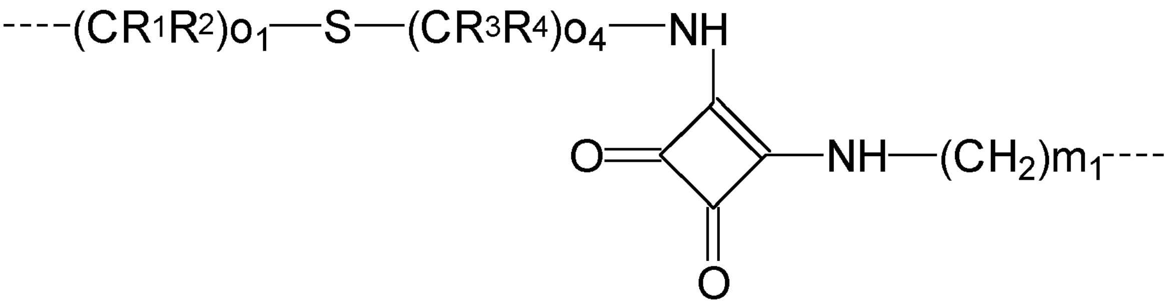

$-(CH_2)o_1-(CR^3R^4)o_2-O-(CH_2)o_4-C(O)-NH-(CH_2)m_2-(OCH_2CH_2)m_1-.$

$-(CR^1R^2)o_1-(CR^3R^4)o_2-(CR^5R^6)o_3-S-(CH_2)m_1-(CR^{13}R^{14})m_2-$

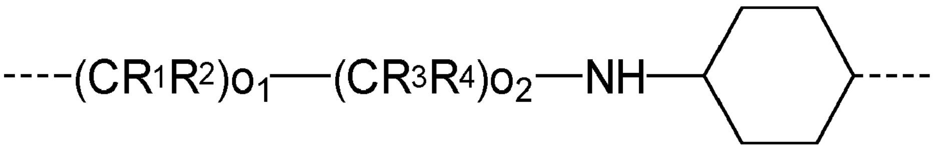

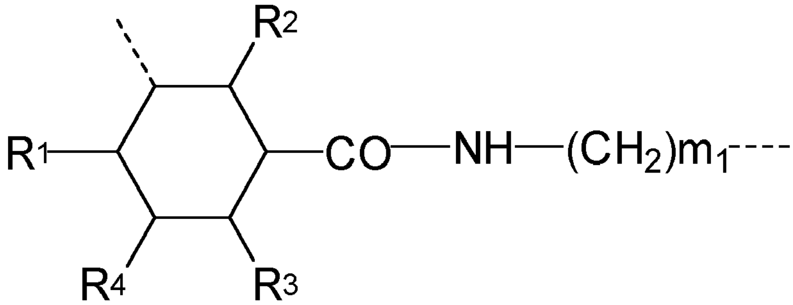

Preferably the linker A represents $-A^a-A^d-$, and more preferably fragment $-A^a-A^d-$ is selected from

$-(CR^1R^2)o_3-(C_2H_4O)o_2-(CR^3R^4)o_1-(CH_2)m_1-(CR^{13}R^{14})m_2-$

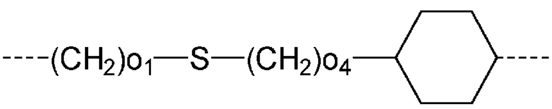

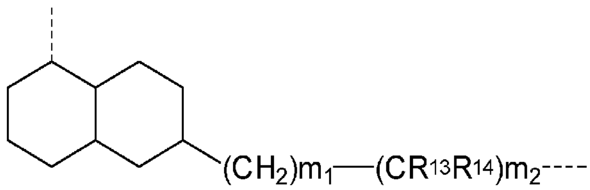

Preferably, the linker -A- represents $-A^a-$, wherein $-A^a-$ is selected from:

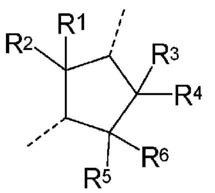

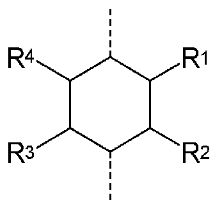

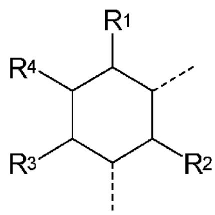

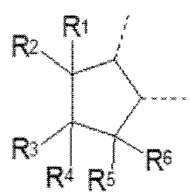

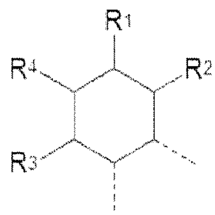

$-(CH_2)_{o1}-$, $-(CR^1R^2)_{o1}-$, $-(CR^1R^2)_{o3}-(CH_2-CH_2-O)_{o2}-(CR^3R^4)_{o1}-$, $-(CR^1R^2)_{o1}-(CR^3R^4)_{o2}-(CR^5R^6)_{o3}$, $-(CH_2-CH_2-O)_{o2}-(CR^1R^2)_{o3}-(CR^3R^4)_{o1}-$, $-(CH_2)_{o1}-(CR^3R^4)_{o2}-S-(CH_2)_{o4}-$, $-(CH_2)_{o1}-(CR^3R^4)_{o2}-O-(CH_2)_{o4}-$, $-(CH_2)_{o1}-(CR^3R^4)_{o2}-NH-(CH_2)_{o4}-$, $-(CR^1R^2)_{o1}-S-(CR^3R^4)_{o4}$, $-(CR^1R^2)_{o1}-O-(CR^3R^4)_{o4}$, $-(CR^1R^2)_{o1}-NH-(CR^3R^4)o_4$, or $-(CR^1R^2)_{o1}-C(O)-(CR^3R^4)_{o4}$.

In a preferred embodiment, substituents $R^1$-$R^{14}$, $R^{16}$ and $R^{17}$ are selected from: -H, $-OCH_3$, $-OC_2H_5$, $-CH_3$, $-C_2H_5$, $-C_3H_7$, -F, $-CH_2F$, $-CF_2H$, $-CF_3$, $-CH(CH_3)_2$, $-C_4H_9$, $-CH_2-CH(CH_3)_2$, $-CH(CH_3)-C_2H_5$, $-C(CH_3)_3$, $-CH_2-CH_2-OCH_3$, $-CH_2-OCH_3$, $-SCH_3$, $-SC_2H_5$, $-NR^{15}R^{22}$, $-NHR^{15}$, $-NHR^{22}$, $-NHR^{23}$, $-NHR^{24}$, $-NHR^{25}$, $-NH-C(O)-R^{15}$, $-NH-C(O)-R^{22}$, $-NH-C(O)-R^{23}$, $-NH-C(O)-R^{24}$, $-NH-C(O)-R^{25}$.

Even more preferred are linkers, wherein -A- represents $-A^a-$. Preferably, fragment $-A^a-$ has the meaning: $-(CH_2)_{o1}-$, $-(CR^1R^2)_{o1}-$, $-(CH_2)_{o1}-(CR^3R^4)_{o2}-(CH_2)_{o3}$, $-(CH_2-CH_2-O)_{o2}-(CH_2)_{o1}-$, $-(CH_2-CH_2-O)_{o2}-(CR^1R^2)-(CH_2)_{o1}-$, $-(CH_2)_{o1}-(CR^3R^4)_{o2}-S-(CH_2)_{o4}$, $-(CR^1R^2)_{o1}-S-(CH_2)_{o4}$, $-(CH_2)_{o1}-(CR^3R^4)_{o2}-O-(CH_2)_{o4}$, $-(CR^1R^2)_{o1}-O-(CH_2)_{o4}$, or $-(CR^1R^2)_{o1}-C(O)-(CR^3R^4)_{o4}$,

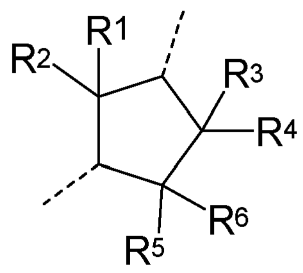 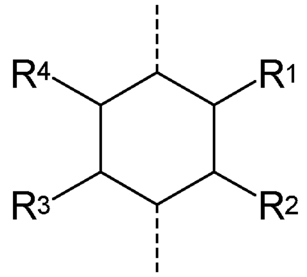 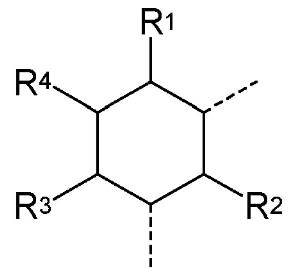

wherein

o1 and o4 are integers selected from 1, 2, 3, 4, 5, 6;
o2 and o3 are integers selected from 0, 1, 2, 3, 4, 5, 6;
and, substituents $R^1$-$R^6$ are selected from: -H, $-OCH_3$, $-OC_2H_5$, $-CH_3$, $-C_2H_5$, $-C_3H_7$, -F, $-CH_2F$, $-CF_2H$, $-CF_3$, $-CH(CH_3)_2$, $-C_4H_9$, $-CH_2-CH(CH_3)_2$, $-CH(CH_3)-C_2H_5$, $-C(CH_3)_3$, $-CH_2-CH_2-OCH_3$, $-CH_2-OCH_3$, $-SCH_3$, $-SC_2H_5$, $-NHR^{15}$, $-NHR^{22}$, $-NHR^{23}$, $-NHR^{24}$, $-NHR^{25}$, $-NH-C(O)-R^{15}$, $-NH-C(O)-R^{22}$, $-NH-C(O)-R^{23}$, $-NH-C(O)-R^{24}$, $-NH-C(O)-R^{25}$.

Synthetic saccharide of *S. pneumoniae* ST4

*Streptococcus pneumoniae* serotype 4 CPS is included in all pneumococcal conjugate vaccines. The SP4 CPS consists of a tetrasaccharide repeating unit with the sequence β-(1,3)-ManNAc-α-(1,3)-FucNAc-α-(1,3)-GalNAc-α-(1,4)-Gal containing an acid labile *trans-2,* 3 (*S*)-pyruvate on the galactose moiety.

The synthetic *Streptococcus pneumoniae* serotype 4 oligosaccharide has preferably the following general formula (**IV**):

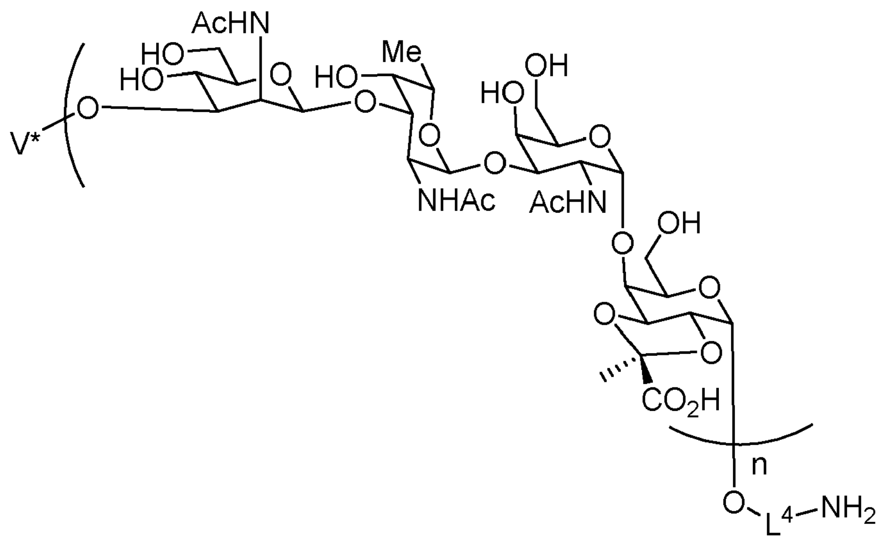

(**IV**)

wherein

n is an integer selected from 1, 2 and 3;
V*- represents H-;
-L4- is selected from: $-L^a-$, $-L^a-L^e-$, $-L^a-L^b-L^e-$ and $-L^a-L^d-L^e-$;
$-L^a-$ is selected from: $-(CH_2)_o-$, $-(CH_2-CH_2-O)_o-C_2H_4-$ and $-(CH_2-CH_2-O)_o-CH_2$;
$-L^b-$ represents -O-;
$-L^d-$ is selected from: $-(CH_2)_q-$, $-(CF_2)_q-$, $-(CH_2-CH_2-O)_q-C_2H_4-$, and $-(CH_2-CH_2-O)q-CH_2-$;
$-L^e-$ is selected from: $-(CH_2)_{p1}-$, $-(CF_2)_{p1}-$, $-C_2H_4-(O-CH_2-CH_2)_{p1}-$,

$-CH_2-(O-CH_2-CH_2)_{p1}-$ and $-(CH_2)_{p1}-O-(CH_2)_{p2}-$;
o, q, p1 and p2 are independently of each other an integer selected from 1, 2, 3, 4, 5, and 6;
or a diastereoisomer or a pharmaceutically acceptable salt thereof.

The *Streptococcus pneumoniae* serotype 4 oligosaccharide is preferably covalently linked to a carrier protein through the nitrogen atom of the $-O-L^4-NH_2$ group.

<u>Synthetic saccharide ST5</u>

*S. pneumoniae* serotype 5 (SP5) is globally the fifth most prevalent IPD-causing serotype among young children, and the second most prevalent serotype amongst children in the poorest countries of the world, eligible to the support of the Global Alliance for Vaccines and Immunization (GAVI). Furthermore, SP5 was recently identified as the causative agent of an epidemic of severe pneumonia among young Israeli army recruits and community outbreaks of invasive infection in impoverished, urban populations in Canada. A very recent study showed that SP5 is a frequent cause of IPD outbreaks among children and adults in Spain. Although most SP5 subtypes are still susceptible to antibiotics, the emergence and dissemination of antibiotic-resistant SP5 bacteria is of concern.

The SP5 CPS is a component of the most recent PCV-10 and PCV-13 conjugate vaccines. The SP5 CPS consists of a branched pentasaccharide repeating unit with the sequence [→4)-β-D-Glc*p*-(1→4)-[α-L-Pne*p*NAc-(1→2)-β-D-Glc*p*A-(1→3)]-α-L-Fuc*p*NAc-(1→3)-β-D-Sug*p* (1→], where Sugp is 4-keto-D-FucNAc (systematic name: 2-acetamido-2,5-dideoxy-D-*xylo*-hexos-4-ulose) and PneNAc is *N*-acetyl-L-pneumosamine.

The synthetic *Streptococcus pneumoniae* serotype 5 oligosaccharide has preferably the following general formula (**V**):

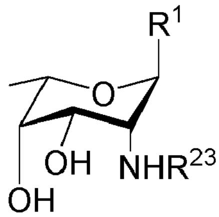

(**V**)

wherein

$R^1$ is selected from $R^2$, $R^4$,

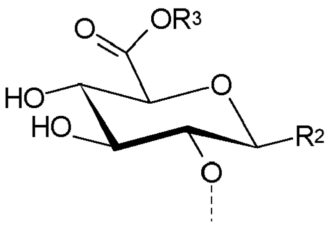

,

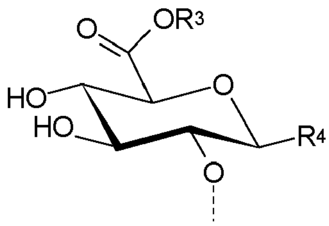

;

$R^2$ represents

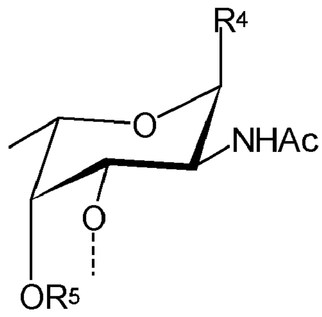

;

$R^3$ is selected from -H, $-CH_3$, $-C_2H_5$, $-C_3H_7$, $-C_4H_9$ and $-CF_3$;
$R^4$ represents $R^6$ or

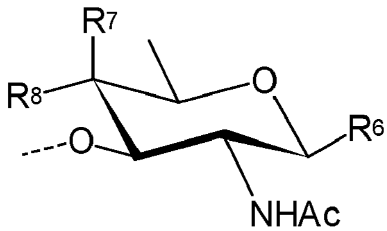

;

$R^5$ represents -H or

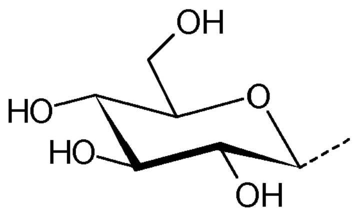

;

$R^6$ represents **-O-L⁵-NH₂;**
$R^7$ and $R^8$ are independently of each other selected from -H and -OH and cannot be simultaneously -H;
$R^7$ and $R^8$ can form together a =O residue; or in other words $R^7$ and $R^8$ can form together with the carbon atom to which they are attached to a carbonyl group C=O;
$R^{23}$ is selected from -H, $-C(O)CH_3$, $-C(O)CF_3$ and $-C(O)CCl_3$;
- **L**$^{\mathbf{5}}$- is a linker;

and pharmaceutically acceptable salts thereof.

**[0116]** - **L**$^{\mathbf{5}}$- is defined as a linker and is part of the fragment $-O-L-NH_2$. Thus, the linker - **L**$^{\mathbf{5}}$- is bound to an oxygen atom and to the nitrogen atom of the $NH_2$-group. It is preferred that at least two carbon atoms of the linker are between the oxygen atom and the $NH_2$-group, like $-O-C-C-NH_2$. The linker - **L**$^{\mathbf{5}}$- can be an aliphatic chain, wherein the aliphatic chain can optionally include an aromatic chain inserted in it, or a number of heteroatoms oscillating from 0 to 10.

The linker **L**$^{\mathbf{5}}$ preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms.

The shortest atom chain between the oxygen (i.e. the oxygen of -O- **L**$^{\mathbf{5}}$-$NH_2$) and the $NH_2$-group consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the $NH_2$-group) consists of 2 to 6 atoms, these are preferably carbon atoms. In case the shortest chain consists of 4 to 8 atoms, the chain may contain 1, or 2 heteroatoms selected from O, N and S. In case the shortest chain consists of 9 to 14 atoms, the chain may contain 1, 2, 3, or 4 heteroatoms selected from O, N and S. It is preferred that the shortest chain contains 0, 1, or 2 sulphur atoms and/or 0, 1, or 2 nitrogen atoms and/or 0, 1, 2, or 3 oxygen atoms.

It is also preferred that the linker - **$L^5$**-, or the shortest chain is fully or partially fluorinated. The linker - **$L^5$**- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker - **$L^5$**- may also contain amide (-NH-CO-, -CO-NH-) and/or urea (-NH-CO-NH-) residues and preferably only one amide or urea residue. The linker may also contain substituents and preferably one substituent such as $R^{10}$ or two substituents such as $R^{10}$ and $R^{14}$, which have the meanings as defined herein and which are preferably selected from: -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$, and $-N(C_2H_5)_2$;

In case the linker is fluorinated, more than two substituents -F are preferred.

Preferably the linker - **$L^5$**- is selected from: $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-CF_2-$, $-(CF_2)_2-$, $-(CF_2)_3-$, $-(CF_2)_4-$, $-(CF_2)_5-$, $-(CF_2)_6-$, $-(CF_2)_7-$, $-(CF_2)_8-$, $-(CF_2)_9-$, $-(CF_2)_{10}-$, $-(CH_2)_2-O-(CH_2)_2-$, $-CH_2-O-(CH_2)_3-$, $-(CH_2)_3-O-CH_2-$, $-CH_2-O-(CH_2)_2-$, $-(CH_2)_2-O-CH_2-$, $-(CH_2)_3-O-(CH_2)_2-$, $-(CH_2)_2-O-(CH_2)_3-$, $-(CH_2)_4-O-CH_2-$, $-CH_2-O-(CH_2)_4-$, $-L^a-$, $-L^a-L^e-$, $-L^a-L^b-L^e-$, $-L^a-L^b-L^d-L^c-L^e-$, $-L^a-L^d-L^e-$;
wherein

$-L^a-$ is selected from: $-(CH_2)_m-$, $-(CF_2)_m-$, $-(CH_2-CH_2-O)_m-C_2H_4-$, $-(CH_2-CH_2-O)m-CH_2-$, $-(CR^{10}R^{11})_m-$,

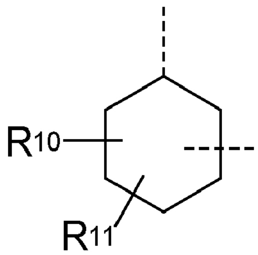
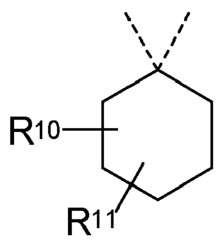
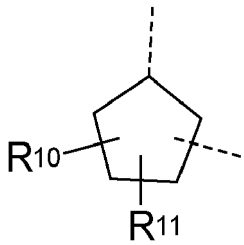
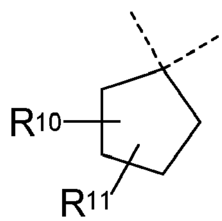
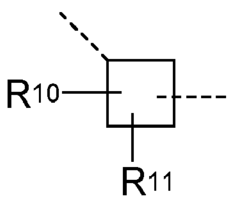
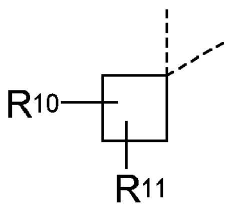
;

$-L^b-$ and $-L^c-$ are independently of each other selected from: -O-, -S-, -NH-C(O)-NH-, -NH-C(S)-NH- -NH-C(O)-, -C(O)-NH-, -NH-C(O)-O-, $-NR^9-$, $-NR^{18}-$, $-SO_2-$,

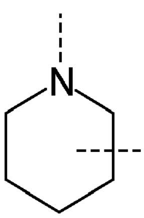
,
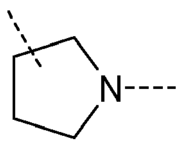
,
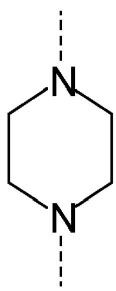
,
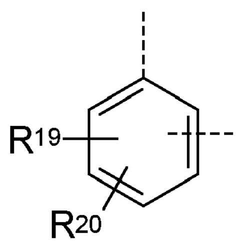
,

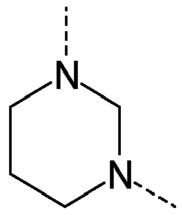 , 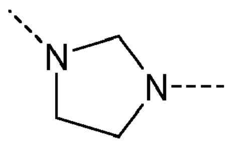 , 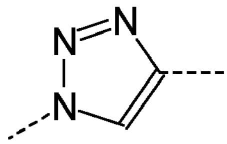 , 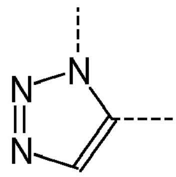 ,

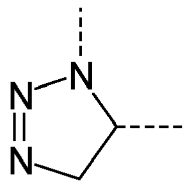 , 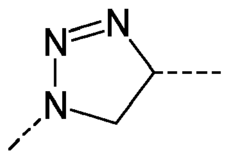 , 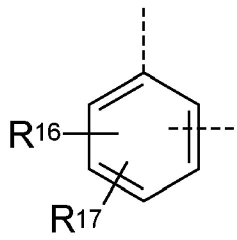 ;

$-L^d-$ represents $-(CH_2)_n-$, $-(CF_2)_n-$, $-(CR^{12}R^{13})_n-$, $-(CH_2-CH_2-O)_n-C_2H_4-$, $-(CH_2-CH_2-O)_n-CH_2-$,

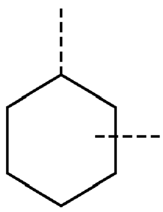 , 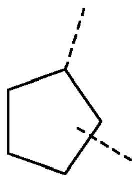 , 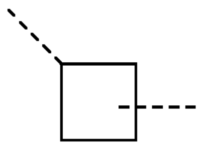 , 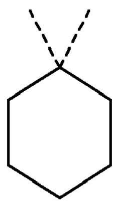 ,

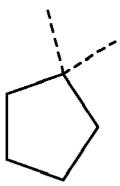 , 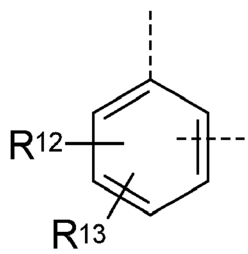 ;

$-L^e-$ is selected from: $-(CH_2)_{p1}-$, $-(CF_2)_{p1}-$, $-C_2H_4-(O-CH_2-CH_2)_{p1}-$, $-CH_2-(O-CH_2-CH_2)_{p1}-$, $-(CH_2)_{p1}-O-(CH_2)_{p2}-$, $-(CH_2)_{p1}-S-(CH_2)_{p2}-$, $-(CR^{14}R^{15})_{p1}-$, $-(CR^{14}R^{15})_{p1}-O-(CR^{21}R^{22})_{p2}-$, $-(CR^{14}R^{15})_{p1}-S-(CR^{21}R^{22})_{p2}-$,

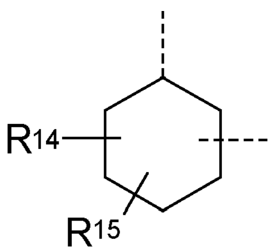 , 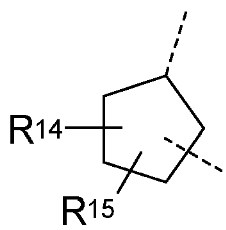 , 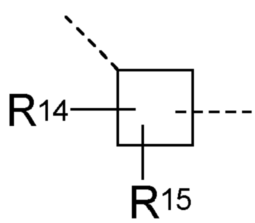 , 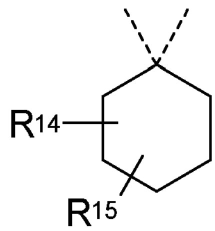 ,

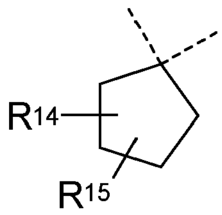
,
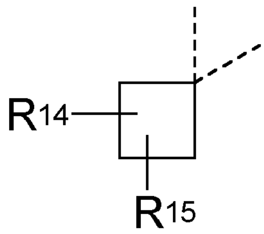
;

$R^9$ and $R^{18}$ are independently of each other selected from: $-CH_3$, $-C_2H_5$, $-C_3H_7$, and $-C(O)CH_3$;
$R^{10}$, $R^{11}$, $R^{12}$, $R^{13}$, $R^{14}$, $R^{15}$, $R^{16}$, $R^{17}$, $R^{19}$, $R^{20}$, $R^{21}$ and $R^{22}$ are independently of each other selected from: -H, -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$ and $-N(C_2H_5)_2$;
m, n, p1 and p2 are independently of each other an integer selected from 1, 2, 3, 4, 5, 6, 7, 8, 9, and 10.

Synthetic saccharide ST8

Preferably, the conjugate of a saccharide from *S. pneumoniae* serotype 8 and a carrier protein is a synthetic oligosaccharide from *S. pneumoniae* type 8 covalently coupled to $CRM_{197}$.

Even more preferably, the conjugate of a saccharide from S. *pneumoniae* serotype 8 and a carrier protein has the following general formula (**VI**):

$$[V^*\text{-}U_{x+3}\text{-}U_{x+2}\text{-}U_{x+1}\text{-}U_x\text{-}O\text{-}L^8\text{-}NH\text{-}W^1]_{m1}\text{-carrier protein} \qquad (VI)$$

wherein

x is an integer selected from 1, 2, 3 and 4;

$U_1 = U_5 =$ 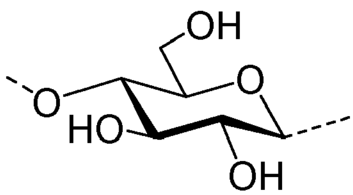 ; $U_2 = U_6 =$ 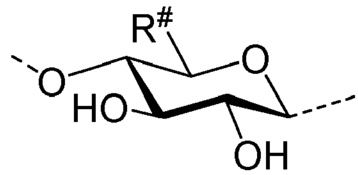 ;

$U_3 = U_7 =$ 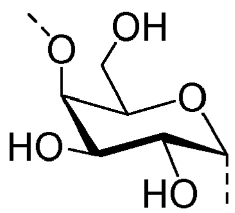 ; $U_4 =$ 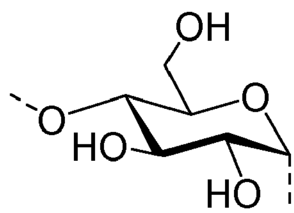 ;

V*- represents H-, $H\text{-}U_x$- or $H\text{-}U_{x+1}\text{-}U_x$-;
$R^\#$ represents -COOH or $-CH_2OH$;
$L^8$represents a linker;
m1 is comprised between 2 and 18;
$-W^1$- is selected from:

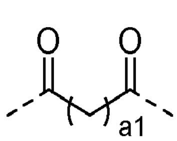
,
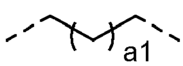
, and
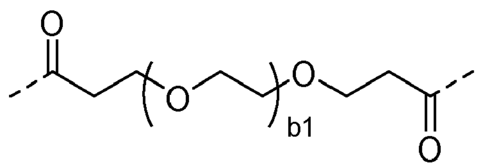
;

a1 represents an integer from 1 to 10;
b1 represents an integer from 1 to 4; and
carrier protein is selected from detoxified pneumolysin, or an immunogenic fragment thereof, and pneumococcal surface protein A, or an immunogenic fragment thereof.

$-L^8-$ is defined as a linker and is part of the fragment $-O-L^8-NH-$. Thus, the linker $-L^8-$ is bound to an oxygen atom and to the nitrogen atom of the $-NH-W^1-$ fragment. It is preferred that at least two carbon atoms of the linker are between the oxygen atom and the $-NH-W^1-$ fragment, like $-O-C-C-NH-W^1-$. The linker $-L^8-$ can be an aliphatic chain, wherein the aliphatic chain can optionally include an aromatic chain inserted in it, or a number of heteroatoms oscillating from 0 to 10. The linker $-L^8-$ preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms. The shortest atom chain between the oxygen atom (i.e. the oxygen of $-O-L^1-NH_2$) and the $-NH-W^1-$ fragment consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the $-NH-W^1-$ fragment) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker $-L^8-$ or the shortest chain is fully or partially fluorinated. The linker $-L^8-$ may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker $-L^8-$ may also contain amide (-NH-CO-, -CO-NH-) and/or urea (-NH-CO-NH-) residues and preferably only one amide or urea residue. The linker may also contain substituents and preferably two substituents, such as $R^{10}$ and $R^{11}$, or four substituents such as $R^{10}$, $R^{11}$, $R^{15}$, and $R^{14}$, which have the meanings as defined herein and which are preferably selected from: -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$, and $-N(C_2H_5)_2$.

In case the linker $-L^8-$ is fluorinated, more than two substituents -F are preferred.

Linker $-L^8-$ is preferably selected from: $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-CF_2-$, $-(CF_2)_2-$, $-(CF_2)_3-$, $-(CF_2)_4-$, $-(CF_2)_5-$, $-(CF_2)_6-$, $-(CF_2)_7-$, $-(CF_2)_8-$, $-(CF_2)_9-$, $-(CF_2)_{10}-$, $-(CH_2)_2-O-(CH_2)_2-$, $-CH_2-O-(CH_2)_3-$, $-(CH_2)_3-O-CH_2-$ $-CH_2-O-(CH_2)_2-$, $-(CH_2)_2-O-CH_2-$, $-(CH_2)_3-O-(CH_2)_2-$, $-(CH_2)_2-O-(CH_2)_3-$, $-(CH_2)_4-O-CH_2-$ $-CH_2-O-(CH_2)_4-$, $-L^a-$, $-L^a-L^e-$, $-L^a-L^b-L^e-$, $-L^a-L^b-L^d-L^c-L^e-$, $-L^a-L^d-L^e-$;
wherein
$-L^a-$ is selected from: $-(CH_2)_o-$, $-(CF_2)_o-$, $-(CH_2-CH_2-O)_o-C_2H_4-$, $-(CH_2-CH_2-O)_o-CH_2-$, $-(CR^{10}R^{11})_o-$,

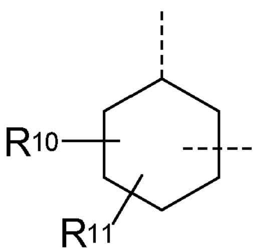 ,
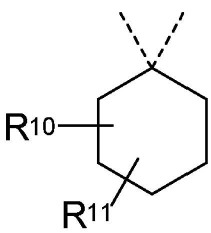 ,
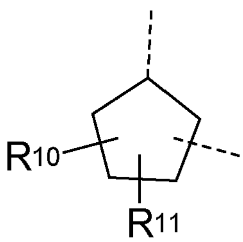 ,
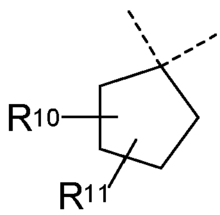 ,

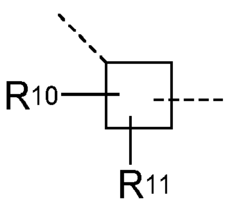 , and
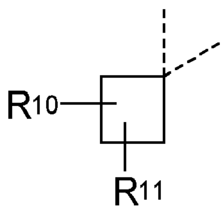 ;

$-L^b-$ and $-L^c-$ are independently of each other selected from: -O-, -NH-C(O)-NH-, -NH-C(S)-NH- -NH-C(O)-, -C(O)-NH-, -NH-C(O)-O-, $-NR^9-$, $-NR^{18}-$, $-SO_2-$,

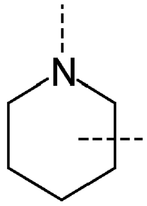,
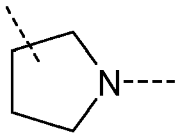,
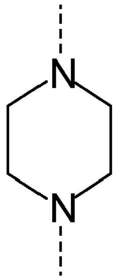,
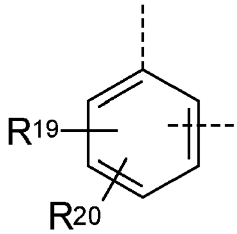,

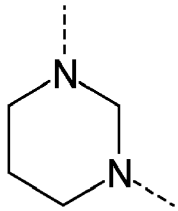,
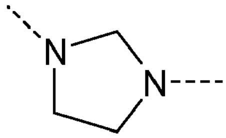,
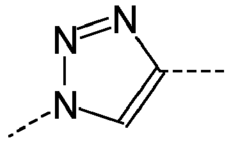,
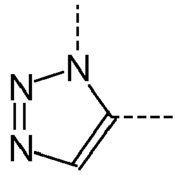,

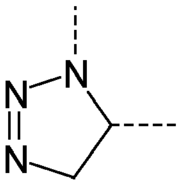,
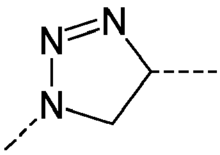,
And
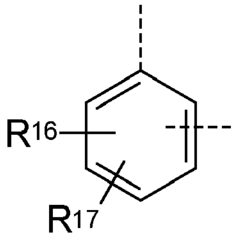;

$-L^d-$ represents: $-(CH_2)_q-$, $-(CF_2)_{q}-$, $-(CR^{12}R^{13})_q-$, $-(CH_2-CH_2-O)_q-C_2H_4-$ $-(CH_2-CH_2-O)_q-CH_2-$,

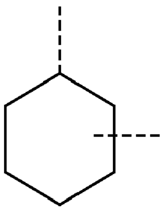,
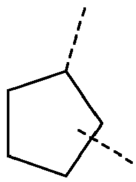,
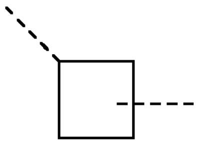,
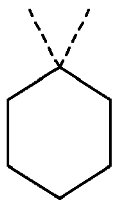,

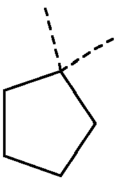,
and
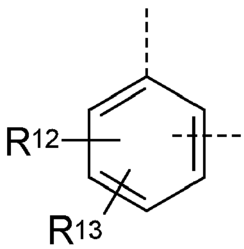;

$-L^e-$ is selected from: $-(CH_2)_{p1}-$, $-(CF_2)_{p1}-$, $-C_2H_4-(O-CH_2-CH_2)_{p1}-$, $-CH_2-(O-CH_2-CH_2)_{p1}-$ $-(CH_2)_{p1}-O-(CH_2)_{p2}-$ $-(CR^{14}R^{15})_{p1}-$, $-(CR^{14}R^{15})_{p1}-O-(CR^{21}R^{22})_{p2}-$

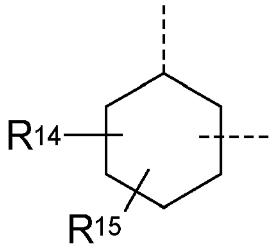 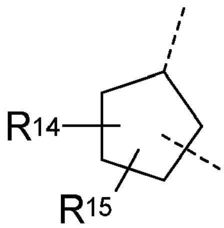 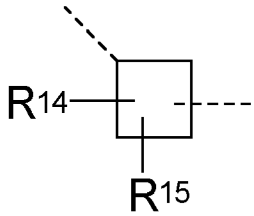 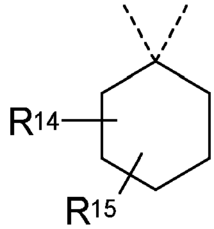

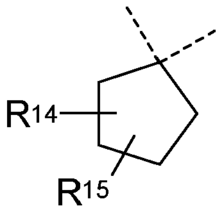 and 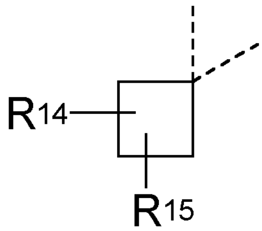 ;

$R^9$ and $R^{18}$ are independently of each other selected from: $-CH_3$, $-C_2H_5$, $-C_3H_7$, and $-C(O)CH_3$;
$R^{10}$, $R^{11}$, $R^{12}$, $R^{13}$, $R^{14}$, $R^{15}$, $R^{16}$, $R^{17}$, $R^{19}$, $R^{20}$, $R^{21}$ and $R^{22}$ are independently of each other selected from: -H, -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$ and $-N(C_2H_5)_2$;
o, q, p1 and p2 are independently of each other an integer selected from 1, 2, 3, 4, 5, 6, 7, 8, 9, and 10.

A spacer as used herein is preferably bifunctional. The spacer is optionally heterobifunctional or homobifunctional, having for example a reactive amino group and a reactive carboxylic acid group, 2 reactive amino groups or two reactive carboxylic acid groups. The spacer has for example between 4 and 20, 4 and 12, 5 and 10 carbon atoms. A possible spacer is adipic acid dihydrazide (ADH). Other spacers include B-propionamido, nitrophenyl-ethylamine, haloalkyl halides, glycosidic linkages, hexane diamine and 6-aminocaproic acid.

## PREPARATION OF CONJUGATED CAPSULAR POLYSACCHARIDES

The capsular polysaccharide can be conjugated to a carrier protein by using any known coupling technique. The conjugation method may rely on activation of the saccharide with 1-cyano-4-dimethylamino pyridinium tetrafluoroborate (CDAP) to form a cyanate ester. The activated saccharide may thus be coupled directly or *via* a spacer group to an amino group on the carrier protein. For example, the spacer could be cystamine or cysteamine to give a thiolated polysaccharide, which could be coupled to the carrier *via* a thioether linkage obtained after reaction with a maleimide-activated carrier protein (for example using GMBS) or a haloacetylated carrier protein (for example using iodoacetimide [e.g. ethyl iodoacetimide HCl] or N-succinimidyl bromoacetate or SIAB, or SIA, or SBAP). Preferably, the cyanate ester (optionally made by CDAP chemistry) is coupled with hexane diamine or ADH and the amino-derivatised saccharide is conjugated to the carrier protein using carbodiimide (e.g. EDAC or EDC) chemistry via a carboxyl group on the protein carrier. Such conjugation methods are described in WO 93/15760, WO 95/08348 and WO 96/29094.

Other suitable conjugation techniques rely on the use of carbodiimides, hydrazides, active esters, norborane, *p*-nitrobenzoic acid, *N*-hydroxysuccinimide, S-NHS (N-hydroxysulfosuccinimide), EDC, TSTU (2-succinimide-1,1,3,3-tetramethyluronium tetrafluoroborate) (WO 98/42721). Conjugation may involve a carbonyl linker which may be formed by reaction of a free hydroxyl group of the saccharide with CDI (1,1'-carbonyldiimidazole) followed by reaction with a protein to form a carbamate linkage. This may involve reduction of the anomeric terminus to a primary hydroxyl group, optional protection/deprotection of the primary hydroxyl group, reaction of the primary hydroxyl group with CDI to form a CDI carbamate intermediate and coupling the CDI carbamate intermediate with an amino group on a protein.

The conjugates can also be prepared by direct reductive amination methods as described in US 4673574. Other preparation methods are described in EP0161188, EP208375 and EP0477508.

A further method involves the coupling of a cyanogen bromide (or CDAP) activated saccharide derivatised with adipic acid dihydrazide (ADH) to the protein carrier by carbodiimide condensation, for example using EDAC.

In an embodiment, a hydroxyl group (preferably an activated hydroxyl group for example a hydroxyl group activated to make a cyanate ester [e.g. with CDAP]) on a saccharide is linked to an amino or carboxylic group on a protein either directly or indirectly (through a spacer). Where a spacer is present, a hydroxyl group on a saccharide is preferably linked to an amino group on a linker, for example by using CDAP conjugation. A further amino group in the linker for example ADH) may be conjugated to a carboxylic acid group on a protein, for example by using carbodiimide chemistry, for example by using EDAC. In an embodiment, the pneumococcal capsular saccharide(s) is conjugated to

the spacer first before the spacer is conjugated to the carrier protein. Alternatively, the spacer may be conjugated to the carrier before conjugation to the saccharide.

A combination of techniques may also be used, with some conjugates being prepared by CDAP, and some by reductive amination.

In general the following types of chemical groups on a protein carrier can be used for conjugation:

- Carboxyl (for instance *via* aspartic acid or glutamic acid). In one embodiment this group is linked to amino groups on saccharides directly or to an amino group on a spacer with carbodiimide chemistry e.g. with EDAC.

  - Amino group (for instance *via* lysine). In one embodiment this group is linked to carboxyl groups on saccharides directly or to a carboxyl group on a spacer with carbodiimide chemistry e.g. with EDAC. In another embodiment this group is linked to hydroxyl groups activated with CDAP or CNBr on saccharides directly or to such groups on a spacer; to saccharides or spacers having an aldehyde group; to saccharides or spacers having a succinimide ester group.
  - Sulfhydryl (for instance *via* cysteine). In one embodiment this group is linked to a bromo or chloro acetylated saccharide or linker with maleimide chemistry. In one embodiment this group is activated/modified with bis diazobenzidine.
  - Hydroxyl group (for instance *via* tyrosine). In one embodiment this group is activated/modified with bis diazobenzidine.
  - Imidazolyl group (for instance *via* histidine). In one embodiment this group is activated/modified with bis diazobenzidine.
  - Guanidyl group (for instance *via* arginine).
  - Indolyl group (for instance *via* tryptophan).

On a saccharide, in general the following groups can be used for conjugation: OH, COOH or $NH_2$. Aldehyde groups can be generated after different treatments known in the art such as: periodate, acid hydrolysis, hydrogen peroxide, etc. Examples of direct coupling approaches include, but are not limited to:

CPS-OH + CNBr or CDAP → CPS-cyanate ester + $NH_2$-carrier protein → conjugate;
CPS-aldehyde + $NH_2$-carrier protein → Schiff base + $NaCNBH_3$ → conjugate;
CPS-COOH + $NH_2$-carrier protein + EDAC → conjugate;
CPS-$NH_2$ + carrier protein-COOH + EDAC → conjugate;

Indirect coupling *via* spacer approaches include, but are not limited to:

CPS-OH + CNBr or CDAP → CPS-cyanate ester + $NH_2$-$NH_2$ → CPS-$NH_2$ + COOH-carrier protein + EDAC → conjugate;
CPS-OH + CNBr or CDAP → CPS-cyanate ester + $NH_2$-SH → CPS-SH + SH-carrier protein (native protein with an exposed cysteine or obtained after modification of amino groups of the protein by SPDP for instance) → CPS-S-S-carrier protein;
CPS-OH + CNBr or CDAP → CPS-cyanate ester + $NH_2$-SH → CPS-SH + maleimide-carrier protein (modification of amino groups) → conjugate;
CPS-OH + CNBr or CDAP → CPS-cyanate ester + $NH_2$-SH → CPS-SH + haloacetylated-carrier protein → conjugate;
CPS-COOH + EDAC + $NH_2$-$NH_2$ → CPS-$NH_2$ + EDAC + COOH-carrier protein → conjugate;
CPS-COOH + EDAC+ $NH_2$-SH → CPS-SH + SH-carrier protein (native protein with an exposed cysteine or obtained after modification of amino groups of the protein by SPDP for instance) → saccharide-S-S-carrier protein;
CPS-COOH + EDAC+ $NH_2$-SH → CPS-SH + maleimide-carrier protein (modification of amino groups) → conjugate;
CPS-COOH + EDAC + $NH_2$-SH → CPS-SH + haloacetylated-carrier protein → conjugate;
CPS-aldehyde + $NH_2$-$NH_2$ → CPS-$NH_2$ + EDAC + carrier protein-COOH → conjugate.

In a preferred embodiment, CDAP (1-cyano-dimethylaminopyridinium tetrafluoroborate) cyanylating reagent is used for the synthesis of capsular polysaccharide-protein conjugates. This coupling method avoids hydrolysis of the alkaline sensitive polysaccharides and allows direct coupling to the carrier protein. The polysaccharide is dissolved in water or a saline solution. CDAP is dissolved in acetonitrile and added immediately to the polysaccharide solution. The CDAP reacts with the hydroxyl groups of the polysaccharide to form a cyanate ester in an activation step. Afterwards, the carrier protein is added. Amino groups of lysine react with the activated polysaccharide to form an isourea covalent linkage. After the conjugation reaction, a large excess of glycine is added to quench residual activated functions. The formed conjugate is then passed through a gel permeation to remove unreacted carrier protein and residual reagents.

Another preferred conjugation method is the reductive amination, wherein the capsular polysaccharide is oxidized with sodium periodate in an activation step and subsequently brought to reaction with the amino group of a carrier protein in the presence of sodium borohydride or sodium cyanoborohydride. The reductive amination can be performed in aqueous media or in DMSO. The crude conjugate is then passed through a HA or DEAE column and filtered sterile

Type of covalent conjugation

In an embodiment, the pneumococcal saccharide of the present invention is covalently linked (either directly or through a linker) to an amino acid residue of a carrier protein. In an embodiment, the pneumococcal saccharide is covalently linked to a carrier protein through a chemical linkage obtainable using a chemical conjugation method, optionally selected from the group consisting of carbodiimide chemistry, reductive animation, cyanylation chemistry (for example CDAP chemistry), maleimide chemistry, hydrazide chemistry, ester chemistry, and N-hydroxysuccinimide chemistry either directly or via a linker. As used herein, the term **"directly linked"** means that the two entities are connected via a chemical bond, preferably a covalent bond. As used herein, the term **"indirectly linked"** means that the two entities are connected via a linking moiety (as opposed to a direct covalent bond). In certain embodiments the linker is adipic acid dihydrazide. In an embodiment, the chemical conjugation method is selected from the group consisting of carbodiimide chemistry, reductive animation, cyanylation chemistry (for example CDAP chemistry), maleimide chemistry, hydrazide chemistry, ester chemistry, and Nhydroysuccinimide chemistry. Conjugates can be prepared by direct reductive amination methods as described in, US20071 0 184072 (Hausdorff) US 4365170 (Jennings) and US 4673574 (Anderson). Other methods are described in EP-0-161-188, EP-208375 and EP-0-477508. The conjugation method may alternatively rely on activation of the pneumococcal saccharide with 1-cyano-4-dimethylamino pyridinium tetrafluoroborate (CDAP) to form a cyanate ester. Such conjugates are described in PCT published application WO 93/15760 Uniformed Services University and WO 95/08348 and WO 96/29094. See also Chu C. et al. Infect. Immunity, 1983, pages 245-256.

In general the following types of chemical groups on a carrier protein can be used for conjugation:

A) Carboxyl (for instance via aspartic acid or glutamic acid). In one embodiment this group is linked to amino groups on saccharides directly or to an amino group on a linker with carbodiimide chemistry e.g. with EDAC.

B) Amino group (for instance via lysine). In one embodiment this group is linked to carboxyl groups on saccharides directly or to a carboxyl group on a linker with carbodiimide chemistry e.g. with EDAC. In another embodiment this group is linked to hydroxyl groups activated with CDAP or CNBr on saccharides directly or to such groups on a linker; to saccharides or linkers having an aldehyde group; to saccharides or linkers having a succinimide ester group.

C) Sulphydryl (for instance via cysteine). In one embodiment this group is linked to a bromo or chloro acetylated saccharide or linker with maleimide chemistry. In one embodiment this group is activated/modified with bis diazobenzidine.

D) Hydroxyl group (for instance via tyrosine). In one embodiment this group is activated/modified with bis diazobenzidine.

E) Imidazolyl group (for instance via histidine). In one embodiment this group is activated/modified with bis diazobenzidine.

F) Guanidyl group (for instance via arginine).

G) Indolyl group (for instance via tryptophan). On a saccharide, in general the following groups can be used for a coupling: OH, COOH or $NH_2$. Aldehyde groups can be generated after different treatments such as: periodate, acid hydrolysis, hydrogen peroxide, etc.

**Immunogenic Compositions**

The pneumococcal conjugates of the invention are particularly suited for inclusion in **immunogenic compositions** and **vaccines**. The present invention provides an immunogenic composition comprising the conjugate of the invention.

In accordance with one embodiment, the present invention is directed to an immunogenic composition comprising one or more conjugates of a synthetic *Streptococcus pneumoniae* oligosaccharide with a carrier protein selected from detoxified pneumolysin or an immunogenic fragment thereof, and pneumococcal surface protein A, or an immunogenic fragment thereof.

In accordance with one particular embodiment, the present invention is directed to an immunogenic composition comprising (i) a conjugate of a synthetic *Streptococcus pneumoniae* oligosaccharide with detoxified pneumolysin or an immunogenic fragment thereof, and (ii) a conjugate of a synthetic *Streptococcus pneumoniae* oligosaccharide with pneumococcal surface protein A or an immunogenic fragment thereof.

In accordance with one more particular embodiment, the present invention is directed to an immunogenic composition comprising a conjugate of *Streptococcus pneumoniae* serotype 3, and a mixture consisting of capsular polysaccharides from *Streptococcus pneumoniae* serotypes 1, 4, 5, 6A, 6B, 7F, 9V, 14, 18C, 19A, 19F and 23F individually conjugated to $CRM_{197}$ carrier protein.

In accordance with one still more particular embodiment, the present invention is directed to an immunogenic composition comprising a conjugate of *Streptococcus pneumoniae* serotype 3, and a mixture consisting of capsular polysaccharides from *Streptococcus pneumoniae* serotypes 1, 4, 5, 6A, 6B, 7F, 9V, 14, 18C, 19A, 19F and 23F individually conjugated to $CRM_{197}$ carrier protein, wherein the conjugate has the general formula (**I**)

$$[H\text{-}(C)_{c3}\text{-}(D\text{-}C)_{c2}\text{-}(D)_{c1}\text{-}O\text{-}L^3\text{-}NH\text{-}W^3]_{m3}\text{-carrier protein} \qquad (\mathbf{I})$$

wherein

C represents: 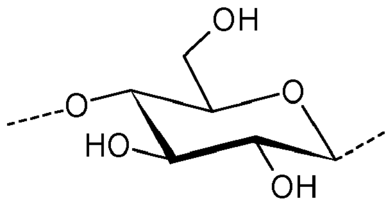

D represents: 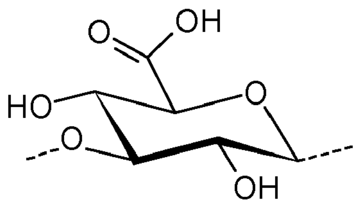

c2 is an integer selected from 1, 2, 3, 4, 5, 6, 7, 8, 9 and 10;
c1 and c3 represent independently of each other an integer which is 0 or 1 ;
$L^3$ represents a linker;
m3 is comprised between about 2 and about 18;
$-W^3-$ is selected from:

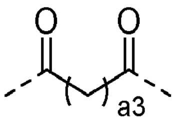 , 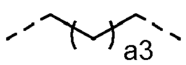 , and 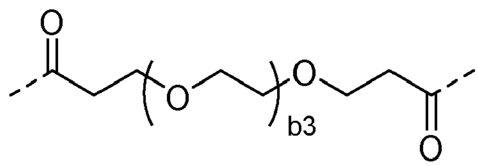 ;

a3 represents an integer from 1 to 10;
b3 represents an integer from 1 to 4; and
carrier protein is selected from detoxified pneumolysin or an immunogenic fragment thereof, and pneumococcal surface protein A, or an immunogenic fragment thereof.

In accordance with another particular embodiment, the present invention is directed to an immunogenic composition comprising one or more conjugates of a synthetic *Streptococcus pneumoniae* oligosaccharide with a carrier protein selected from detoxified pneumolysin or an immunogenic fragment thereof, and pneumococcal surface protein A, or an immunogenic fragment thereof,
wherein the one or more conjugates are selected from a conjugate of a synthetic *Streptococcus pneumoniae* serotype 1 oligosaccharide, a conjugate of a synthetic *Streptococcus pneumoniae* serotype 2 oligosaccharide, a conjugate of a synthetic *Streptococcus pneumoniae* serotype 3 oligosaccharide, a conjugate of a synthetic *Streptococcus pneumoniae*

serotype 4 oligosaccharide, a conjugate of a synthetic *Streptococcus pneumoniae* serotype 5 oligosaccharide, a conjugate of a synthetic *Streptococcus pneumoniae* serotype 8 oligosaccharide.

Immunogenic compositions comprise an immunologically effective amount of the pneumococcal saccharide conjugate of the invention, as well as any other components. By **"immunologically effective amount"**, it is meant that the administration of that amount to an individual, either as a single dose or as part of a series is effective for treatment or prevention. This amount varies depending on the health and physical condition of the individual to be treated, age, the degree of protection desired, the formulation of the vaccine and other relevant factors. It is expected that the amount will fall in a relatively broad range that can be determined through routine trials. In an embodiment, the immunogenic composition of the invention further comprises a pharmaceutically acceptable excipient, carrier or diluent.

As used herein, the term "**effective amount**," in the context of administering a therapy (e.g. an immunogenic composition or vaccine of the invention) to a subject refers to the amount of a therapy which has a prophylactic and/or therapeutic effect(s). In certain embodiments, an "effective amount" refers to the amount of a therapy which is sufficient to achieve one, two, three, four, or more of the following effects: (i) reduce or ameliorate the severity of a bacterial infection or symptom associated therewith; (ii) reduce the duration of a bacterial infection or symptom associated therewith; (iii) prevent the progression of a bacterial infection or symptom associated therewith; (iv) cause regression of a bacterial infection or symptom associated therewith; (v) prevent the development or onset of a bacterial infection, or symptom associated therewith; (vi) prevent the recurrence of a bacterial infection or symptom associated therewith; (vii) reduce organ failure associated with a bacterial infection; (viii) reduce hospitalization of a subject having a bacterial infection; (ix) reduce hospitalization length of a subject having a bacterial infection; (x) increase the survival of a subject with a bacterial infection; (xi) eliminate a bacterial infection in a subject; (xii) inhibit or reduce a bacterial replication in a subject; and/or (xiii) enhance or improve the prophylactic or therapeutic effect(s) of another therapy.

As used herein, the term "**subject**" refers to an animal, in particular a mammal such as a primate (e.g. human).

**Pharmaceutically acceptable excipients and carriers** can be selected by those of skill in the art. For example, the pharmaceutically acceptable excipient or carrier can include a buffer, such as Tris (trimethamine), phosphate (e.g. sodium phosphate), acetate, borate (e.g. sodium borate), citrate, glycine, histidine and succinate (e.g. sodium succinate), suitably sodium chloride, histidine, sodium phosphate or sodium succinate. The pharmaceutically acceptable excipient may include a salt, for example sodium chloride, potassium chloride or magnesium chloride. Optionally, the pharmaceutically acceptable excipient contains at least one component that stabilizes solubility and/or stability. Examples of solubilizing/stabilizing agents include detergents, for example, laurel sarcosine and/or polysorbate (e.g. TWEEN 80 (Polysorbate-80)). Examples of stabilizing agents also include poloxamer (e.g. poloxamer 124, poloxamer 188, poloxamer 237, poloxamer 338 and poloxamer 407). The pharmaceutically acceptable excipient may include a non-ionic surfactant, for example polyoxyethylene sorbitan fatty acid esters, TWEEN 80 (Polysorbate-80), TWEEN 60 (Polysorbate-60), TWEEN 40 (Polysorbate-40) and TWEEN 20 (Polysorbate-20), or polyoxyethylene alkyl ethers (suitably polysorbate-80). Alternative solubilizing/stabilizing agents include arginine, and glass forming polyols (such as sucrose, trehalose and the like). The pharmaceutically excipient may be a preservative, for example phenol, 2-phenoxyethanol, or thiomersal. Other pharmaceutically acceptable **excipients** include sugars (e.g. lactose, sucrose), and proteins (e.g. gelatine and albumin).

Pharmaceutically acceptable **carriers** include water, saline solutions, aqueous dextrose and glycerol solutions. Numerous pharmaceutically acceptable excipients and carriers are described, for example, in Remington's Pharmaceutical Sciences, by E. W. Martin, Mack Publishing Co. Easton, PA, 5th Edition (975). Immunogenic compositions of the invention may also contain **diluents** such as water, saline, glycerol etc. Additionally, auxiliary substances, such as wetting or emulsifying agents, pH buffering substances, polyols and the like may be present. In an embodiment, the immunogenic composition of the invention additionally comprises one or more buffers, e.g. phosphate buffer and/or sucrose phosphate glutamate buffer. In other embodiments, the immunogenic composition of the invention does not comprise a buffer. In an embodiment, the immunogenic composition of the invention additionally comprises one or more salts, e.g. sodium chloride, calcium chloride, sodium phosphate, monosodium glutamate, and aluminum salts (e.g. aluminum hydroxide, aluminum phosphate, alum (potassium aluminum sulfate), or a mixture of such aluminum salts). In other embodiments, the immunogenic composition of the invention does not comprise a salt.

In an embodiment, the immunogenic composition of the invention comprises 10 or more, 11 or more, 12 or more, 13 or more, 14 or more, 15 or more, 16 or more, 17 or more, 18 or more, 19 or more, 20 or more synthetic saccharide conjugates from different *S. pneumoniae* serotypes. The immunogenic composition of the invention may comprise 10, 11, 12, 13, 14, 15, 16, 17, 18, 19, 20, 21, 22, 23, 24, or 25 synthetic saccharide conjugates from different *S. pneumoniae* serotypes.

The immunogenic compositions of the invention may also comprise *S. pneumoniae* capsular saccharides individually conjugated to a carrier protein. The S. pneumoniae capsular saccharides individually conjugated to a carrier protein may be selected from a Streptococcus pneumoniae serotypes 1, 2, 3, 4, 5, 6A, 6B, 7A, 7B, 7C, 8, 9A, 9L, 9N, 9V, 10A, 10B, 10C, 10F, 11A, 11 B, 11C, 11D, 11F, 12A, 12B, 12F, 13, 14, 15A, 15B, 15C, 15F, 16A, 16F, 17A, 17F, 18A, 18B, 18C, 18F, 19A, 19B, 19C, 19F, 20, 21, 22A, 22F, 23A, 23B, 23F, 24A, 24B, 24F, 25A, 25F, 26, 27, 28A, 28F,

29, 31, 32A, 32F, 33A, 33B, 33C, 33D, 33F, 34, 35A, 35B, 35C, 35D, 35F, 36, 37, 38, 39, 40, 41A, 41F, 42, 43, 44, 45, 46, 47A, 47F or 48 capsular saccharide. Preferably, the *S. pneumoniae* capsular saccharides individually conjugated to a carrier protein are selected from a *Streptococcus pneumoniae* serotypes 1, 4, 5, 6A, 6B, 7F, 9V, 14, 18C, 19A, 19F, and 23F. In an embodiment, at least four serotypes are included in the composition, e.g. 6B, 14, 19F and 23F (suitably conjugated to a carrier protein). In another embodiment, at least 7 serotypes are included in the composition, e.g. 4, 6B, 9V, 14, 18C, 19F and 23F (suitably conjugated to a carrier protein). In another embodiment the immunogenic composition comprises 10 or more, 11 or more, 12 or more, 13 or more, 14 or more, 15 or more, 16 or more, 17 or more, 19 or more, or 20 capsular saccharides from different S. pneumoniae serotypes (suitably conjugated to a carrier protein). In an embodiment the immunogenic composition comprises 10 to 23 capsular saccharides from different S. pneumoniae serotypes (suitably conjugated to a carrier protein). In an embodiment, the vaccine may be an 11-valent vaccine. For example, a 11-valent vaccine may comprise saccharides from serotypes 1, 4, 5, 6A, 6B, 7F, 9V, 14, 18C, 19F and 23F. In an embodiment, the vaccine may be an 12-valent or 13-valent vaccine. A 12 or 13-valent pediatric (infant) vaccine may also include the 11 valent formulation supplemented with serotypes 19A, or 22F, whereas a 13-valent elderly vaccine may include the 11 valent formulation supplemented with serotypes 19A and 22F, 8 and 12F, or 8 and 15, or 8 and 19A, or 8 and 22F, or 12F and 15, or 12F and 19A, or 12F and 22F, or 15 and 19A, or 15 and 22F. In an embodiment, the vaccine may be a 14-valent or 15-valent vaccine. A 14 or 15-valent pediatric vaccine may include the 11 valent formulation described above supplemented with serotypes 3, 19A and 22F; serotypes 8, 19A and 22F; serotypes 12F, 19A and 22F; serotypes 15, 19A and 22F; serotypes 3, 8, 19A and 22F; serotypes 3, 12F, 19A and 22F; serotypes 3, 15, 19A and 22F. In an embodiment, the vaccine may be a 16-valent vaccine. A 16 valent vaccine may include the 11 valent formulation described above supplemented with serotypes 3, 15B, 19A, 22F and 23F. A 16 valent vaccine may include the 11 valent formulation described above supplemented with serotypes 3, 15B, 19A, 22F and 33F. In an embodiment, the vaccine may be a 19-valent vaccine. A 19 valent vaccine may include the 11 valent formulation described above supplemented with serotypes 8, 10A, 11A, 12F, 15B, 19A, 22F and 23F. A 19 valent vaccine may include the 11 valent formulation described above supplemented with serotypes 8, 10A, 11A, 12F, 15B, 19A, 22F and 33F. In an embodiment, the vaccine may be a 20-valent vaccine. A 20 valent vaccine may include the 11 valent formulation described above supplemented with serotypes 3, 8, 10A, 11A, 12F, 15B, 19A, 22F and 23F. A 20 valent vaccine may include the 11 valent formulation described above supplemented with serotypes 3, 8, 10A, 11A, 12F, 15B, 19A, 22F and 33F. In an embodiment, the vaccine may be a 21-valent vaccine. In an embodiment, the vaccine may be a 22-valent vaccine. In an embodiment, the vaccine may be a 23-valent vaccine. In an embodiment, the vaccine may be a 24-valent vaccine. In an embodiment, the vaccine may be a 25-valent vaccine.

IMMUNOGENIC COMPOSITIONS WITH ADDITIONAL ANTIGENS

The immunogenic composition of the invention may optionally further comprise additional antigens. Examples of such additional antigens are *S. pneumoniae* proteins selected from pneumococcal virulence factors, such as pneumococcal surface protein A (PspA), and detoxified pneumolysin, dPly, or an immunogenic fragment of the aforementioned.

In accordance with a particular embodiment, the present invention is directed to an immunogenic composition comprising one or more conjugates of a synthetic *Streptococcus pneumoniae* oligosaccharide with a carrier protein selected from detoxified pneumolysin or an immunogenic fragment thereof, and pneumococcal surface protein A, or an immunogenic fragment thereof, and further comprising at least one unconjugated *Streptococcus pneumoniae* protein selected from detoxified pneumolysin or an immunogenic fragment thereof, and pneumococcal surface protein A, or an immunogenic fragment thereof.

In accordance with one particular embodiment, the present invention is directed to an immunogenic composition comprising (i) a conjugate of a synthetic *Streptococcus pneumoniae* oligosaccharide with detoxified pneumolysin or an immunogenic fragment thereof, and (ii) a conjugate of a synthetic *Streptococcus pneumoniae* oligosaccharide with pneumococcal surface protein A or an immunogenic fragment thereof, and further comprising at least one unconjugated *Streptococcus pneumoniae* protein selected from detoxified pneumolysin or an immunogenic fragment thereof, and pneumococcal surface protein A, or an immunogenic fragment thereof.

In accordance with one more particular embodiment, the present invention is directed to an immunogenic composition comprising a conjugate of *Streptococcus pneumoniae* serotype 3, and a mixture consisting of capsular polysaccharides from *Streptococcus pneumoniae* serotypes 1, 4, 5, 6A, 6B, 7F, 9V, 14, 18C, 19A, 19F and 23F individually conjugated to $CRM_{197}$ carrier protein, and further comprising at least one unconjugated *Streptococcus pneumoniae* protein selected from detoxified pneumolysin or an immunogenic fragment thereof, and pneumococcal surface protein A, or an immunogenic fragment thereof.

In accordance with one still more particular embodiment, the present invention is directed to an immunogenic composition comprising a conjugate of *Streptococcus pneumoniae* serotype 3, and a mixture consisting of capsular polysaccharides from *Streptococcus pneumoniae* serotypes 1, 4, 5, 6A, 6B, 7F, 9V, 14, 18C, 19A, 19F and 23F individually

conjugated to $CRM_{197}$ carrier protein,
wherein the conjugate has the general formula (**I**)

$$[H\text{-}(C)_{c3}\text{-}(D\text{-}C)_{c2}\text{-}(D)_{c1}\text{-}O\text{-}L^3\text{-}NH\text{-}W^3]_{m3}\text{-carrier protein} \qquad (I)$$

wherein

C represents: 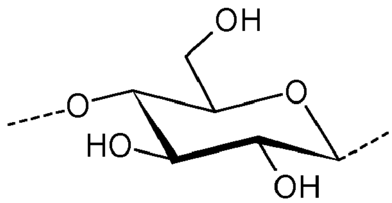

D represents: 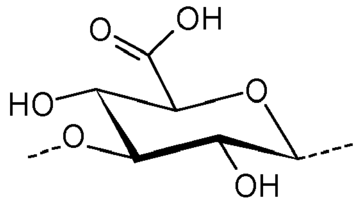

c2 is an integer selected from 1, 2, 3, 4, 5, 6, 7, 8, 9 and 10;
c1 and c3 represent independently of each other an integer which is 0 or 1 ;
$L^3$ represents a linker;
m3 is comprised between about 2 and about 18;
$-W^3-$ is selected from:

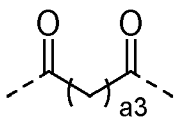 , 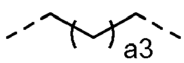 , and 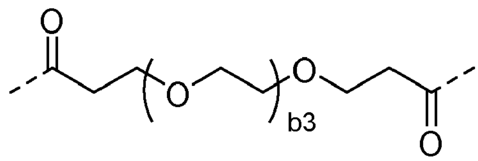 ;

a3 represents an integer from 1 to 10;
b3 represents an integer from 1 to 4; and
carrier protein is selected from detoxified pneumolysin or an immunogenic fragment thereof, and pneumococcal surface protein A, or an immunogenic fragment thereof,

and further comprising at least one unconjugated *Streptococcus pneumoniae* protein selected from detoxified pneumolysin or an immunogenic fragment thereof, and pneumococcal surface protein A, or an immunogenic fragment thereof.

In accordance with another particular embodiment, the present invention is directed to an immunogenic composition comprising one or more conjugates of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the one or more conjugates are selected from a conjugate of a synthetic *Streptococcus pneumoniae* serotype 1 oligosaccharide, a conjugate of a synthetic *Streptococcus pneumoniae* serotype 2 oligosaccharide, a conjugate of a synthetic *Streptococcus pneumoniae* serotype 3 oligosaccharide, a conjugate of a synthetic *Streptococcus pneumoniae* serotype 4 oligosaccharide, a conjugate of a synthetic *Streptococcus pneumoniae* serotype 5 oligosaccharide, a conjugate of a synthetic *Streptococcus pneumoniae* serotype 8 oligosaccharide, and further comprising at least one unconjugated *Streptococcus pneumoniae* protein selected from detoxified pneumolysin or an immunogenic fragment thereof, and pneumococcal surface protein A, or an immunogenic fragment thereof.

**USES**

It was found that immunization of mice with conjugates of synthetic *S. pneumoniae* serotype 3 (ST3) oligosaccharides with detoxified pneumolysin was able to raise a protective immune response.

Indeed, it was found the mice immunized with ST3-tetrasaccharide detoxified pneumolysin conjugate showed specific antibodies against ST3-tetrasaccharide and ST3-capsular polysaccharide at levels comparable as mice immunized with ST3-tetrasaccharide $CRM_{197}$ conjugate (**Figure 5a, 5b**).

In accordance, mice immunized with ST3-tetrasaccharide detoxified pneumolysin showed a reduced disease severity after infection with *S. pneumoniae* serotype 3 (**Figure 4 a-d**).

Moreover, ST2-hexasaccharide or ST3-tetrasaccharide conjugates with detoxified pneumolysin (dPly) or PspA were also able to protect mice against infection with *S. pneumoniae* serotype 2. In these experiments, mice were intranasally immunized with ST2-hexasaccharide or ST3-tetrasaccharide individually conjugated to detoxified pneumolysin (dPly) or PspA, or with ST2-hexasaccharide conjugated to dPly *plus* ST2-hexasaccharide conjugated to PspA, or with ST3-hexasaccharide conjugated to dPly *plus* ST3-hexasaccharide conjugated to PspA. After immunization, these mice were challenged with *S. pneumoniae* D39 serotype 2.

The immunization with the different conjugates triggered a systemic immune response in all tested mice. Antibodies against pneumolysin, PspA and *S. pneumoniae* serotype 3 capsular polysaccharide were produced accordingly to the vaccine received (**Figure 6**).

The development of mucosal immunity (level of IgA and IgG in nasal tissue) was evaluated after infection with *S. pneumoniae* D39 serotype 2. The immunized mice showed relatively high local immune response to *S.pneumoniae* serotype 3 capsular polysaccharide, synthetic ST3-tetrasaccharide and synthetic ST2-hexasaccharide, accordingly to the received immunization. However, mice injected with ST2-hexasaccharide glycoconjugate did not show the local antibody response cross-reactive to the *S.pneumoniae* serotype 2 capsular polysaccharide (**Figure 7**), as seen for the systemic immune response **(Figure 6**). Mucosal IgA and IgG antibodies specific for pneumolysin and PspA were also produced respectively to the vaccine received (**Figure 8**).

These results prove that the synthetic saccharide conjugates are immunogenic and able to trigger a local mucosal immune response, which is essential for colonization inhibition.

Importantly, the intranasal vaccination with ST2-hexasaccharide dPly conjugate, ST2-hexasaccharide PspA conjugate, composition of ST2-hexasaccharide dPly *plus* ST2-hexasaccharide PspA conjugates, ST3-tetrasaccharide dPly conjugate, ST3-tetrasaccharide PspA conjugate, and composition of ST3-tetrasaccharide dPly *plus* ST3-tetrasaccharide PspA conjugates induced a significant reduction of bacterial load in the nasal cavity compared to the control mice (**Figure 9**), showing induction of serotype cross-reactive protection by the ST3-tetrasaccharide conjugates.

As mice immunized with ST3-conjugates by intranasal route showed a systemic antibody response against ST3 tetrasaccharide and capsular polysaccharide, similarly to mice immunized with ST3-conjugates by subcutaneous route, immunization by intranasal route could substitute the current immunization by subcutaneous way in human as it is less painful and stressful especially for young children (non-invasive method). Intranasal vaccination may also trigger protection against infections at other mucosal sites, e.g., lungs, intestines or genital tract, and deliver cross-protection against different bacterial strains through mucosal antibody secretion.

Thus, the above findings show that conjugation of synthetic *S. pneumoniae* oligosaccharides to pneumococcus proteins pneumolysin and PspA gives both local protection against bacteria colonization (first step for pneumococcal infection) but also systemic defence by antibody-mediated opsonophagocytic clearance of the bacteria during the infection.

Furthermore, the inventive synthetic conjugates open new opportunities in the field of synthetic carbohydrate vaccines for veterinary use. Indeed, piglets can develop severe diseases such as sepsis, meningitis, pneumonia, endocarditis as well as arthritis upon infection with *Streptococcus suis.* Currently, infected animals are treated individually with a mixture of antibiotics which are very expensive and difficult to control. This leads to a high level of antimicrobial resistance (99.61% of multidrug resistance bacteria were found in pigs). Infection caused by these bacteria can be also transmitted from animal to humans (outbreak in 2005, Sichuan Province in China - 640 pigs and 39 people died from *Streptococcus suis* infection). Therefore, a lot of effort has been invested for developing *Streptococcus suis* vaccines for pigs.

Piglets are the natural host for *Streptococcus suis* which share many similarities in pathogenesis with *Streptococcus pneumoniae* infection in humans. Piglets could develop severe disease upon infection with *Streptococcus suis* like sepsis, meningitis, pneumonia, endocarditis as well as arthritis .

It was found that the inventive synthetic conjugates with pneumolysin are immunogenic also in pigs, as they induced production of antibodies specific for pneumolysin as well as for *S. pneumoniae* synthetic oligosaccharide or capsular polysaccharide (**Figures 10, 11**). The generated antibodies were able to kill pneumococci (**Figure 12**) and to neutralize the toxic effect of pneumolysin *in vitro* (**Figure 15**).

Finally it was found that also antibodies produced in mice immunized with ST3-tetrasaccharide pneumolysin conjugate were able to inhibit pneumolysin toxic effect on lung epithelial cell integrity (**Figure 14**). Thus, pneumolysin-neutralizing antibodies produced after immunization may block acute lung injury in the early course of pneumococcal pneumonia and following respiratory failure, suggesting that the ST3-tetrasaccharide pneumolysin conjugates may have additional beneficial effects in the prevention and treatment of severe pneumococcal pneumonia.

Thus, one embodiment of the invention also refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, **for use** or to an immunogenic composition comprising one or more of **said** conjugates for use in raising a protective immune response in a human and/or animal host.

One particular embodiment of the invention also refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, for use or to an immunogenic composition comprising one or more of **said** conjugates for use in the prevention and/or treatment of a disease caused by *Streptococcus pneumoniae.*

One more particular embodiment of the invention further refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, **for use** or to an immunogenic composition comprising one or more of **said** conjugates for use in the prevention and/or treatment of a disease caused by *Streptococcus pneumoniae* serotype 2 and/or serotype 3.

One further embodiment of the invention refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, **for** use in raising a protective immune response in a human and/or animal host, wherein the synthetic *Streptococcus pneumoniae* oligosaccharide is selected from the group of serotypes comprising serotype 1, 2, 3, 4, 5, 8.

One particular embodiment of the invention also refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, for use in the prevention and/or treatment of a disease caused by *Streptococcus pneumoniae,* wherein the synthetic *Streptococcus pneumoniae* oligosaccharide is selected from the group of serotypes comprising serotype 1, 2, 3, 4, 5, 8.

One more particular embodiment of the invention further refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, **for use** in the prevention and/or treatment of a disease caused by *Streptococcus pneumoniae* serotype 2 and/or serotype 3, wherein the synthetic *Streptococcus pneumoniae* oligosaccharide is selected from the group of serotypes comprising serotype 1, 2, 3, 4, 5, 8.

One further embodiment of the invention refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, **for** use in raising a protective immune response in a human and/or animal host, wherein the synthetic *Streptococcus pneumoniae* oligosaccharide is of *Streptococcus pneumoniae* serotype 3, and wherein the conjugate has the general formula (I)

$$\mathbf{[H\text{-}(C)_{c3}\text{-}(D\text{-}C)_{c2}\text{-}(D)_{c1}\text{-}O\text{-}L^3\text{-}NH\text{-}W^3]_{m3}\text{-}carrier\ protein} \qquad \mathbf{(I)}$$

wherein

C represents: 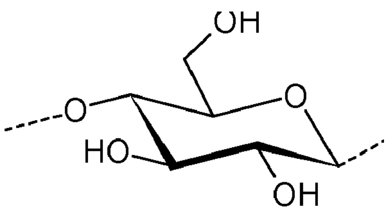

D represents: 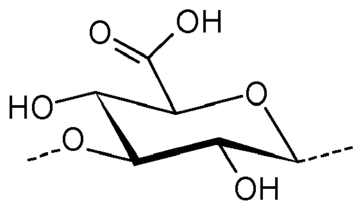

c2 is an integer selected from 1, 2, 3, 4, 5, 6, 7, 8, 9 and 10;
c1 and c3 represent independently of each other an integer which is 0 or 1 ;
$L^3$ represents a linker;

m3 is comprised between about 2 and about 18;
$-W^3-$ is selected from:

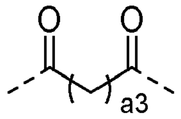
,
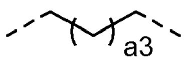
, and
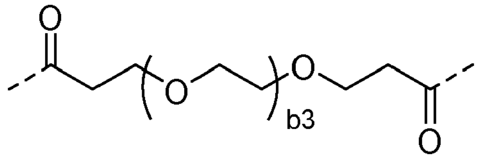
;

a3 represents an integer from 1 to 10;
b3 represents an integer from 1 to 4; and

carrier protein is selected from detoxified pneumolysin or an immunogenic fragment thereof, and pneumococcal surface protein A, or an immunogenic fragment thereof.

One particular embodiment of the invention also refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, for use in the prevention and/or treatment of a disease caused by *Streptococcus pneumoniae,* wherein the synthetic *Streptococcus pneumoniae* oligosaccharide is of *Streptococcus pneumoniae* serotype 3, and wherein the conjugate has the general formula (**I**)

$$\mathbf{[H\text{-}(C)_{c3}\text{-}(D\text{-}C)_{c2}\text{-}(D)_{c1}\text{-}O\text{-}L^3\text{-}NH\text{-}W^3]_{m3}\text{-}carrier\ protein} \qquad (\mathbf{I})$$

wherein

C represents:
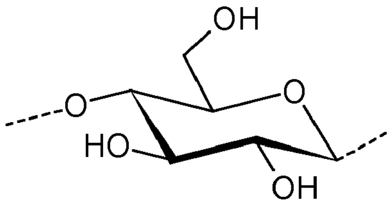

D represents:
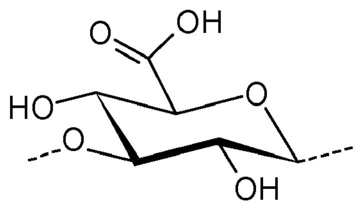

c2 is an integer selected from 1, 2, 3, 4, 5, 6, 7, 8, 9 and 10;
c1 and c3 represent independently of each other an integer which is 0 or 1;
$L^3$ represents a linker;
m3 is comprised between about 2 and about 18;
$-W^3-$ is selected from:

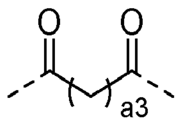
,
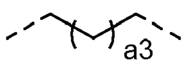
, and
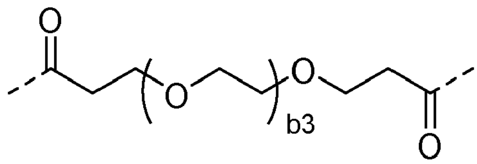
;

a3 represents an integer from 1 to 10;
b3 represents an integer from 1 to 4; and

carrier protein is selected from detoxified pneumolysin or an immunogenic fragment thereof, and pneumococcal surface

protein A, or an immunogenic fragment thereof.

One more particular embodiment of the invention further refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, **for use** in the prevention and/or treatment of a disease caused by *Streptococcus pneumoniae* serotype 2 and/or serotype 3, wherein the synthetic *Streptococcus pneumoniae* oligosaccharide is of *Streptococcus pneumoniae* serotype 3, and wherein the conjugate has the general formula (**I**)

$$\mathbf{[H\text{-}(C)_{c3}\text{-}(D\text{-}C)_{c2}\text{-}(D)_{c1}\text{-}O\text{-}L^{3}\text{-}NH\text{-}W^{3}]_{m3}\text{-carrier protein}} \qquad (\mathbf{I})$$

wherein

C represents: 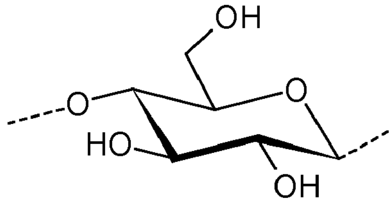

D represents: 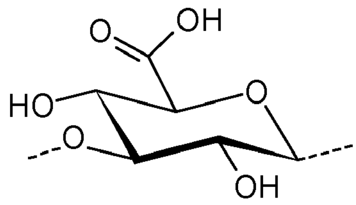

c2 is an integer selected from 1, 2, 3, 4, 5, 6, 7, 8, 9 and 10;
c1 and c3 represent independently of each other an integer which is 0 or 1 ;
$L^3$ represents a linker;
m3 is comprised between about 2 and about 18;
$-W^3-$ is selected from:

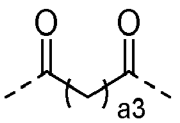 , 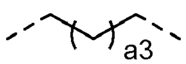 , and 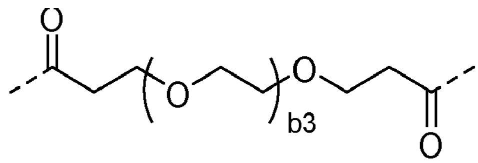 ;

a3 represents an integer from 1 to 10;
b3 represents an integer from 1 to 4; and

carrier protein is selected from detoxified pneumolysin or an immunogenic fragment thereof, and pneumococcal surface protein A, or an immunogenic fragment thereof.

One further embodiment of the invention refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, **for** use in raising a protective immune response in a human and/or animal host, wherein the synthetic *Streptococcus pneumoniae* oligosaccharide is of *Streptococcus pneumoniae* serotype 2, wherein the conjugate has the general formula (**II**)

$$\mathbf{[B^{*}\text{-}A_{y+3}\text{-}A_{y+2}\text{-}A_{y+1}\text{-}A_{y}\text{-}O\text{-}L^{2}\text{-}NH\text{-}W^{2}]_{m2}\text{-carrier protein}} \qquad (\mathbf{II})$$

wherein y is an integer selected from 1, 2, 3 and 4,

$A_1 = A_5 =$ 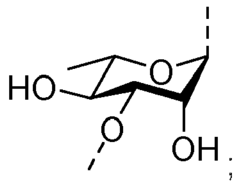 ; $A_2 = A_6 =$ 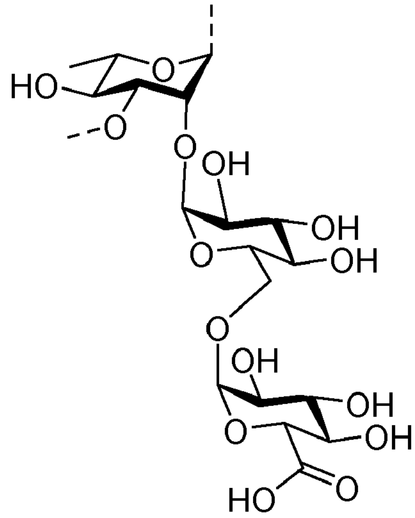 ,

$A_3 = A_7 =$ 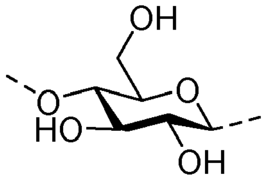 ; $A_4 =$ 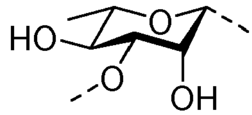 ;

B*- represents H-, H-$A_y$-, H-$A_{y+1}$-$A_y$-, H-$A_{y+2}$-$A_{y+1}$-$A_y$- or H-$A_{y+3}$-$A_{y+2}$-$A_{y+1}$-$A_y$-;
$L^2$ represents a linker;
m2 is comprised between about 2 and about 18,
-$W^2$- is selected from:

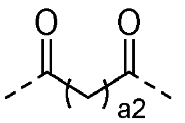 , 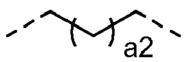 , and 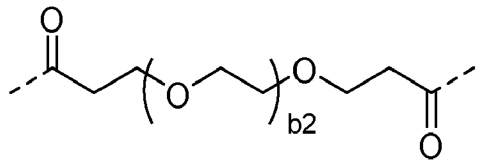 ;

a2 represents an integer from 1 to 10;
b2 represents an integer from 1 to 4; and

carrier protein is selected from detoxified pneumolysin or an immunogenic fragment thereof, and pneumococcal surface protein A, or an immunogenic fragment thereof.

One particular embodiment of the invention also refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, for use in the prevention and/or treatment of a disease caused by *Streptococcus pneumoniae,* wherein the synthetic *Streptococcus pneumoniae* oligosaccharide is of *Streptococcus pneumoniae* serotype 2, wherein the conjugate has the general formula (**II**)

**[B*-$A_{y+3}$-$A_{y+2}$-$A_{y+1}$-$A_y$-O-$L^2$-NH-$W^2$]$_{m2}$-carrier protein** (**II**)

wherein y is an integer selected from 1, 2, 3 and 4,

$A_1 = A_5 =$ 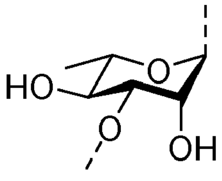 ; $A_2 = A_6 =$ 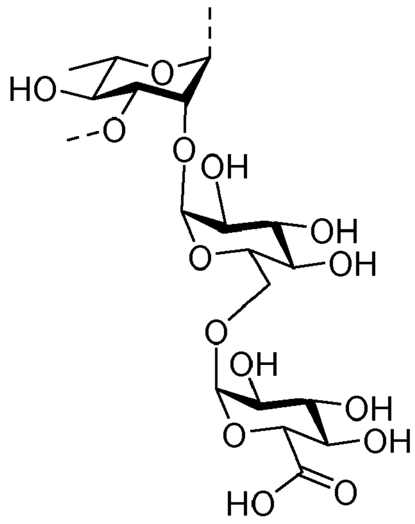 ,

$A_3 = A_7 =$ 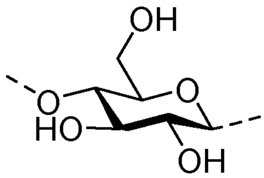 ; $A_4 =$ 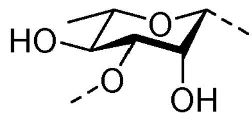 ;

B*- represents H-, H-$A_y$-, H-$A_{y+1}$-$A_y$-, H-$A_{y+2}$-$A_{y+1}$-$A_y$- or H-$A_{y+3}$-$A_{y+2}$-$A_{y+1}$-$A_y$-;
$L^2$ represents a linker;
m2 is comprised between about 2 and about 18,
-$W^2$- is selected from:

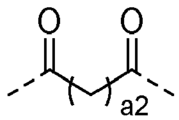 , 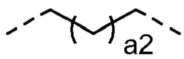 , and 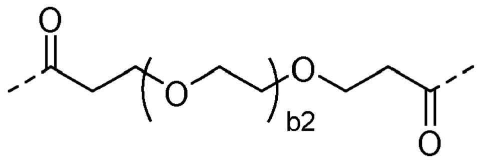 ;

a2 represents an integer from 1 to 10;
b2 represents an integer from 1 to 4; and

carrier protein is selected from detoxified pneumolysin or an immunogenic fragment thereof, and pneumococcal surface protein A, or an immunogenic fragment thereof.

One more particular embodiment of the invention further refers to a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, **for use** in the prevention and/or treatment of a disease caused by *Streptococcus pneumoniae* serotype 2 and/or serotype 3, wherein the synthetic *Streptococcus pneumoniae* oligosaccharide is of *Streptococcus pneumoniae* serotype 2, wherein the conjugate has the general formula (**II**)

$$[B^*\text{-}A_{y+3}\text{-}A_{y+2}\text{-}A_{y+1}\text{-}A_y\text{-}O\text{-}L^2\text{-}NH\text{-}W^2]_{m2}\text{-carrier protein} \qquad \textbf{(II)}$$

wherein y is an integer selected from 1, 2, 3 and 4,

$A_1 = A_5 =$ 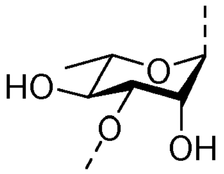 ; $A_2 = A_6 =$ 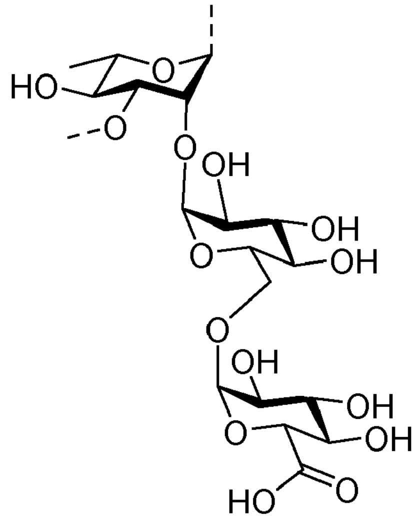 ,

$A_3 = A_7 =$ 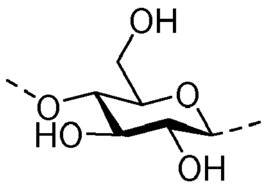 ; $A_4 =$ 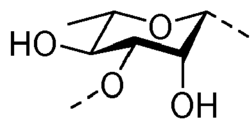 ;

B*- represents H-, H-$A_y$-, H-$A_{y+1}$-$A_y$-, H-$A_{y+2}$-$A_{y+1}$-$A_y$- or H-$A_{y+3}$-$A_{y+2}$-$A_{y+1}$-$A_y$-;
$L^2$ represents a linker;
m2 is comprised between about 2 and about 18,
-$W^2$- is selected from:

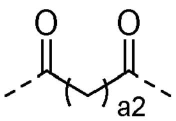 , 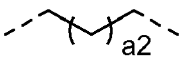 , and 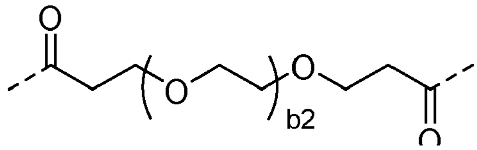 .

a2 represents an integer from 1 to 10;
b2 represents an integer from 1 to 4; and

carrier protein is selected from detoxified pneumolysin or an immunogenic fragment thereof, and pneumococcal surface protein A, or an immunogenic fragment thereof.

The diseases caused by *Streptococcus pneumoniae,* or in particular by *Streptococcus pneumoniae* serotype 2 and/or serotype 3, include pneumonia, meningitis, otitis media, bacteremia and acute exacerbation of chronic bronchitis, invasive pneumococcal disease, exacerbation of chronic obstructive pulmonary disease, sinusitis, arthritis and conjunctivitis.

## Vaccine Formulations

A further aspect of the present invention is directed to a vaccine that comprises the inventive immunogenic composition disclosed herein, a physiologically acceptable vehicle and an adjuvant. Thus, a vaccine can be obtained by formulating the inventive immunogenic composition with a physiologically acceptable vehicle and an adjuvant by methods known in the art.

Therefore, an embodiment of the invention is directed to a vaccine comprising a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, or an immunogenic composition comprising one or more of said conjugates, together with at least one pharmaceutically acceptable adjuvant and/or excipient.

A further embodiment of the invention is directed to a vaccine comprising a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the synthetic *Streptococcus pneumoniae* oligosaccharide is selected from the group of serotypes comprising serotype 1, 2, 3, 4, 5, 8, together with at least one pharmaceutically acceptable adjuvant and/or excipient.

A particular embodiment of the invention is directed to a **vaccine comprising a conjugate** of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the synthetic *Streptococcus pneumoniae* oligosaccharide is of *Streptococcus pneumoniae* serotype 3, together with at least one pharmaceutically acceptable adjuvant and/or excipient.

A more particular embodiment of the invention is directed to a **vaccine comprising a conjugate** of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the synthetic *Streptococcus pneumoniae* oligosaccharide is of *Streptococcus pneumoniae* serotype 3, wherein the conjugate has the general formula (**I**)

$$[H\text{-}(C)_{c3}\text{-}(D\text{-}C)_{c2}\text{-}(D)_{c1}\text{-}O\text{-}L^3\text{-}NH\text{-}W^3]_{m3}\text{-carrier protein} \qquad (\mathbf{I})$$

wherein

C represents: 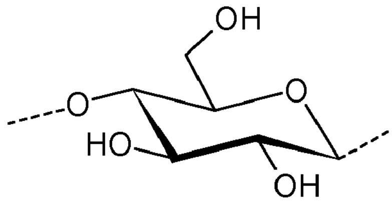

D represents: 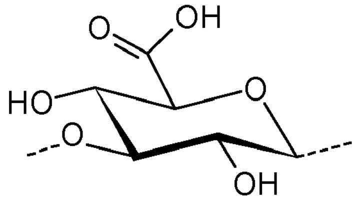

- c2 is an integer selected from 1, 2, 3, 4, 5, 6, 7, 8, 9 and 10;
- c1 and c3 represent independently of each other an integer which is 0 or 1;
- $L^3$ represents a linker;
- m3 is comprised between about 2 and about 18;
- $-W^3-$ is selected from:

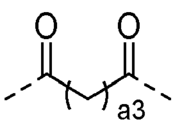 , 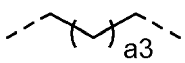 , and 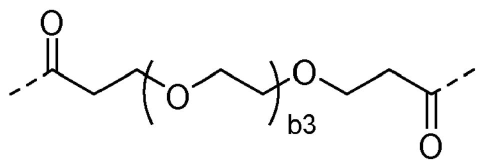 ;

- a3 represents an integer from 1 to 10;
- b3 represents an integer from 1 to 4; and
- carrier protein is selected from detoxified pneumolysin or an immunogenic fragment thereof, and pneumococcal surface protein A, or an immunogenic fragment thereof,

together with at least one pharmaceutically acceptable adjuvant and/or excipient.

A still more particular embodiment of the invention is directed to a **vaccine comprising a conjugate** of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the

synthetic *Streptococcus pneumoniae* oligosaccharide is of *Streptococcus pneumoniae* serotype 2, together with at least one pharmaceutically acceptable adjuvant and/or excipient.

A still more particular embodiment of the invention is directed to a **vaccine comprising a conjugate** of a synthetic *Streptococcus pneumoniae* oligosaccharide with a carrier protein selected from detoxified pneumolysin or an immunogenic fragment thereof, and pneumococcal surface protein A, or an immunogenic fragment thereof, wherein the synthetic *Streptococcus pneumoniae* oligosaccharide is of *Streptococcus pneumoniae* serotype 2, wherein the conjugate has the general formula (**II**)

$$[B^*\text{-}A_{y+3}\text{-}A_{y+2}\text{-}A_{y+1}\text{-}A_y\text{-}O\text{-}L^2\text{-}NH\text{-}W^2]_{m2}\text{-carrier protein} \qquad \textbf{(II)}$$

wherein y is an integer selected from 1, 2, 3 and 4,

$A_1 = A_5 =$ 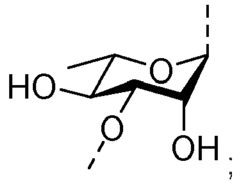 ; $A_2 = A_6 =$ 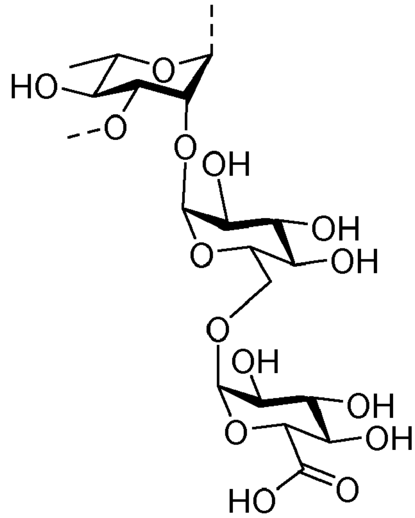 ,

$A_3 = A_7 =$ 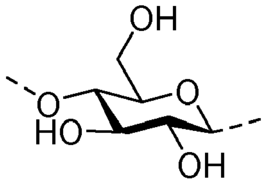 ; $A_4 =$ 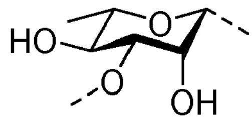 ;

B*- represents H-, H-$A_y$-, H-$A_{y+1}$-$A_y$-, H-$A_{y+2}$-$A_{y+1}$-$A_y$- or H-$A_{y+3}$-$A_{y+2}$-$A_{y+1}$-$A_y$-;
$L^2$ represents a linker;
m2 is comprised between about 2 and about 18,
-$W^2$- is selected from:

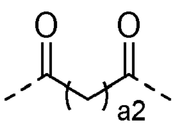 , 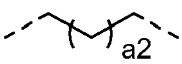 , and 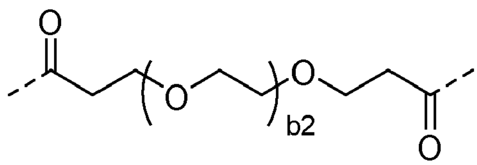 ;

a2 represents an integer from 1 to 10;
b2 represents an integer from 1 to 4; and
carrier protein is selected from detoxified pneumolysin or an immunogenic fragment thereof, and pneumococcal surface protein A, or an immunogenic fragment thereof,

together with at least one pharmaceutically acceptable adjuvant and/or excipient.

In accordance with one particular embodiment, the present invention is directed to a **vaccine comprising an immunogenic composition** comprising (i) a conjugate of a synthetic *Streptococcus pneumoniae* oligosaccharide with

detoxified pneumolysin or an immunogenic fragment thereof, and (ii) a conjugate of a synthetic *Streptococcus pneumoniae* oligosaccharide with pneumococcal surface protein A or an immunogenic fragment thereof, together with at least one pharmaceutically acceptable adjuvant and/or excipient.

A further embodiment of the invention is directed to a **vaccine comprising an immunogenic composition** comprising a conjugate of *Streptococcus pneumoniae* serotype 3, and a mixture consisting of capsular polysaccharides from *Streptococcus pneumoniae* serotypes 1, 4, 5, 6A, 6B, 7F, 9V, 14, 18C, 19A, 19F and 23F individually conjugated to $CRM_{197}$ carrier protein, together with at least one pharmaceutically acceptable adjuvant and/or excipient.

One still more particular embodiment of the present invention is directed to a **vaccine comprising an immunogenic** composition comprising a conjugate of *Streptococcus pneumoniae* serotype 3, and a mixture consisting of capsular polysaccharides from *Streptococcus pneumoniae* serotypes 1, 4, 5, 6A, 6B, 7F, 9V, 14, 18C, 19A, 19F and 23F individually conjugated to $CRM_{197}$ carrier protein, wherein the conjugate has the general formula (**I**)

$$\mathbf{[H\text{-}(C)_{c3}\text{-}(D\text{-}C)_{c2}\text{-}(D)_{c1}\text{-}O\text{-}L^3\text{-}NH\text{-}W^3]_{m3}\text{-}carrier\ protein} \qquad (\mathbf{I})$$

wherein

C represents: 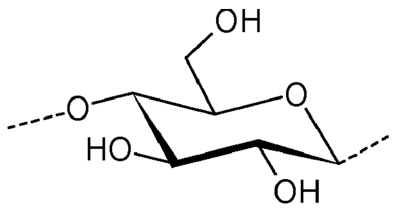

D represents: 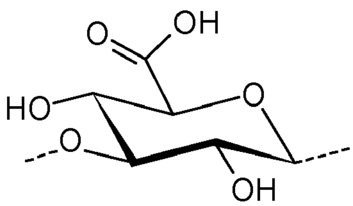

c2 is an integer selected from 1, 2, 3, 4, 5, 6, 7, 8, 9 and 10;
c1 and c3 represent independently of each other an integer which is 0 or 1;
$L^3$ represents a linker;
m3 is comprised between about 2 and about 18;
$-W^3-$ is selected from:

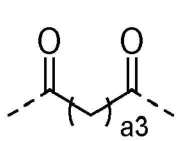, 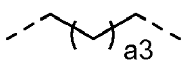, and 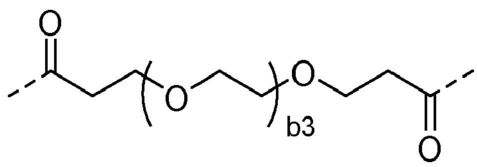;

a3 represents an integer from 1 to 10;
b3 represents an integer from 1 to 4; and
carrier protein is selected from detoxified pneumolysin or an immunogenic fragment thereof, and pneumococcal surface protein A, or an immunogenic fragment thereof,

together with at least one pharmaceutically acceptable adjuvant and/or excipient.

One still more particular embodiment of the present invention is directed to a **vaccine comprising an immunogenic composition** comprising one or more conjugates of a synthetic *Streptococcus pneumoniae* oligosaccharide with a carrier protein selected from detoxified pneumolysin or an immunogenic fragment thereof, and pneumococcal surface protein A, or an immunogenic fragment thereof,
wherein the one or more conjugates are selected from a conjugate of a synthetic *Streptococcus pneumoniae* serotype 1 oligosaccharide, a conjugate of a synthetic *Streptococcus pneumoniae* serotype 2 oligosaccharide, a conjugate of a synthetic *Streptococcus pneumoniae* serotype 3 oligosaccharide, a conjugate of a synthetic *Streptococcus pneumoniae*

serotype 4 oligosaccharide, a conjugate of a synthetic *Streptococcus pneumoniae* serotype 5 oligosaccharide, a conjugate of a synthetic *Streptococcus pneumoniae* serotype 8 oligosaccharide,
together with at least one pharmaceutically acceptable adjuvant and/or excipient.

Said vaccine includes a nasal formulation, preferably nasal drops, nasal powder, a nasal gel or a nasal spray.

Therefore, a further embodiment of the invention is directed to a vaccine comprising a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, or an immunogenic composition comprising one or more of said conjugates, together with at least one pharmaceutically acceptable adjuvant and/or excipient, wherein the vaccine includes a nasal formulation, preferably nasal drops, nasal powder, a nasal gel or a nasal spray.

In a further embodiment, said vaccine comprises the adjuvant Cholera Toxin subunit B or the adjuvant Aluminum hydroxide.

Thus, an embodiment of the invention is directed to a vaccine comprising a conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, or an immunogenic composition comprising one or more of said conjugates, together with at least one pharmaceutically acceptable adjuvant and/or excipient, wherein the adjuvant is Cholera Toxin subunit B or Aluminum hydroxide.

A physiologically acceptable vehicle may include non-toxic levels of alcohols and salts, 5% dextrose or other sugars, saline, and other pharmaceutically acceptable excipients, and any combination of any of these solvents. Such excipients are well known and described. Preferably used physiologically acceptable **vehicles** are water, buffered saline, succinic acid, polysorbate 80, polyols (e.g., glycerol, propylene glycol, liquid polyethylene glycol) and dextrose solutions.

Vaccines are preferably in aqueous form, particularly at the point of administration, but they can also be presented in non-aqueous liquid forms or in dried forms e.g. as gelatin capsules, or as lyophilisates, etc. Vaccines may include one or more preservatives, such as thiomersal or 2-phenoxyethanol. Mercury-free compositions are preferred, and preservative-free vaccines can be prepared.

Vaccines may include a physiological salt, such as a sodium salt e.g. to control tonicity. Sodium chloride (NaCl) is typical and may be present at between 1 and 20 mg/ml. Other salts that may be present include potassium chloride, potassium dihydrogen phosphate, disodium phosphate dehydrate, magnesium chloride, calcium chloride, etc..

Vaccines can have an osmolality of between 200 mOsm/kg and 400 mOsm/kg. Vaccines may include compounds (with or without an insoluble metal salt) in plain water (e.g. w.f.i.), but will usually include one or more buffers. Typical buffers include: a phosphate buffer; a Tris buffer; a borate buffer; a succinate buffer; a histidine buffer (particularly with an aluminium hydroxide adjuvant); or a citrate buffer. Buffer salts will typically be included in the 5-20 mM range. Vaccines typically have a pH between 5.0 and 9.5 e.g. between 6.0 and 8.0. Vaccines are preferably sterile and gluten free.

Vaccines are suitable for administration to **animal (and, in particular, human) patients, and thus, include both human and veterinary uses**. They may be used in a method of raising an immune response in a patient, comprising the step of administering the composition to the patient. The vaccines of the present invention may be administered before a subject is exposed to a *Streptococcus pneumoniae* and/or after a subject is exposed to a *Streptococcus pneumoniae.*

Vaccines may be prepared in unit dose form. In some embodiments a unit dose may have a volume of between 0.1 - 1.0 mL e.g. about 0.5 mL. The invention also provides a delivery device (e.g. syringe, nebulizer, sprayer, inhaler, dermal patch, etc.) containing a vaccine of the invention e.g. containing a unit dose. This device can be used to administer the composition to a vertebrate subject.
The invention also provides a sterile container (e.g. a vial) containing a vaccine of the invention e.g. containing a unit dose. The invention also provides a unit dose of a vaccine of the invention.
The invention also provides a hermetically sealed container containing a vaccine of the invention. Suitable containers include e.g. a vial.

Vaccines of the invention **may be prepared in various forms**. For example, the vaccines may be prepared as injectables, either as liquid solutions or suspensions. Solid forms suitable for solution in, or suspension in, liquid vehicles prior to injection can also be prepared (e.g. a lyophilized composition or a spray-freeze dried composition). The vaccine may be prepared for topical administration e.g. as an ointment, cream or powder. The vaccine may be prepared for oral administration e.g. as a tablet or capsule, as a spray, or as a syrup (optionally flavored). The vaccine may be prepared for pulmonary administration e.g. by an inhaler, using a fine powder or a spray. The vaccine may be prepared as a suppository. The vaccine may be prepared for nasal, aural or ocular administration e.g. as a spray or drops. Injectables for intramuscular administration are typical. Techniques for the formulation and administration of the vaccine of the present invention may be found in "Remington's Pharmaceutical Sciences" Mack Publishing Co., Easton PA.

The **amount of conjugate** in each vaccine dose is selected as an amount that induces an immunoprotective response without significant, adverse effects. Such amount can vary depending upon the *S. pneumoniae* serotype. Preferably, each dose will comprise 0.1 to 100 μg of each synthetic oligosaccharide or capsular polysaccharide, more

preferably 0.1 to 10 µg, and most preferably 1 to 5 µg.

Said vaccine may be prepared in the form of a suspension or may be lyophilized. The suspension form may be stored frozen. In the lyophilized form, it is preferable to add one or more stabilizers. Optionally, one or more adjuvants may be added as well. Any conventional stabilizers and adjuvants may be included in a vaccine according to this invention.

Vaccination can be performed at any age.

The vaccine can also be administered in form of its pharmaceutically active salt optionally using substantially nontoxic pharmaceutically acceptable carrier, **excipients, adjuvants or diluents**. The vaccine of the present invention is prepared in a conventional solid or liquid carrier or diluents and a conventional pharmaceutically-made adjuvant at suitable dosage level in a known way. The preferred preparations and formulations are in administrable form, which is suitable for oral application. These administrable forms, for example, include pills, tablets, film tablets, coated tablets, capsules, powders and deposits.

The inventive vaccine may be **administered** by any appropriate means, including but not limited to inhalation; injection (intravenous, intraperitoneal, intramuscular, subcutaneous); by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

The vaccine of the present invention will typically be administered in admixture with suitable **carrier materials** suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. Moreover, when desired or needed, suitable **binders**, **lubricants**, disintegrating agents and coloring agents may also be incorporated in the mixture.

Suitable **binders** include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the vaccine of the present invention may be formulated in **sustained release form** to provide the rate controlled release of any one or more of the components or active ingredients to optimize the therapeutic effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

**Liquid form preparations** include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

**Aerosol preparations** suitable for inhalation may include solutions and solids in powder form, which may be in combination with a pharmaceutically acceptable carrier such as inert compressed gas, e.g. nitrogen.

For preparing **suppositories**, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are **solid form preparations** that are intended to be converted, shortly before use, to liquid form preparations for either oral or parenteral administration. Such liquid forms include solutions, suspensions and emulsions.

The vaccine of the present invention may also be deliverable **transdermally**. The transdermal compositions may take the form of creams, lotions, aerosols and/or emulsions and can be included in a transdermal patch of the matrix or reservoir type as are conventional in the art for this purpose.

The term **capsule** refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

**Tablet** means compressed or molded solid dosage form containing the active ingredients with suitable diluents. The tablet can be prepared by compression of mixtures or granulations obtained by wet granulation, dry granulation or by compaction well known to a person skilled in the art.

Oral gels refer to the active ingredients dispersed or solubilized in a hydrophilic semi-solid matrix.

Powders for constitution refer to powder blends containing the active ingredients and suitable diluents which can be suspended in water or juices.

Suitable **diluents** are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from

about 5 to about 95% by weight of the total composition, preferably from about 25 to about 75%, more preferably from about 30 to about 60% by weight, and most preferably from about 40 to 50% by weight.

The term **disintegrants** refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

**Lubricant** refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and D, L-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

**Glidents** are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1% to about 7% by weight of the total composition, more preferably from about 0.2 to 5% by weight, and most preferably from about 0.5 to about 2% by weight.

**Coloring agents** are excipients that provide coloration to the composition or the dosage form. Such excipients can include food grade dyes and food grade dyes adsorbed onto a suitable adsorbent such as clay or aluminum oxide. The amount of the colouring agent can vary from about 0.01 to 10% by weight of the composition, preferably from about 0.05 to 6% by weight, more preferably from about 0.1 to about 4% by weight of the composition, and most preferably from about 0.1 to about 1%.

Techniques for the formulation and administration of the vaccine of the present invention may be found in "Remington's Pharmaceutical Sciences" Mack Publishing Co., Easton PA.

A **therapeutically effective dosage** of one conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof, refers to that amount of said conjugate that results in an at least a partial immunization against a disease. Toxicity and therapeutic efficacy of such conjugates can be determined by standard pharmaceutical, pharmacological, and toxicological procedures in cell cultures or experimental animals. The dose ratio between toxic and therapeutic effect is the **therapeutic index**. The actual amount of the composition administered will be dependent on the subject being treated, on the subject's weight, the severity of the affliction, the manner of administration and the judgement of the prescribing physician.

The inventive vaccine comprises an effective amount of an **adjuvant** i.e. an amount which, when administered to an individual, either in a single dose or as part of a series, is effective for enhancing the immune response to a co-administered *S. pneumoniae* antigen or. a *S. pneumoniae* conjugate. This amount can vary depending upon the health and physical condition of the individual to be treated, age, the taxonomic group of individual to be treated (e.g. non-human primate, primate, etc.), the capacity of the individual's immune system to synthesize antibodies, the degree of protection desired, the formulation of the vaccine, the treating doctor's assessment of the medical situation, and other relevant factors. The amount will fall in a relatively broad range that can be determined through routine trials.

The term "**adjuvant**" as used herein refers to an immunological adjuvant i.e. a material used in a vaccine composition that modifies or augments the effects of said vaccine by enhancing the immune response to a given antigen contained in the vaccine without being antigenically related to it. For the persons skilled in the art, classically recognized examples of immunological adjuvants include, but are not restricted to

(1) aluminum salts (alum), such as aluminum hydroxide, aluminum phosphate, aluminum sulfate, etc.;
(2) oil-in-water emulsion formulations (with or without other specific immunostimulating agents such as muramyl peptides (defined below) or bacterial cell wall components), such as, for example,

(a) MF59 (WO 90/14837), containing 5% Squalene, 0.5% Tween 80, and 0.5% Span 85 (optionally containing various amounts of MTP-PE (see below, although not required)) formulated into submicron particles using a microfluidizer such as Model 110Y microfluidizer (Microfluidics, Newton, MA),
(b) SAF, containing 10% Squalene, 0.4% Tween 80, 5% pluronic-blocked polymer L121 , and thr-MDP (see below) either microfluidized into a submicron emulsion or vortexed to generate a larger particle size emulsion, and
(c) Ribi(TM) adjuvant system (RAS), (Corixa, Hamilton, MT) containing 2% Squalene, 0.2% Tween 80, and one or more bacterial cell wall components from the group consisting of 3-O-deaylated monophosphorylipid A (MPL(TM)) described in U.S. Patent No. 4,912,094 (Corixa), trehalose dimycolate (TDM), and cell wall skeleton (CWS), preferably MPL + CWS (Detox™);

(3) saponins, which are a heterologous group of sterol glycosides and triterpenoid glycosides that are found in the bark, leaves, stems, roots and even flowers of a wide range of plant species. Saponins from the bark of the *Quillaia saponaria,* Molina tree have been widely studied as adjuvants. Saponins can also be commercially obtained from Smilax ornata (sarsaprilla), Gypsophilla paniculata (brides veil), and Saponaria oficianalis (soap root). Saponin adjuvant formulations include purified formulations, such as QS21, as well as lipid formulations, such as ISCOMs. Saponin compositions have been purified using HPLC and RP-HPLC. Specific purified fractions using these techniques have been identified, including QS7, QS 17, QS 18, QS2 1, QH-A, QH-B and QH-C. Saponin formulations may also comprise a sterol, such as cholesterol. Combinations of saponins and cholesterols can be used to form unique particles called immunostimulating complexes (ISCOMs). ISCOMs generally include a phospholipid such as phosphatidylethanolamine or phosphatidylcholine. Any known saponin can be used in ISCOMs. Preferably, the ISCOM includes one or more of QuilA, QHA & QHC;
(4) bacterial lipopolysaccharides, synthetic lipid A analogs such as aminoalkyl glucosamine phosphate compounds (AGP), or derivatives or analogs thereof, which are available from Corixa, and which are described in U.S. Patent No. 6,113,918; one such AGP is 2-[(R)-3-tetradecanoyl-oxytetradecanoylamino]ethyl 2-deoxy-4-O-phosphono-3-O-[(R)-3-tetradecanoyloxytetradecanoyl]-2-[(R)-3-tetradecanoyloxytetradecanoylamino-β-D-glucopyranoside, which is also known as 529 (formerly known as RC529), which is formulated as an aqueous form or as a stable emulsion, synthetic polynucleotides such as oligonucleotides containing CpG motif(s) (U.S. Patent No. 6,207,646);
(5) cytokines, such as interleukins (e.g., IL-1 , IL-2, IL-4, IL-5, IL-6, IL-7, IL-12, IL-15, IL-18, etc.), interferons (e.g., gamma interferon), granulocyte macrophage colony stimulating factor (GM-CSF), macrophage colony stimulating factor (M-CSF), tumor necrosis factor (TNF), costimulatory molecules B7-1 and B7-2, etc.;
(6) detoxified mutants of a bacterial ADP-ribosylating toxin such as a cholera toxin (CT) either in a wild-type or mutant form, for example, where the glutamic acid at amino acid position 29 is replaced by another amino acid, preferably a histidine, in accordance with WO 00/18434 (see also WO 02/098368 and WO 02/098369), a pertussis toxin (PT), or an E. coli heat-labile toxin (LT), particularly LT-K63, LT-R72, CT-S109, PT-K9/G129 (see, e.g., WO 93/13302 and WO 92/19265); and
(7) other substances that act as immunostimulating agents to enhance the effectiveness of the composition. Muramyl peptides include, but are not limited to, N-acetyl-muramyl-L-threonyl-D-isoglutamine (thr-MDP), N-acetyl-normuramyl-L-alanine-2-(1'-2'dipalmitoyl-sn-glycero-3-hydroxyphosphoryloxy)-ethylamine (MTP-PE)1 etc.
(8) microparticles (i.e. a particle of 100 nm to 150 pm in diameter, more preferably 200 nm to 30 pm in diameter, or 500 nm to 10 pm in diameter) formed from materials that are biodegradable and non-toxic. Such non-toxic and biodegradable materials include, but are not restricted to poly(α-hydroxy acid), polyhydroxybutyric acid, polyorthoester, polyanhydride, polycaprolactone;#
(9) CD1d ligands, such as an α-glycosylceramide, phytosphingosine-containing α-glycosylceramides, OCH, KRN7000 [(2*S*,3*S*,4*R*)-1-*O*-(α-D-galactopyranosyl)-2-(*N*-hexacosanoylamino)-1,3,4-octadecanetriol], CRONY-101, 3"-sulfogalactosyl-ceramide;
(10) immunostimulatory oligonucleotides, such CpG motif containing ones (a dinucleotide sequence containing an unmethylated cytosine residue linked by a phosphate bond to a guanosine residue), or Cpl motif containing ones (a dinucleotide sequence containing cytosine linked to inosine), or a double-stranded RNA, or an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded;
(11)compounds containing lipids linked to a phosphate-containing acyclic backbone, such as the TLR4 antagonist E5564;
(12) oil emulsions (e.g. Freund's adjuvant).

Theoretically, each molecule or substance that is able to favor or amplify a particular situation in the cascade of immunological events, ultimately leading to a more pronounced immunological response, can be defined as an **adjuvant.**

In principle, through the use of adjuvants in vaccine formulations, one can

- direct and optimize immune responses that are appropriate or desirable for the vaccine;
- enable mucosal delivery of vaccines, i.e. administration that results in contact of the vaccine with a mucosal surface such as buccal or gastric or lung epithelium and the associated lymphoid tissue;
- promote cell-mediated immune responses;
- enhance the immunogenicity of weaker immunogens, such as highly purified or recombinant antigens;
- reduce the amount of antigen or the frequency of immunization required to provide protective immunity; and
- improve the efficacy of vaccines in individuals with reduced or weakened immune responses, such as newborns, the aged, and immunocompromised vaccine recipients.

Although little is known about their mode of action, it is currently believed that **adjuvants** augment immune responses by one of the following mechanisms:

- increasing the biological or immunologic half-life of antigens;
- improving antigen delivery to antigen-presenting cells (APCs), as well as antigen processing and presentation by the APCs e.g., by enabling antigen to cross endosomal membranes into the cytosol after ingestion of antigen-adjuvant complexes by APC;
- mimicking danger inducing signals from stressed or damaged cells, which serve to initiate an immune response;
- inducing the production of immunomodulatory cytokines;
- biasing the immune response towards a specific subset of the immune system; and
- blocking the rapid dispersal of the antigen challenge.

In one embodiment of the present invention the vaccine comprises the herein disclosed immunogenic composition, wherein each conjugate is adsorbed on aluminum phosphate.

Another aspect of the present invention relates to pharmaceutical formulations and pharmaceutical compositions containing the vaccine as an active ingredient, together with at least one pharmaceutically acceptable carrier, excipient, solvent and/or diluents.

Further preferred, the pharmaceutical composition is formulated in the form of a lyophilisate or liquid buffer solution.

Synthesis of *S. pneumoniae* serotype 2 conjugates of general formula (II)

The conjugates of general formula **II**

$$[B^*\text{-}A_{y+3}\text{-}A_{y+2}\text{-}A_{y+1}\text{-}A_y\text{-}O\text{-}L^2\text{-}NH\text{-}W^2]_{m2}\text{-carrier protein} \qquad \textbf{(II)}$$

wherein

y is an integer selected from 1, 2, 3 and 4;

$A_1 = A_5 =$ 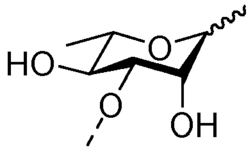 ; $A_2 = A_6 =$ 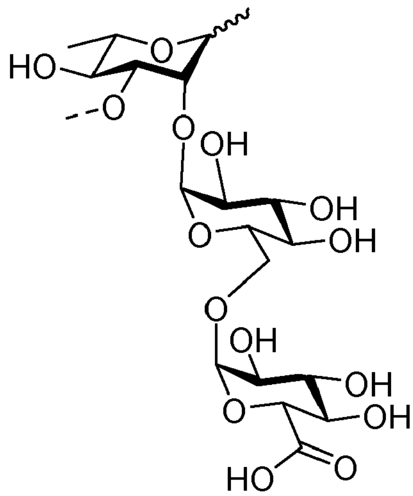 ,

$A_3 = A_7 =$ 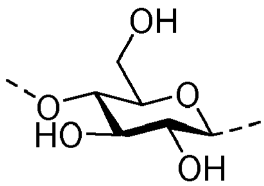 ; $A_4 =$ 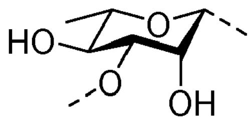 ;

B*- represents: H-, H-$A_y$-, H-$A_{y+1}$-$A_y$-, H-$A_{y+2}$-$A_{y+1}$-$A_y$- or H-$A_{y+3}$-$A_{y+2}$-$A_{y+1}$-$A_y$-;
$L^2$ represents a linker and is defined as above;
m2 is comprised between about 2 and about 18;
-$W^2$- is selected from:

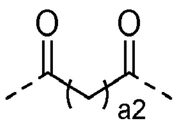 , 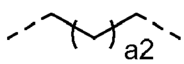 , and 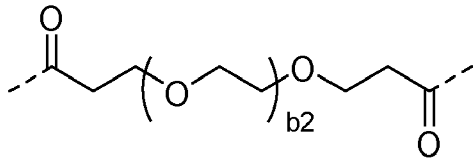 ;

a2 represents an integer from 1 to 10;
b2 represents an integer from 1 to 4; and
carrier protein has the meaning of carrier protein as defined above can be obtained starting from a saccharide of general formula **2*** and carrier protein using coupling methods well known to the skilled person (see **Scheme 1).**

**II**

⇓

**B*-$A_{y+3}$-$A_{y+2}$-$A_{y+1}$-$A_y$-O-$L^2$-$NH_2$** + **carrier-protein**
**2***

**Scheme 1**: Retrosynthetic analysis of the *S. pneumoniae* serotype 2 conjugates of general formula (**II**).

Such coupling methods include, for example, treatment of the saccharide **2*** with a commercially available bifunctional spacer (Thermo Fischer, Sigma Aldrich) such as, disuccinimidyl adipate (DSA), bis-(4-nitrophenyl) adipate, bis-(4-nitrophenyl)succinate, disuccinimidyl glutarate (DSG), bis(sulfosuccinimidyl)disuccinimidyl glutarate bis(sulfosuccinimidyl)disuccinimidyl suberate (DSS), bis(sulfosuccinimidyl)suberate ($BS^3$), sebacic acid bis(N-succinimidyl) ester bis(succinimidyl)penta(ethyleneglycol), bis(succinimidyl) tetra(ethyleneglycol), in presence of a base, such as triethylamine, pyridine, followed by reaction of the resulting construct with the carrierprotein. Alternatively, saccharide **2*** can be reacted with a suitable dialdehyde, such as glutaraldehyde, under reductive amination conditions, and the resulting construct can be subsequently treated with the carrier protein under reductive amination conditions.

A saccharide of general formula **2*** can be efficiently assembled starting from building blocks **3***, **4***, **5***, **6***, **7***, as well as amino-alcohol linker **8*** as starting material (**Scheme 2**) and using glycosylation and deprotection method well known to the skilled person in the art. Building block **6*** can be prepared from known building block **9*** (Bundle D. R. et al. ACS Chem. Biol. 2012, 7, 1754). Conversion of building block **9*** to building block **6*** involves installing the Fmoc protecting group at 4th position of the glucoside by reaction with FmocCl and pyridine in dichloromethane, followed by treatment with CAN in a mixture of acetonitrile and water, and finally installation of the trichloroacetimidate leaving group at the anomeric position by treatment with $CCl_3CN$ and DBU in dichloromethane at room temperature.

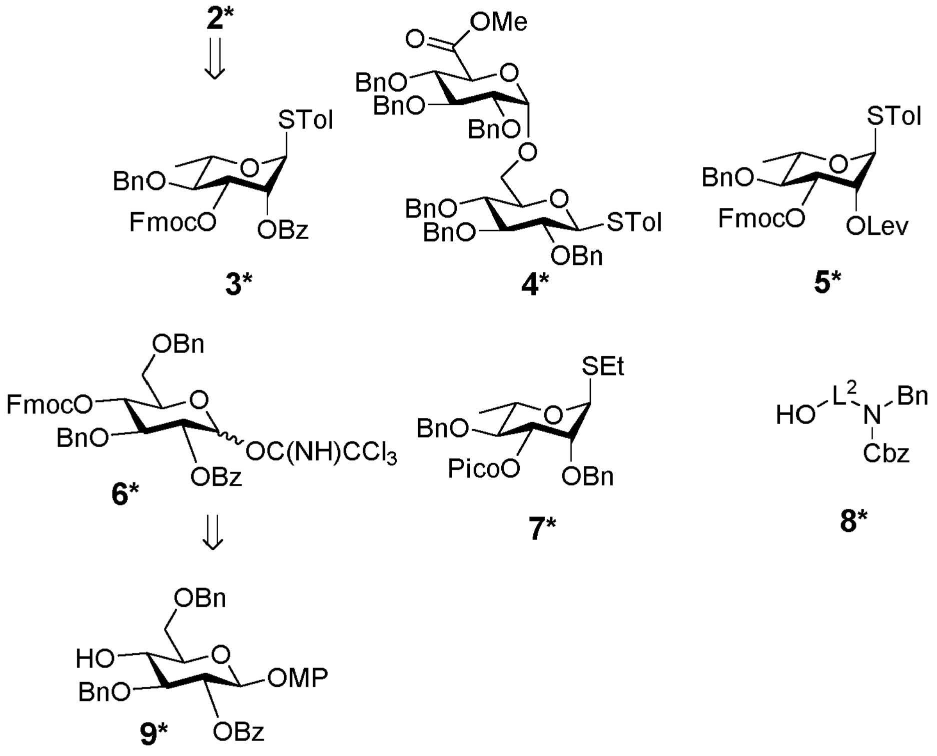

2*

3* 4* 5*

6* 7* 8*

9*

**Scheme 2**: Retrosynthetic analysis of a saccharide of general formula **2***.

Synthesis of *S. pneumoniae* serotype 3 conjugates of general formula (III)

The conjugates of general formula **III**

$$[H-(C)_{c3}-(D-C)_{c2}-(D)_{c1}-O-L^3-NH-W^3]_{m3}\text{-carrier protein} \qquad (III)$$

wherein C, D, c1, c2, c3, L3, m3, $-W^3-$ and carrier protein have the meanings defined herein, can be generated starting from saccharide **10*** and carrier protein using coupling methods well known to the skilled person.

**III**

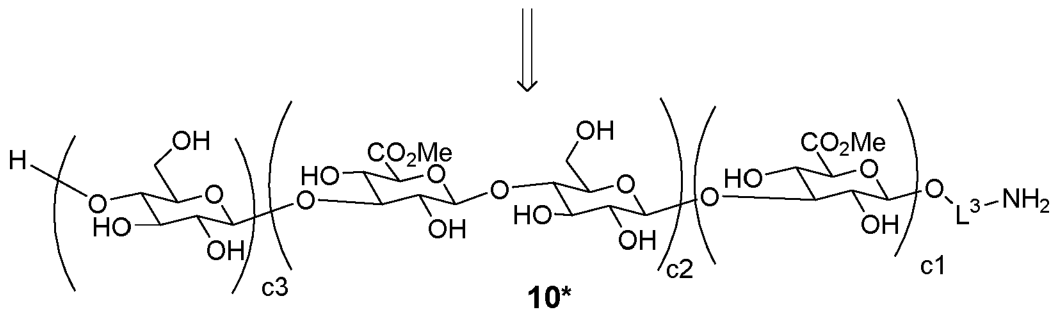

**10***

+

**carrier protein**

**Scheme 3**: Retrosynthetic analysis of the *S. pneumoniae* serotype 3 conjugates of general formula (**III**).

Such coupling methods include, for example, treatment of the saccharide **10*** with a commercially available bifunctional spacer (Thermo Fischer, Sigma Aldrich) such as, disuccinimidyl adipate (DSA), bis-(4-nitrophenyl) adipate, bis-(4-nitrophenyl)succinate, disuccinimidyl glutarate (DSG), bis(sulfosuccinimidyl) disuccinimidyl glutarate bis(sulfosuccinimidyl)disuccinimidyl suberate (DSS), bis(sulfosuccinimidyl)suberate ($BS^3$), sebacic acid bis(N-succinimidyl) ester, bis(succinimidyl)penta(ethyleneglycol), bis(succinimidyl)tetra(ethyleneglycol), in presence of a base, such as trimethylamine or pyridine, followed by reaction of the resulting construct with the carrier protein. Alternatively, saccharide **10*** can be reacted with a suitable dialdehyde, such as glutaraldehyde, under reductive amination condition, and the resulting construct can be subsequently treated with the carrier protein under reductive amination conditions.

Saccharides **10*** can be prepared following synthetic methods disclosed in the international patent application WO 2015/040140A1.

<u>Synthesis of S. *pneumoniae* serotype 8 conjugates of general formula (**VI**)</u>

The conjugates of general formula **VI**

$$[V^*\text{-}U_{x+3}\text{-}U_{x+2}\text{-}U_{x+1}\text{-}U_x\text{-}O\text{-}L^8\text{-}NH\text{-}W^1]_{m1}\text{-carrier protein} \qquad (\mathbf{VI})$$

wherein V*-, x, $U_{x+3}$, $U_{x+2}$, $U_{x+1}$, $U_x$, $L^8$, $-W^1-$, m1 and carrier protein have the meanings defined herein, can be obtained starting from a saccharide of general formula **1*** and carrier protein1 using coupling methods well known to the skilled person (see **Scheme 1**).

**VI**

$$V^*\text{-}U_{x+3}\text{-}U_{x+2}\text{-}U_{x+1}\text{-}U_x\text{-}O\text{-}L^8\text{-}NH_2 \quad + \quad \text{carrier protein}$$

**1***

**Scheme 1**: Retrosynthetic analysis of the *S. pneumoniae* serotype 8 conjugates of general formula (**VI**).

Such coupling methods include, for example, treatment of the saccharide **1*** with a commercially available bifunctional spacer (Thermo Fischer, Sigma Aldrich) such as, disuccinimidyl adipate (DSA), bis-(4-nitrophenyl) adipate, bis-(4-nitrophenyl)succinate, disuccinimidyl glutarate (DSG), bis(sulfosuccinimidyl)disuccinimidyl glutarate bis(sulfosuccinimidyl)disuccinimidyl suberate (DSS), bis(sulfosuccinimidyl)suberate ($BS^3$), sebacic acid bis(N-succinimidyl) ester bis(succinimidyl)penta(ethyleneglycol), bis(succinimidyl) tetra(ethyleneglycol), in presence of a base, such as triethylamine, pyridine, followed by reaction of the resulting construct with carrier protein. Alternatively, saccharide **1*** can be reacted with a suitable dialdehyde, such as glutaraldehyde, under reductive amination condition, and the resulting construct can be subsequently treated with the carrier protein under reductive amination conditions.

A saccharide of general formula **1*** can be synthesized *via* several synthetic routes. For example, saccharide **1*** can be assembled starting from thioglycoside building blocks **BB2, BB3, BB4**,and **BB5,** and functionalized solid support **BB1** *(*Angew. Chem. Int. Ed. 2013, 52, 5858.) (see **Scheme 5**) by automated solid phase synthesis.

The synthetic process, which is summarized in **Scheme 5** involves:

- assembly of the desired oligosaccharide, which includes
  glycosylation with the appropriate thioglycoside (**BB2, BB3, BB4** or **BB5**) by activation with NIS/TfOH; followed by removal of the temporary protecting group Fmoc by treatment with $Et_3N$;

- cleavage from the solid support; and

- removal of the permanent protecting groups.

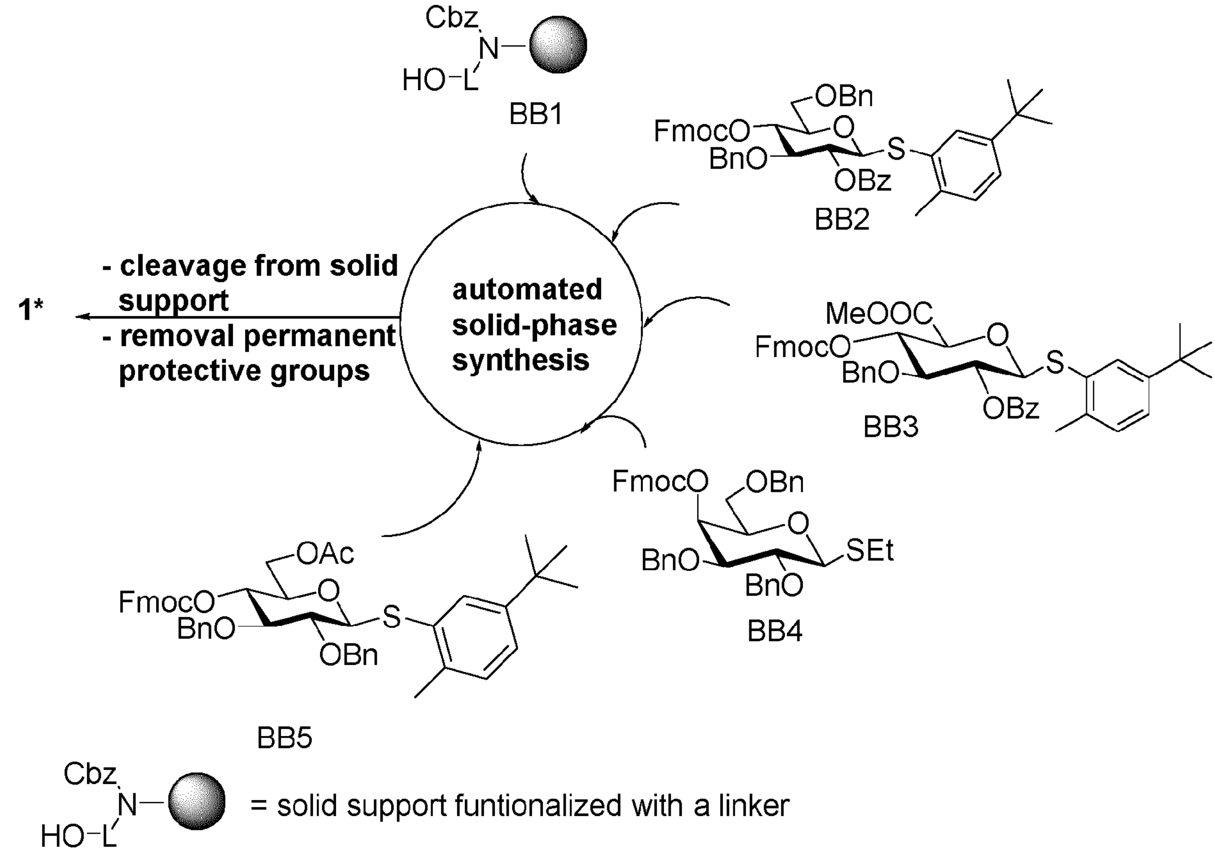


**Scheme 5**: Automated solid phase synthesis of saccharides of general formula **1***.

**Description of the figures**

**Figure 1** shows in a) characterization of purified detoxified pneumolysin (dPly) PlyW433E by SDS-PAGE gel to analyse the fractions from the pneumolysin purification process. Flow-through (line 2), washing steps (line 3 and 4) as well as elutions (line 5 and 6) were separated by 10% SDS-PAGE electrophoresis and stained with PageBlue protein staining solution The Page Ruler™ Prestained Protein Ladder was used as a molecular weight marker (line 1). b) MALDI-TOF-MS evaluation of average molecular size of ST3 tetrasaccharide dPly conjugate and of dPly carrier protein as a standard. c) Characterization of ST3-tetrasaccharide and ST2-hexasaccharide conjugated to dPly or PspA by SDS-PAGE electrophoresis. Line 1: 1 $\mu$g of ST3-tetrasaccharide PspA conjugate; Line 2: 1 $\mu$g of ST3-tetrasaccharide dPly conjugate; Line 3: 1 $\mu$g of ST3-tetrasaccharide $CRM_{197}$ conjugate; Line 4: 1$\mu$g of ST2-hexasaccharide PspA conjugate; Line 5: 1$\mu$g of ST2-hexasaccharide dPly conjugate; Line 6: 1$\mu$g of ST2-hexasaccharide $CRM_{197}$ conjugate; Line 7: 1$\mu$g of PspA; Line 8: 1$\mu$g of pneumolysin; Line 9: 1$\mu$g of $CRM_{197}$. PageRuler™ Plus Prestained Protein Ladder was used as a molecular weight standard.

**Figure 2** shows MALDI-TOF analysis of ST3-tetrasaccharide and ST2-hexasaccharide conjugated to detoxified pneumolysin (dPly) and PspA carrier proteins. The analysis was carried out to measure the average molecular size of conjugates and the loading ratio of the glycan on the carrier protein. a) recombinant pneumolysin (55 kDa), b) PspA (33.5 kDa), c) ST2-hexasaccharide-dPly, 69.4 kDa; d) ST2-hexasaccharide-PspA, 47.9 kDa; e) ST3-tetrasaccharide-dPly, 66.3 kDa; f) ST3-tetrasaccharide-PspA, 44 kDa.

**Figure 3** shows calculation of glycan loading ratio per protein based on the mass of compound evaluated by MALDI-TOF-MS.

**Figure 4** shows evaluation of the protective effect in mice of ST3-tetrasaccharide detoxified pneumolysin (ST3-dPly) conjugate, ST3-tetrasaccharide $CRM_{197}$ (ST3-CRM) conjugate, and Prevnar13® vaccine after challenge with *S.pneumoniae* serotype 3. Mice immunized with ST3-dPly or ST3-$CRM_{197}$ conjugates or Prevnar13® vaccines,

were transnasally infected with live *S.pneumoniae* serotype 3. Control group received PBS only. After 48 h they were analysed for body weight (a), body temperature (b), bacterial burden in lungs (c) and blood (d). Colony-forming units (CFU) were calculated for single animals, and values were transformed to a logarithm scale. Two animals from group vaccinated with ST3-dPly conjugated were excluded as immunization did not work correctly (lack of specific antibodies). Data are individual values from *n* = 8 mice (n=6 for ST3-dPly conjugate group) with mean displayed. Statistical analysis was performed by the Kruskal-Wallis and Dunn's multiple comparison tests.

**Figure 5** shows evaluation of mouse specific antibodies against ST3-tetrasaccharide (a) or capsular polysaccharide (b) of *S. pneumoniae* serotype 3 in mice immunized with ST3-tetrasaccharide detoxified pneumolysin conjugates or ST3-$CRM_{197}$ conjugates or Prevnar13®, used as control. Samples were analyzed by Elisa. Graphs show mean fluorescence intensity ± SD value, and individual points represent values obtained from single animals (*n* = 16) measured in triplicate.

**Figure 6** shows titers of mouse IgG antibodies specific for pneumolysin, PspA, capsular polysaccharide of *S.pneumoniae* serotype 2 (ST2-CPS) and serotype 3 (ST3-CPS) in post-immune (*n* = 6) and final bleeding (*n*=12) serum from mice intranasally immunized with conjugates of ST3-tetrasaccharide with dPly (ST3-dPly) or PspA (ST3-PspA), or a mixture of the two conjugates (ST3-dPly + ST3-PspA), or with conjugates of ST2-hexasaccharide with dPly (ST2-dPly) or PspA (ST2-PspA), or a mixture of the two conjugates (ST2-dPly + ST2-PspA). Immune responses elicited by the inventive conjugates were compared to those elicited by ST3-tetrasaccharide $CRM_{197}$ (ST3-$CRM_{197}$) vaccine and to the control group (PBS). Each dot represents an individual mouse. Mean ± SD values are shown.

**Figure 7** shows evaluation of mouse mucosal IgG (a) and IgA (a) antibody responses specific for capsular polysaccharide (CPS) of S. *pneumoniae* serotype 3, CPS of *S.pneumoniae* serotype 2, ST3-tetrasaccharide and ST2-hexasaccharide in nasal tissue of mice intranasally immunized with conjugates of ST3-tetrasaccharide with dPly (ST3-dPly) or PspA (ST3-PspA), or a mixture of the two conjugates (ST3-dPly + ST3-PspA), or with conjugates of ST2-hexasaccharide with dPly (ST2-dPly) or PspA (ST2-PspA), or a mixture of the two conjugates (ST2-dPly + ST2-PspA). Immune responses elicited by the inventive conjugates were compared to those elicited by ST3-tetrasaccharide $CRM_{197}$ (ST3-$CRM_{197}$) and to the control group (PBS). Antibody levels were evaluated by glycan array. Each dot represents an individual mouse (n = 12). Mean ± SD values are shown.

**Figure 8** shows evaluation of mouse mucosal IgG (a) and IgA (b) antibody response to pneumolysin and PspA carrier proteins in nasal tissue of mice intranasally immunized with conjugates of ST3-tetrasaccharide with dPly (ST3-dPly) or PspA (ST3-PspA), or a mixture of the two conjugates (ST3-dPly + ST3-PspA), or with conjugates of ST2-hexasaccharide with dPly (ST2-dPly) or PspA (ST2-PspA), or a mixture of the two conjugates (ST2-dPly + ST2-PspA). Antibody levels were evaluated by Elisa. Each dot represents an individual animal (n = 12). Mean ± SD values are shown. Legend as for Figure 7.

**Figure 9** shows protection conferred in mice against *S. pneumoniae* serotype 2 by intranasal vaccination with ST2-hexasaccharide (homologous glycan) or with ST3-tetrasaccharide (heterologous glycan) conjugated to dPly or PspA. Results show bacterial recovery (CFU/ml) of *S. pneumoniae* serotype 2 (D39) from nasal tissue 3 days after the intranasal challenge of C57BL/6 mice (*n* = 12) with $3.5 \times 10^6$ CFU. Each dot represents a single animal and bars show the group median ±SD. Statistical significance was analyzed using one-way ANOVA with multi comparison test; *p < 0.001, **p<0.0001.

**Figure 10** and (b) pneumolysin carrier proteins, as measured by ELISA. Antibody levels were analyzed in pigs immunized with ST3-conjugates, or Prevnar13®, or in control pigs at day 0, day 10 (first boost), day 20 (second boost), and day 35-36 (final bleeding). Graphs shows data obtained from single animals, and their mean±SD at each time point; *n* = 6 animal per group.

**Figure 11** shows evaluation of antibody immune response of pigs immunized with conjugates of ST3-tetrasaccharide to detoxified pneumolysin or $CRM_{197}$ in blood samples collected at the final time point. Antibody response against (a) synthetic *S.pneumoniae* ST3-tetrasaccharide, (b) *S.pneumoniae* ST3 native CPS, (c) $CRM_{197}$ and (d) native pneumolysin, measured in serum samples obtained from pigs vaccinated with Alum alone, Prevnar13®, ST3-tetrasaccharide $CRM_{197}$ conjugate, or ST3-tetrasaccharide dPly conjugate. Mean fluorescence intensity (MFI) of six animals measured in triplicates were plotted. Data were analyzed by t-test, *p < 0.05.

**Figure 12** shows *in vitro* opsonophagocytic killing activity of serum samples from immunized pigs with ST3-tetrasaccharide dPly or ST3-tetrasaccharide $CRM_{197}$ conjugates. The commercial vaccine Prevnar13® and Alum only were used as a positive and negative control, respectively. a) Comparison of killing activity of sera from immunized pigs at increasing dilutions. Data represent bacterial CFU reduction in percentage of negative control wells (samples lacking either antibody or complement). Means ± SD of two independent experiments. b) Evaluation of antibody titers of serum samples responsible for 50% killing of bacteria in the opsonophagocytic killing assay (OPKA). Antibody titers are evaluated on the basis of the serum dilution mediating 50% bacterial killing, estimated through non-linear interpolation of the OPKA data. Human reference serum 007sp was used as a control.

**Figure 13** shows inhibition of pneumolysin induced hemolysis by serum samples, expected to contain anti-pneumolysin antibodies, from mice immunized with ST3-tetrasaccharide detoxified pneumolysin (ST3-dPly) conjugates.

Hemoglobin release is expressed as the absorbance at 540 nm of assay supernatants (mean ± SD). a) Inhibition of hemolytic activities of native pneumolysin. b) Evaluation of serum independent inhibition of pneumolysin hemolytic activities using whole bacteria cell lysates of *S. pneumoniae* serotype 2, 3 and 8. The statistical significance was calculated by paired t-test.

**Figure 14** shows that serum samples from mice immunized with ST3-tetrasaccharide detoxified pneumolysin conjugate inhibit pneumolysin-induced epithelial barrier disruption. A549 cells were treated with whole cell lysates of *S. pneumoniae* serotype 2, 3 and 8 with or without serum samples from mice immunized with ST3-dPly (collected on day 35). Transcellular electrical resistance was continuously monitored and normalized to baseline resistance. Statistical significance was calculated by unpaired t-tested between bacteria lysate with and without antibodies. * $p<0.0001$.

**Figure 15** shows inhibition of pneumolysin induced lysis of human red blood cells by serum samples of immunized pigs. Comparison was done between animals immunized with ST3-tetrasaccharide $CRM_{197}$ conjugate or with ST3-tetrasaccharide detoxified pneumolysin conjugate, or with Prevnar13®, and alum control group, at five-fold serum dilution. Hemoglobin release is expressed as the absorbance at 540 nm of assay supernatants (mean ± SD). Data points are shown for single animals in each group (n = 6). The statistical significance was calculated by one-way ANOVA; * $p<0.05$.

## EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

## Methods

### Animal experiments

Mice experiments were conducted in accordance to the guideline of the ethic committee at the University of Greifswald, the German regulations of the Society for Laboratory Animal Science (GV-SOLAS) and the European Health Law of the Federation of Laboratory Animal Science Associations (FELASA). The trial was approved by the Landesamt für Landwirtschaft, Lebensmittelsicherheit und Fischerei Mecklenburg-Vorpommern (LALLF M-V, Rostock, Germany) and the LALLF M-V ethical board (LALLF M-V permit no. 7221.3-1-061/17). All efforts were made to minimize the discomfort of the animals and ensure the highest ethical standards.

### Pigs

Thirty healthy, six-week-old, 20 kg weight female pigs (German landrace Sus domesticus) purchased from BHZP (BHZP GmbH; An der Wassermühle 8; 21368 Dahlenburg-Ellringen) were kept at the animal facility of Friedrich-Loeffler-Institut, Bundesforschungsinstitut für Tiergesundheit Federal Research Institute for Animal Health Greifswald Insel Riems.

### Intranasal infection of mice with *S.pneumoniae* serotype 3.

*S. pneumoniae* serotype 3 (ST3) was plated from frozen stocks on Columbia Agar plates with 5% (v/v) sheep blood, grown for approximately nine hours at 37 °C/5% $CO_2$ and inoculated as single colonies in Todd Hewitt Broth with 0.5% (w/v) yeast extract (growth medium). Cultures were cultured at 37 °C/5% $CO_2$ to mid-log growth phase (OD600 = 0.3 - 0.4) and harvested by centrifugation (5000 rpm / 10 min).

Mice were anesthetized by intraperitoneal (i.p) administration of ketamine (80 mg/kg, Ketavet®, Pfizer, Berlin, Germany) and xylazine (25 mg/kg, Rompun®, Bayer, Leverkusen, Germany). Infection with *S. pneumoniae* serotype 3 (5 x $10^6$ CFU) was performed **intranasally** in a total volume of 20 μL bacteria diluted in PBS. Disease severity was evaluated at 12-hour intervals (more often if animals were severely ill) for 36 - 48 h after bacterial infection to assess appearance, behaviour, grooming, respiration and body weight. Measurement of rectal temperature was made at every time point (BAT-12 Microprobe Thermometer, Physitemp Instruments, Clifton, USA).

Mice were sacrificed 48 hours after bacteria inoculation or when they reached at least one of the predefined human endpoints criteria (body temperature <30°C; bodyweight loss >20%; cumbersome breathing; accelerated breathing in combination with staggering, pain or paleness) by exsanguination via the caudal Vena cava after i.p. injection of ketamine (160 mg/kg body weight) and xylazine (75 mg/kg). Blood was drawn from the Vena cava caudalis, and lungs as well as spleen were removed.

After blood collection, serial dilutions of blood were plated on Columbia agar plates with 5% (v/v) sheep blood and incubated overnight at 37°C in 5% $CO_2$ environment to estimate bacteria recovery (number of CFU in the blood). Blood antibody levels were monitored by glycan microarray and ELISA analysis. Lungs were homogenized by passage through a cell strainer (100 μm pores, BD Bioscience, Heidelberg, Germany) to assess the residual bacterial burden. Serial dilutions of lung homogenates were assessed for bacteria growth (CFU level) as described above.

**Intranasal infection of mice with *S. pneumoniae* serotype 2**

Three weeks after the final immunization, mice were intranasally infected with *S. pneumoniae* D39 (serotype 2). Pneumococci were cultured in advance in liquid Todd Hewitt Broth (THY, Sigma Aldrich, Missouri, USA) supplemented with 10% heat-inactivated (h.i.) FCS until mid-log growth phase (OD600nm 0.35) and stored as glycerol-supplemented aliquots at -80 °C. Appropriate amounts of aliquots were thawed on the day of infection followed by adjustment of the infection dose to 3.5 × 106 CFU in 5 μL of PBS per mouse, verified by plating on blood agar plates. For the intranasal application, mice were anesthetized by intraperitoneal injection of ketamine/xylazine (50 mg/kg weight of mouse ketamine (KetanestS, Pfizer Pharmaceuticals; Provet AG, Berlin, Germany) + 5 mg/kg weight of mouse xylazine (Rompun, Provet AG)). Three days post-infection, mice were euthanized, followed by a collection of nasal tissue, bronchoalveolar lavage (BAL), blood and spleens. BAL samples and homogenates of nasal tissue were serially diluted to determine the bacterial recovery (Log10 CFU/mL). Remaining BAL and the nasal homogenate were snap-frozen and stored at -80 °C for further analyses. Post-challenge blood samples were collected from the heart by puncture of the Ventriculus cordis dexter followed by centrifugation at 1000 × g for 20 min and storage of the collected sera at -20 °C. Isolated spleens (n = 6) were transferred to a culture medium (RPMI 1640 + 10% h.i. FCS + 1% Pen/Strep) and kept on ice until further processing. Single-cell suspensions of harvested spleens were prepared in 15 mL cell culture medium (RPMI 1640 + 10% h.i. FCS + 1% Pen/Strep) followed by the removal of red blood cells (RBC) by incubation with 5 mL RBC lysis buffer for 5 min. The reaction was stopped by the addition of 10 m cell culture medium. After centrifugation, the cells were resuspended in a 1 mL storage medium (h.i. FCS + 10% DMSO) and stored at -80 °C.

**Protein analysis: Sodium Dodecyl Sulfate Polyacrylamide Gel Electrophoresis (SDS-PAGE)**

Polyacrylamide gel electrophoresis of glycoconjugates was performed according to the standard protocol. Discontinuous SDS-PAGE was prepared according to Lämmli's protocol, using a MiniProtean system (Bio-Rad, Hercules, USA). An alkaline separating gel (375 mM Tris/HCl pH 8.8, 10 to 12% (w/v) of a 29:1 acrylamide/*N,N'*-methylenebisacrylamide mixture) and an acidic stacking gel (100 mM Tris/HCl pH 6.8, 4.5% (w/v) of a 29:1 acrylamide/*N*,*N'*-methylenebisacrylamide mixture), polymerized by the addition of TEMED and 10% (w/v) ammonium peroxodisulfate, were used. The glycoconjugates samples were dissolved in SDS-PAGE loading buffer (200 mM Tris-Cl (pH 6.8), 400 mM DTT, 8% SDS, 0.4% bromophenol blue, 40% (v/v) glycerol). An amount of 1-2 μg of glycoconjugate and $CRM_{197}$ (as a positive control) was loaded per lane and PageRuler™ Plus Prestained Protein Ladder 10 to 250 kDa (Thermo Scientific) was used as a size marker (2 μL per lane). All samples were run at 120 V and 25 mA for 90 min and stained with 0.5 % (w/v) Coomassie Brilliant Blue R-250 in 50 % (v/v) methanol and 10 % (v/v) acetic acid for 30 min. Stained gels were destained with 50 % (v/v) methanol and 10 % (v/v) acetic acid.

**Matrix-assisted laser desorption/ionization with time-of-flight detection mass spectrometry MALDI-TOF MS.**

Mass spectra were acquired with an Autoflex Speed MALDI-TOF system (Bruker Daltonics; Bremen, Germany). Samples were spotted using the dried droplet technique with 2,5-dihydroxyacetophenone (DHAP) as matrix on MTP 384 ground steel target plates (Bruker Daltonics). Samples were prepared by mixing protein sample with DHAP matrix and 2% (v/v) trifluoroacetic acid (TFA) before spotting. The mass spectrometer was operated in linear positive mode. Mass spectra were acquired over an m/z range from 30,000 to 210,000 and data was analyzed with the FlexAnalysis

software provided with the instrument.

**Glycan array serum screening**

Individual synthetic oligosaccharide fragments of *S.pneumoniae* CPS, native CPS and related proteins were dissolved in sterile printing buffer (50 mM sodium phosphate buffer, NaPi, pH 8.5) to the final concentration of 100 - 200 μM (volume around 20-50 μL). Compounds were spotted onto CodeLink N-hydroxysuccinimide-activated glass slides (SurModics Inc., Eden Prairie, USA) in two or three replicates using a contact-free piezoelectric microarray spotter (Scienion, Berlin, Germany). After the spotting was finished, slides were incubated for 16 to 24h in a humidity box at room temperature to allow the completion of the coupling reaction and quenched for 1 h at room temperature (using 100 mM ethanolamine in 0.1 M NaPi pH 9) to suppress the free reactive groups on the microarray surface. The slide was washed with water $ddH_2O$, dried by centrifugation (5 min at 300x g) and stored in at 4°C until use.

Directly before the assay, the slide was blocked with the blocking buffer (1 % (w/v) BSA in PBS) for one hour at room temperature or overnight at 4°C. A FlexWell grid (FlexWell 64, Grace Bio-Labs, Bend, US) was attached and 20-40 μL of the serum samples were applied into wells. The slide with serum samples was incubated for 1h at 37°C in a light-protected humidified box. The microarray was washed three times by applying 50 μL of washing buffer (PBS + 0.1% Tween-20) into every well.

The secondary fluorescently labeled antibodies diluted in sample buffer (1% BSA (w/v) in PBS) were added (see secondary antibodies dilution in Table 1) and incubated for 1h at 37°C. The slide was washed three times with 50 μL PBS + 0.1% Tween-20 and $ddH_2O$ and dried by centrifugation (5 min at 300x g).

The fluorescence read-out was performed using an Axon GenePix 4300A microarray scanner and GenePix Pro 7 software (Molecular Devices, Sunnyvale, CA, USA). Image analysis was carried out with the GenePix Pro 8 software (Graphpad Software Inc., La Jolla, USA). The photomultiplier tube (PMT) voltage was adjusted such that scans were free of saturation signals.

**Table 1. Fluorescently labeled antibodies used for glycan array and Elisa**

| Assay | Antibody | Provider | Catalog number | Dilution |
|---|---|---|---|---|
| Glycan array | Anti-mouse IgG (H+L) FITC | Life Technologies, Carlsbad, CA, USA | A-31574 | 1:400 |
| | Anti-mouse IgG1 Alexa Fluor® 594 | Life Technologies, Carlsbad, CA, USA | A-21125 | 1:400 |
| | Anti-mouse IgG2a (y2a) Alexa Fluor® 647 | Life Technologies, Carlsbad, CA, USA | A-21241 | 1:400 |
| | Anti-mouse IgG3 (y3) Alexa Fluor® 488 | Life Technologies, Carlsbad, CA, USA | A-21151 | 1:200 |
| | Anti-mouse IgM (μ chain) Alexa Fluor® 546 | Life Technologies, Carlsbad, CA, USA | A-21045 | 1:200 |
| Elisa | Anti-mouse IgG (Fc-specyfic) HRP | Dianova, Hamburg, Germany | 115-035-164 | 1:10,000 |
| | Anti-mouse IgM (μ chain) HRP | Life Technologies, Carlsbad, CA, USA | M-31507 | 1:3000 |

**Enzyme-Linked Immunosorbent Assay (ELISA) for capsular polysaccharide or for carrier proteins pneumolysin or PspA.**

ELISA was performed using high-binding 96-well polystyrene microtiter plates (Corning, USA) coated with different capsular polysaccharides (SSI Diagnostica, Kopenhagen) at concentration 10 μg/mL (50 μL per well) in PBS (overnight incubation at 4°C), or with detoxified pneumolysin (PlyW433E) or PspA. The plates were washed three times with PBS + 0.1% Tween-20 and blocked with 1% BSA-PBS at RT for 1 h. After three washing steps with PBS + 0.1 % Tween-20, plates were incubated with serial dilutions of serum in duplicate or triplicate for 1 h at 37°C. The plates were washed with PBS + 0.1% Tween-20 and treated with horseradish peroxidase (HRP)-labeled secondary antibody diluted in 1 % BSA-PBS (see secondary antibodies dilution in Table 1) followed by incubation for 1 h at 37°C. The plates were washed three times with PBS + 0.1% Tween-20 and the color was developed using HRP substrate 3,3',5,5'-tetramethylbenzidine (TMB substrate; BD Biosciences, San Jose, USA). The reaction was stopped by quenching with 2% $H_2SO_4$. The absorbance was recorded at 450 nm using a standard ELISA plate reader.

***In-vitro* opsonophagocytic killing assay (OPKA).**

The *in-vitro* opsonophagocytic killing assay was performed using a standard protocol (**Figure 12**). Briefly, effector human leukemia HL-60 cells, were differentiated at concentration of $4\times10^5$ cells/mL in complete RPMI cell culture medium (90% RPMI 1640, 10% FCS, 1 mM L-glutamine and penicillin-streptomycin solution; PAN Biotech, Germany) added of 0.8 % dimethylformamide (DMF; 99.8% purity; Fisher Scientific, Fair Lawn, N.J., USA) for 5 - 6 days at 37°C in the presence of 5% $CO_2$. After differentiation, the cells were harvested by centrifugation (300 $\times$ g, 5 min) and then viable cells were counted by using 1% trypan blue exclusion and resuspended in opsonophagocytic buffer (HBSS with $Ca^{2+}$ and $Mg^{2+}$, 0.1% gelatin, and 10% FBS; HyClone) at a density of 1 x $10^7$ cells/mL directly before use. The frozen stock of *Streptococcus pneumoniae* previously grown in growth medium at 37°C / 5% $CO_2$ to mid-log phase ($OD_{600}$= 0.3 - 0.4), was diluted in the assay buffer to a final density of $10^6$ CFU/ml. Individual or pooled pig serum samples (10 μL per well) were heat-inactivated and aliquoted in round bottom non-treated 96-well plates at four-fold dilution intervals (serum serial dilutions from 1:8 to 1:8192) and treated with the bacterial suspensions (20 μL per well) to initiate opsonization (incubation for 15 min at 37°C). After preopsonization, 10 μL of external complement source (baby rabbit complement, CedarLane, Ontario, Canada) as well as $4\times10^5$ differentiated HL-60 cells in a volume of 40 μL were added to each well (phagocyte/bacteria ratio 400:1) and plates were incubated for 45 min at 37°C in 5% $CO_2$ environment (preferably with shaking at 220 rpm). The phagocytic reaction was stopped by putting the plate on ice for 20 min. Viable extracellular pneumococci were determined by plating aliquots (5 μL) from each well on Columbia Agar plates with 5% (v/v) sheep blood (BD, New Jersey, USA) and incubating at 37°C in 5% $CO_2$. for several hours to allow bacteria growing (6-8 in case of *S.pneumoniae* serotype 3). Visible colony forming units (CFU) were counted. Negative controls lacking either antibody, HL-60 or complement, as well as standard control WHO 007sp typing serum (Human Anti-Pneumococcal capsule Reference Serum, NIBSC, Herts, UK) were used. The assay was repeated two to three times independently. Percentage killing of bacteria was calculated as CFU reduction relative to negative control wells. Serum dilution responsible for 50% killing of bacteria was estimated through non-linear interpolation of the dilution-killing OPKA data.

**Preparation of detoxified pneumolysin carrier protein (dPly)**

Transformation of BL21 cells with pneumolysin plasmid

BL21 competent *E. coli* cells were used for transformation with the plasmid expression vector containing *S. pneumoniae* detoxified pneumolysin (dPly) mutant PlyW433E based on the pET101/D-TOPO backbone containing a polyhistidine-tag The plasmid was obtained from the work group of L.S. McDaniel, University of Mississippi Medical Center, USA (Taylor et al., PlosOne 2013, 8(4), e61300). BL21 cells were slowly thawed on ice and gently mixed to ensure the even suspension. The purified plasmid (1 μL, in a concentration range 1 - 10 ng/μL) was added directly to the BL21 cells, stirred and incubated on ice for 5 min. Next, the tubes were heated for precisely 30 s in a 42°C water bath avoiding shaking and placed on ice for 2 min. The room temperature SOC medium (Super Optimal broth with Catabolite repression, Merck, Darmstadt, Germany) in 250 μL volume was added to each tube and incubated for 60 min at 37°C while shaking at 250 rpm. Cells were plated on selective media (LB agar; 50mg/mL filter-sterilized carbenicillin).

Protein expression

A single clone of dPly transformed BL21 cells were picked and grown in selective LB medium (10 g/L Tryptone, 5 g/L Yeast Extract, 5 g/L NaCl plus, 50 mg/mL carbenicillin to an $OD_{600}$ = 0.5. Isopropyl-b-D-thiogalactopyranoside (IPTG) was added to the culture to a final concentration of 1 mM to induce protein expression. The culture was grown for an additional 4 - 5 hours and bacterial cells were collected by centrifugation for 10 min at 8,000 x g in 4°C. Cells pellet was resuspended in PBS with 1 mg/mL of lysozyme and incubated for 1 hour at 30°C. The cells were cooled for 30 mins, lysed by sonication (10 intervals; 30 sec per interval, with alternating 30s rest periods) and centrifuged for 20 min at 20,000 x g at 4°C. The supernatant containing protein was collected.

Purification of pneumolysin protein

The recombinant dPly protein expressed with a polyhistidine-tag (His-tag) was purified using the Protino® Ni-NTA Column (Macherey-Nagel, Germany) and ÄKTA pure chromatography system (GE Healthcare Life Science, Chicago, USA). The column was equilibrated with equilibration buffer (PBS, 10mM Imidazole, pH 7.4), and cell lysate with recovered recombinant dPly was loaded onto a 1 mL prepacked Ni-NTA column (1 mL/min flow rate, 0.3 MPa pressure limit). The column was extensively washed with washing buffer (PBS, 25mM imidazole pH 7.4). Next, the His-tagged dPly protein was eluted with elution buffer (PBS, 250mM imidazole, pH 7.4) and subsequently dialyzed against PBS

(pH 7.4). Before using for biological assays, the protein was concentrated using Amicon® Ultra 10K filters (Merck Millipore, Massachusetts, USA). Buffers used for purification: extraction buffer: PBS with 50mM Sodium phosphate, 300mM Sodium chloride; equilibration buffer (pH 7.4): PBS, with 10mM Imidazole; washing buffer (pH 7.4): PBS with 25mM Imidazole, elution buffer (pH 7.4); PBS with 250mM Imidazole.

Pneumolysin purity was checked by Coomassie Blue staining of SDS-polyacrylamide gels and concentration was determined using NanoDrop 1000 Spectrophotometer ($A_{280}$ absorbance was automatically translated to protein concentration based on an intrinsic standard).

**Preparation of pneumococcal surface protein A.**

Pneumococcal surface protein A (Uniprot ID Q54972) was synthetized by using the public coding sequence ENA M74122 (European Nucleotide Archive) as reported earlier (Hammerschmidt, Infect Immun 1999, 67(4): 1683-1687).

Primers incorporating an in-frame BamHI restriction site at the 5' end and a HindIII site at the 3' end were designed from the pspA sequence (GenBank accession no. M74122) in order to amplify PspA by PCR. Amplified fragments were digested with BamHI and Hindlll and ligated into similarly digested pQE30 vector DNA. Oligonucleotides forward SH33 (5'-GGATCCGAAGAAGAATCTCCCGTAGCC-3') and reverse PRP2 (5'-AAGCTTATTAACTGCTTTCTTAAGGTC-3'), were used to amplify the 5' end of PspA, resulting in the N-terminal alpha-helical region of PspA, also identified as lactoferrin binding domain, comprised between amino acids 32 to 318 of protein sequence ID AAA27018.1. His-tagged fusion proteins were purified by chromatography on Ni-nitrilotriacetic acid resins according to the protocols of the manufacturer (Qiagen).

**Red blood cell lysis assay with native pneumolysin.**

The neutralizing ability of pneumolysin-specific antibodies was tested using a red blood cell lysis assay on serum samples of immunized mice and pigs (**Figure 13a**, **and 15**, respectively). 96-well non-treated round-bottom plates were used for the test. Serum samples, 25 μL, were serially diluted in 1 % BSA/PBS. The full-length native pneumolysin was diluted to 1 μg/mL and added to each well in a volume of 25 μL. The plate was incubated for 30 min at 37°C with shaking. Human red blood cells were prepared at 1 % concentration in PBS and distributed to each well in a volume of 50 μL. The plate was incubated for 60 min at 37°C without shaking and centrifuged for 10 min at 1,000 rpm. 80 μL of supernatants from each well were transferred to a new 96-well flat-bottom plate. The absorbance was measured at 414 nm using an ELISA plate reader. Control wells containing non lysed 1% erythrocytes were used to determine the average maximum absorbance value at $A_{414}$. The hemolytic titer was defined as the reciprocal dilution of serum that corresponded to a 50% decrease in the maximum absorbance value at $A_{414}$.

**RBC lysis assay with whole bacteria cell lysates.**

The neutralizing ability of pneumolysin-specific antibodies of serum samples from mice immunized with ST3-dPly was also tested using a red blood cell lysis with bacteria cell lysates (**Figure 13b**). Cultures of *S.pneumoniae* serotype 3 (ST3), *S.pneumoniae* serotype 2 (ST2) or *S.pneumoniae* serotype 8 (ST8), were grown in 2 ml of brain heart infusion broth (37°C, 5% $CO_2$) to log phase ($OD_{600}$ = 0.6). Cells were centrifuged, washed with PBS and re-suspended in 100 μL of cell wall digestion buffer (10 mM Tris pH 7.5, 30% Sucrose, 1x protease inhibitor, 1 mg/mL lysozyme) for 3 h at 37 °C. The protoplasts were additionally lysed by sonication (samples on ice, sonication output control 4, 40W, 5 min). The lysates were centrifuged, and the supernatants were filter sterilized. Samples were filled to 1 mL a volume with F-12 K medium (Ham's F-12K Kaighn's Medium, Thermo Fisher Scientific, Massachusetts, USA). The supernatant was plated at blood agar plates to confirm the absence of bacterial colonies (Figure.

**Antibody response in pigs.**

Blood was collected on day -1, 10, 15, 20, 25 by the *V. cava cranialis* punctuation and on day 35/36 by heart punctuation. Antibody titer and their subclasses were analyzed by glycan array and ELISA as previously described (**Figures 10** and **11**). Protective activity of the antibodies was evaluated by *in vitro* opsonophagocytic killing assay OPKA (**Figure 12**). The neutralizing activity of anti-pneumolysin antibodies was analyzed by RBC lysis assay (**Figure 15**).

<u>Example 1 Preparation of ***S. pneumoniae*** **synthetic saccharide conjugates.**</u>

*S. pneumoniae* serotype 3 synthetic tetrasaccharide (ST3) and serotype 2 synthetic hexasaccharide (ST2) were conjugated to detoxified pneumolysin PlyW433E (dPly), or pneumococcal surface protein A (PspA) using a standardized protocol as described below. The conjugation conditions for PspA and dPly were optimized by using PBS pH

7.4 as a washing solution for proteins and the sugar amount corresponding to the **1:70 equivalents** (m/m protein: sugar).

Conjugation of *S. pneumoniae* oligosaccharides to dPly or Pspa, or $CRM_{197}$

*S.pneumoniae* oligosaccharides (ST3-tetrasaccharide or ST2- hexasaccharide) **(1)** and bis (4-nitrophenyl) adipate **(2)** were combined at 1 to 6.5 equivalent in DMSO and pyridine in v/v ratio 1.6 (Reaction schema below) . Then 180 equivalents of triethylamine were added, and the solution was stirred for three hours at room temperature. The resulting solution was frozen in liquid nitrogen and subsequently lyophilized to give a crude white solid. Excess bis (4-nitrophenyl) adipate was removed by washing the crude solid with chloroform (3 x 1 mL) and dichloromethane (3 x 1 mL) until no more bis (4-nitrophenyl) adipate was observed by the Thin-layer Chromatography (TLC). The resulting compound (3) was taken forward to the next reaction without additional purification.

To a pre-weighed vial of carrier protein (dPly, PspA, $CRM_{197}$ (4; Pfenex, California, USA)) was added autoclaved water. The solution was transferred to an Amicon 10K filter and centrifuged for 8 min at 10,000 rpm. The filtrate was discarded, and the remaining solution was added to the filter and centrifuged for 8 min at 10,000 rpm. The carrier protein (dPly, PspA, $CRM_{197}$) vial was washed with autoclaved water, transferred to the filter and centrifuged for 8 min at 10,000 rpm. The carrier protein (dPly, PspA, $CRM_{197}$) vial was washed with phosphate buffer (pH 8) **(4)**, transferred to the filter and centrifuged for 8 min at 10,000 rpm. Subsequently, the filter was removed, turned upside down into a clean vial and centrifuged for 2 min at 1,000 rpm, giving a colorless filtrate containing carrier protein (dPly, PspA, $CRM_{197}$).

The filtrate was added to a vial containing the tetrasaccharide-linker construct **(3)**. The resulting solution **(5)** was stirred for 18 h, after which the solution was transferred to an Amicon 10K filter and washed with sodium phosphate buffer pH 8 (2 x 400 μL). The sample was centrifuged for 8 min at 10,000 rpm, followed by additional washing with autoclaved water (3 x 400 μL). Prior to the third wash, autoclaved water (200 μL) was added to the filter with thorough mixing. A small sample (10 μL) was taken for MALDI-TOF-MS analysis. Autoclaved water (200 μL) was added to the filter and centrifuged for 8 min at 10,000 rpm. The sample was washed with PBS (400 μL). Afterward, the filter was removed and turned upside down into a clean vial and centrifuged for 2 min at 1,000 rpm. The filtrate was diluted with PBS (350 μL) and stored at 5 °C.

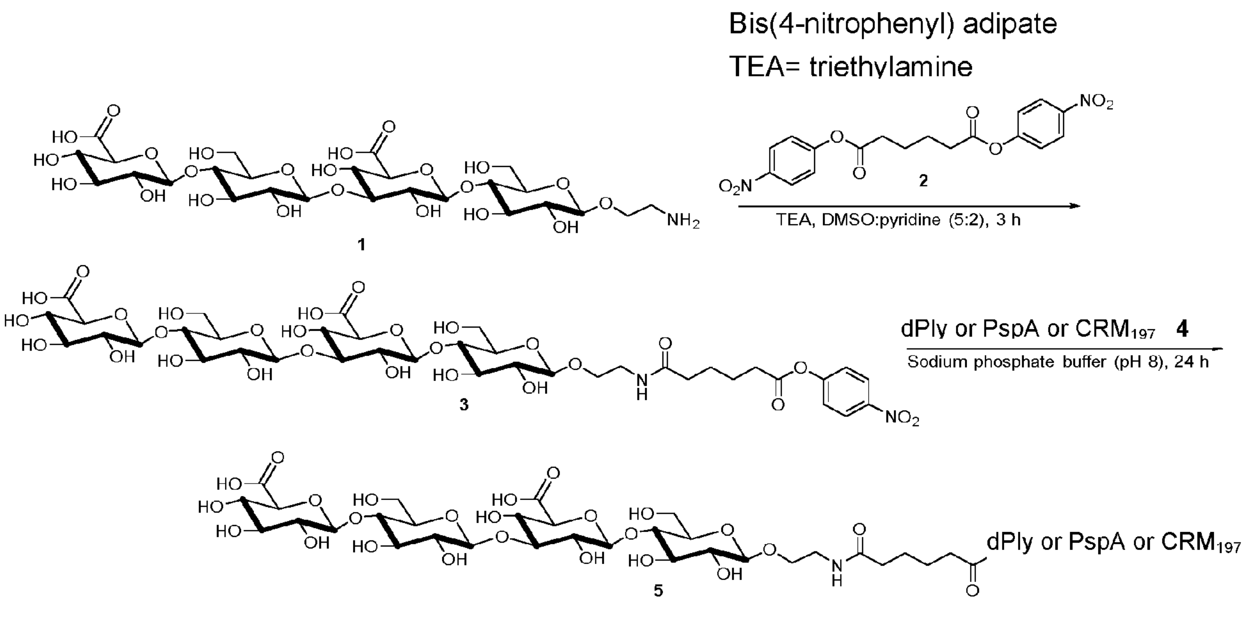


**Conjugation reaction of ST3-tetrasaccharide to a carrier protein.**

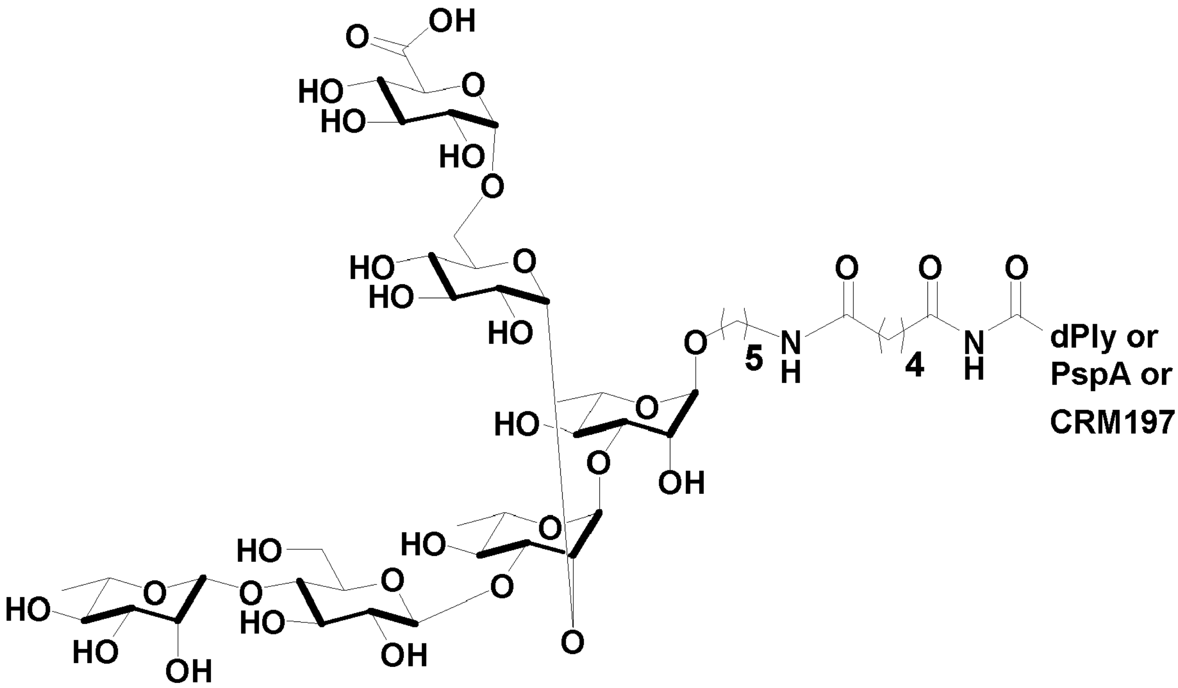

Structure of the ST2 hexasaccharide conjugates

At the end of the procedure, glycoconjugates were characterized by Sodium Dodecyl Sulfate Polyacrylamide Gel Electrophoresis SDS-PAGE (**Figure 1**) and matrix-assisted laser desorption/ionization with time-of-flight detection mass spectrometry MALDI-TOF-MS (**Figure 2**). The glycoconjugates conjugation loading ratios and the concentration of the conjugates are listed in Table 2.

**Table 2. Glycoconjugates used for the s.c. immunization of mice before the challenge with *S.pneumoniae* serotype 3.**

| Construct name | Abbreviation | Loading ratio | Concentration (mg/ml) |
|---|---|---|---|
| ST3-tetrasaccharide detoxified pneumolysin | ST3-dPly | 7 | 2.85 |
| ST3-tetrasaccharide $CRM_{197}$ | ST3- $CRM_{197}$ | 9 | 1.62 |

**Table 3. Glycoconjugates used for the nasal immunization of mice before the challenge with *S.pneumoniae* serotype 2.**

| Construct name | Abbreviation | Loading ratio | Concentration (mg/ml) |
|---|---|---|---|
| ST2-hexasaccharide $CRM_{197}$ | ST2- $CRM_{197}$ | 9.7 | 2.26 |
| ST2-hexasaccharide detoxified pneumolysin | ST2-dPly | 12.2 | 2.34 |
| ST2-hexasaccharide PspA | ST2-PspA | 12.6 | 1.4 |
| ST3-tetrasaccharide $CRM_{197}$ | ST3- $CRM_{197}$ | 11.0 | 2.56 |
| ST3-tetrasaccharide detoxified pneumolysin | ST3-dPly | 12.9 | 1.88 |
| ST3-tetrasaccharide PspA | ST3-PspA | 12.5 | 2.02 |

**Example 2 Vaccine preparation and immunization**

An amount of ST3-tetrasaccharide conjugate was mixed with 125 μg Aluminum Hydroxide ($Al(OH)_3$) adjuvant from Alum gel (Alhydrogel, Brenntag, Denmark) in a total volume of 100 μL of phosphate buffer for s.c. immunization (PBS pH 7.4, PAN-Biotech, Germany). The used amount of ST3- tetrasaccharide pneumolysin and ST3- tetrasaccharide $CRM_{197}$ conjugates corresponded to a dose of 0.4 μg of ST3-tetrasaccharide antigen. The ST3-tetrasaccharide $CRM_{197}$ conjugate was provided by Vaxxilon (Vaxxilon Deutschland GmbH, Berlin, Germany). Different antigen doses were calculated based on the glycan:protein ratio calculated with the formula (**Figure 3**):

$$\text{Loading ratio} = \frac{[\text{MW of glycoconjugate - MW of carrier protein}]}{\text{MW of glycan with linker}}$$

MW = molecular weight

The molecular weights were evaluated by MALDI-TOF-MS.

The vaccine was rotated overnight at 4°C. The conjugates were filter sterilized using 0.22 μm small volume syringe filters. Prior to the formulation, the adjuvant was aliquoted and kept sterile, every time a fresh aliquot was used.

Subcutaneous (s.c.) immunization with ST3 conjugates

Six to eight week old female C57BL/6 mice (Charles River; Sulzfeld, Germany) were immunized according to a prime + 1$^{st}$ boost + 2$^{nd}$ boost schedule, where the 1$^{st}$ boost was made two weeks after the prime, and the 2$^{nd}$ boost two weeks after the 1$^{st}$ boost, and then infected with *S. pneumoniae* serotype 3 (32 mice). Mice were vaccinated s.c. with: (1) conjugate of *S. pneumoniae* serotype 3 tetrasaccharides and dPly (PlyW433E) (ST3-Ply); (2) conjugate of *S. pneumoniae* serotype 3 tetrasaccharide and $CRM_{197}$ (ST3-$CRM_{197}$); (3) positive control of Prevnar13®, and (4) negative control as PBS only (see Table 4 below). The vaccines were prepared as described in the paragraph above.

**Table 4 Experimental groups used for the challenge with S. *pneumoniae* serotype 3 after immunization with ST3-tetrasaccharide pneumolysin conjugate or controls.**

| Group number | Vaccine | Number of animals (*n*) |
|---|---|---|
| 1 | ST3-dPly | 8 |
| 2 | ST3-$CRM_{197}$ | 8 |
| 3 | Prevnar13® | 8 |
| 4 | PBS | 8 |
| | total | 32 |

Intranasal Immunization with ST2 and ST3 conjugates

Six to eight week old female inbred C57BL/6 mice (*n* = 12, Charles River Laboratories, Sulzfeld, Germany) were immunized intranasally with a two-week interval according to the prime + boost + boost regime. Animals received the dose of glycoconjugate equivalent to 2.5 μg of protein, corresponding to approximately 0.5 μg of synthetic oligosaccharide antigen (the amount of glycan slightly varies based on a loading ratio). Conjugates of ST2-hexasaccharide or ST3-tetrasaccharide with PspA or pneumolysin (dose equal to 2.5 μg of protein) were formulated with 4 μg Cholera toxin subunit B (CTB; Sigma-Aldrich, Missouri, United States) as an adjuvant in a total volume of 10 μL. PBS and ST3-tetrasaccharide $CRM_{197}$ conjugate with CTB were administered as a negative control. Blood samples were taken before each immunization step (n = 6) and two weeks after the last immunization (n = 6) for antibody measurements. Therefore, mice were punctured in the facial vein following gentle anesthesia with 2.5% isoflurane/oxygen mixture (Baxter, Illinois, United States).

Example 3. Protection against ***S. pneumoniae* serotype 3 in mice immunized with ST3-tetrasaccharide pneumolysin conjugates or $CRM_{197}$ conjugates.**

To determine whether conjugates of ST3-tetrasaccharide with detoxified pneumolysin or $CRM_{197}$ are functional

in a disease setting, mice were s.c. immunized with a dose of glycoconjugates corresponding to 0.4 μg of ST3-tetrasaccharide antigen following the prime + boost + boost regime as described in Example 1 and **intranasally infected** with *S. pneumoniae* serotype 3 as described in the method section. The steps of immunization and infection are listed in **Table 5.** Two groups of control mice received Prevnar13® and PBS, respectively. Clinical signs, body weight, and rectal temperature were monitored every 12 hours following a clinical score. The pulmonary bacterial outgrowth and blood bacterial load were examined after animals were sacrificed 36 and 48 hours post-infection. Antibody levels specific for ST3-tetrasaccharide and carrier proteins were analysed by glycan microarray or Elisa and correlated with disease severity.

Immunization with ST3-tetrasaccharide $CRM_{197}$ conjugate and commercial vaccine Prevnar13® significantly alleviated the disease severity, as no decrease in body weight and absence of hypothermia where observed when compared to the control PBS treated mice (**Figures 4a** and **4b**). Clinical manifestation of the diseases correlated with bacterial burden in lungs and blood (**Figures 4c** and **4d**) as well as specific antibody levels against ST3-tetrasaccharide and ST3- capsular polysaccharide (**Figure 5a, 5b**). Immunization with ST3-tetrasaccharide pneumolysin conjugate also showed a substantial positive effect on mice infected with *S. pneumoniae* serotype 3 but without statistical significance.

Importantly, mice immunized with ST3-tetrasaccharide pneumolysin conjugate showed specific antibodies against ST3-tetrasaccharide and ST3-capsular polysaccharide at levels comparable as mice immunized with ST3-tetrasaccharide $CRM_{197}$ conjugate (**Figure 5a, 5b**).

**Table 5. Immunization and infection regime for S. *pneumoniae* serotype 3 challenge study.**

| day | Action |
|---|---|
| **0** | Immunization; bleeding |
| **7** | Bleeding |
| **14** | 1$^{st}$ boosting; bleeding |
| **21** | Bleeding |
| **28** | 2$^{nd}$ boosting; bleeding |
| **35** | Bleeding |
| **42** | Bleeding |
| **49** | Infection with *S.pneumoniae* |
| **51-52** | Dissection |

Example 4 Protection against ***S.pneumoniae* Serotype 2 after nasal immunization with *S. pneumoniae* synthetic saccharide conjugates.**

One of the most critical steps in the development of pneumococcal infections is nasopharyngeal colonization by the bacteria. To evaluate the potential of *S.pneumoniae* proteins detoxified pneumolysin (dPly) and pneumococcal surface protein A, PspA, as carrier protein and as an additional antigen for glycoconjugate vaccines, the proteins were conjugated to either synthetic ST2-hexasaccharide or synthetic ST3-tetrasaccharide as described in Example 1; $CRM_{197}$ was used as control carrier protein.

ST3-tetrasaccharide dPly and PspA conjugates were used to test the serotype independent protection against pathogen colonization. ST3-tetrasaccharide $CRM_{197}$ conjugate serves as a negative control.

Pneumococcal colonization model is characterized by the bacteria present in the lower airways with an accompanying inflammatory cell response for at least 14 days post-infection without acute disease symptoms. *S. pneumoniae* D39 serotype 2 was chosen for the bacteria challenge model as it is reported one of the most suitable serotypes for pneumococcus mice colonization studies.

Six to eight weeks old female inbred C57BL/6 mice were immunized **intranasally** three times in a two-week interval with ST2-hexasaccharide or ST3-tetrasaccharide individually conjugated to dPly or PspA, or with a composition comprising both glycoconjugates as described in Example 2 (Table 6).

**Table 6. Experimental groups for *S. pneumoniae* serotype 2 (ST2) challenge study**

| Group | Vaccine | Animal number (*n*) |
|---|---|---|
| **1** | ST2-dPly | 12 |
| **2** | ST2-PspA | 12 |
| **3** | ST2-dPly + ST2-PspA | 12 |
| **4** | ST3- dPly | 12 |

(continued)

| Group | Vaccine | Animal number (*n*) |
|---|---|---|
| **5** | ST3-PspA | 12 |
| **6** | ST3-PspA + ST3- dPly | 12 |
| **7** | ST3-$CRM_{197}$ (negative control) | 12 |
| **8** | PBS (negative control) | 12 |
| | | Total = 96 |

Humoral immune responses following intranasal immunization were evaluated by measuring total antigen-specific IgG titer by ELISA and by glycan array in post-immune serum samples collected two weeks after third immunization. The results showed that intranasal immunization triggered a systemic immune response in all tested mice. Antibodies against pneumolysin, PspA and *S.pneumoniae* serotype 3 capsular polysaccharide were produced accordingly to the vaccine received (**Figure 6**). However, mice injected with ST2-hexasaccharide glycoconjugate vaccine did not show any antibody response cross-reactive to the *S.pneumoniae* serotype 2 capsular polysaccharide.

Three weeks after receiving the final dose of vaccine, mice were intranasally infected with a non-lethal dose of *S. pneumoniae* serotype 2. Three days later, animals were euthanized and live pneumococci were recovered from their nasal tissues. Mucosal immunity was evaluated. Specific antibody levels (IgA and whole IgG) against the capsular polysaccharides of *S. pneumoniae* serotype 2, the capsular polysaccharides of *S. pneumoniae* serotype 3, ST2-hexasaccharide and ST3-tetrasaccharide, and the proteins pneumolysin and PspA, were measured by glycan array and by ELISA (**Figure 7 and Figure 8**).

The immunized mice showed relatively high local immune response (both IgA and IgG) to *S.pneumoniae* serotype 3 capsular polysaccharide, synthetic ST3-tetrasaccharide and synthetic ST2-hexasaccharide, according to the received immunization. However, mice injected with ST2-hexasaccharide glycoconjugate vaccine did not show the local antibody response cross-reactive to the *S.pneumoniae* serotype 2 capsular polysaccharide (**Figure 7**). Mucosal IgA and IgG specific for pneumolysin, and PspA were also produced respectively to the vaccine received (**Figure 8**).

These results prove that the synthetic saccharide conjugates are immunogenic and able to trigger the local mucosal immune response essential for colonization inhibition.

As shown in **Figure 9**, the intranasal vaccination with ST2-hexasaccharide dPly conjugate, ST2-hexasaccharide PspA conjugate, composition of ST2-hexasaccharide dPly + ST2-hexasaccharide PspA conjugates, ST3-tetrasaccharide dPly conjugate, ST3-tetrasaccharide PspA conjugate, and composition of ST3-tetrasaccharide dPly + ST3-tetrasaccharide PspA conjugates induced a significant reduction of bacterial load in the nasal cavity compared to the control mice.

The reduction of bacterial burden in the nasal cavity induced by the different ST3 or ST2 conjugates or combinations thereof (**Figure 9**) were in agreement with the measured mucosal immune response, confirming that the mucosal immune response (IgG and IgA antibodies) is responsible for inhibition of bacteria in the nasopharynx.

**Example 5. Immunization in pig animal model**

Despite many essential differences in terms of immunology and physiology between mice and humans, the murine model still is the gold standard for early pre-clinical studies. However, mouse models reflect human diseases very poorly. Domestic pigs are very similar to humans in terms of anatomy, immunology, and physiology. Therefore, the swine model is considered a highly relevant model for vaccine development.

Herein, six-week-old healthy, female domestic pigs (German landrace *Sus scrofa*) were s.c. or intramuscular (i.m) immunized with 2.2 μg of ST3-tetrasaccharide conjugated to $CRM_{197}$ or pneumolysin and formulated with 0.125 mg of Alum (Aluminium Hydroxide, Alhydrogel). The control groups were Prevnar13® (positive control) and Alum only (negative control). Conjugates were prepared and characterized as described in the previous sections. An immunization was conducted according to the prime + boost + boost regime (**Table 7**). Blood was collected on day 0, 10, 20, and final bleeding on day 35/36 and used for antibody response analysis.

**Table 7. Pig immunization regime**

| day | Action |
|---|---|
| 0 | Immunization, bleeding |
| 10 | 1st boost, bleeding |
| 20 | 2nd boost, bleeding |

(continued)

| day | Action |
|---|---|
| 35/36 | Dissection and bleeding |

Since the immune response to protein antigens is much stronger than to carbohydrate antigens, the level of antibodies against the carrier proteins $CRM_{197}$ or pneumolysin was measured to estimate whether the immunization by itself was successful and the vaccine was immunogenic in a pig model.

These analyses showed the presence of high level of pneumolysin-specific antibodies in the group immunized with ST3-tetrasaccharide dPly conjugate (**Figure 10**). Animals injected with vaccines containing $CRM_{197}$ as carrier protein, ST3-tetrasaccharide $CRM_{197}$ conjugate or Prevnar13®, showed significantly elevated titer of anti-$CRM_{197}$ IgG. The anti-protein response was observed in the immunized group as expected, demonstrating that semi-synthetic ST3-tetrasaccharide $CRM_{197}$ and detoxified pneumolysin conjugated vaccines were immunogenic in the swine model. A detectable low level of anti - protein antibodies was observed in unrelated groups due to nonsterile housing and breeding of the animals. Those conditions are in fact preferable, as they reflect the human situation such as differences in genetic background, diverse microbiota from food and environment or contact with pathogens.

The presence of $IgG_1$ and $IgG_2$ antibodies for the *S.pneumoniae* serotype 3 ST3 tetrasaccharide and for the ST3 capsular polysaccharide (cross-specific) were examined by glycan array. The array contained synthetic and native structures spotted on the glass slides. Antibody isotype distribution of anti-carrier protein antibodies was checked as well.

Results showed that both $IgG_1$ and $IgG_2$ specific for $CRM_{197}$ were present in groups immunized with Prevnar13® or with ST3-tetrasaccharide $CRM_{197}$ glycoconjugate (**Figure 11c**), and both IgG1 and $IgG_2$ for pneumolysin in groups immunized with ST3-tetrasaccharide dPly conjugate (**Figure 11d**). For groups immunized with semisynthetic ST3-tetrasaccharide $CRM_{197}$ or dPly conjugated carbohydrate vaccines, ST3-tetrasaccharide specific IgG1 titer was significantly higher than $IgG_2$ with a $IgG_1$:$IgG_2$ ratio of 2.8 and 2.7 respectively (**Figure 11a**). This is probably due to the adjuvant used for the vaccines, as aluminium hydroxide has Th2 properties associated to IgG1 production.

The level of antibodies cross reactive for *S.pneumoniae* serotype 3 CPS was significantly elevated in the group immunized with Prevnar13®, probably because the vaccine contain also isolated CPS. Groups immunized with ST3-tetrasaccharide $CRM_{197}$ or pneumolysin conjugated glycoconjugates showed a 3.5 - fold increase in CPS-specific IgG1 and a 2 - fold increase in $IgG_2$ compared to the negative control immunized with Alum only. This study provides the first evidence for the immunogenicity of the ST3-tetrasaccharide pneumolysin conjugated glycoconjugate vaccine in a swine model. The positive results of the semi-synthetic glycoconjugate vaccine candidate in two different animal models might considerably help in advancing the vaccine to clinical trials.

## Example 6. Evaluation of protective activity in pigs by *in-vitro* opsonophagocytic killing assay

To confirm the protective activity of ST3-tetrasaccharide specific antibodies, a standard *in-vitro* opsonophagocytic killing assay was performed using *S. pneumoniae* serotype 3 and HL-60 cells, as described in details in the Method section. Human reference serum WHO 007sp served as standardized control to prove that assay was performed within an acceptable range.

The results demonstrated that serum from pigs immunized with ST3-tetrasaccharide dPly conjugate or ST3-tetrasaccharide $CRM_{197}$ conjugate vaccines have significantly higher titers of antibodies responsible for bacteria killing than the group injected with Prevnar13® (**Figure 12**). Indeed the serum dilution factors responsible for 50% killing of bacteriawas of 240 for pigs immunized with ST3-tetrasaccharide dPly, of 528 with ST3-tetrasaccharide $CRM_{197}$, and of 122 with Prevnar13®. In-vitro opsonophagocytic killing assay, the gold standard to measure the efficacy of pneumococcal vaccines proved that antibody raised in pigs immunized with semi-synthetic vaccines have protective opsonophagocytic properties.

## Example 7. Serum from immunized mice inhibit human haemolysis mediated by native pneumolysin or bacterial cell lysates.

It has been shown in many studies that anti-pneumolysin antibodies reduce the cytotoxic effects of pneumolysin and delay the onset of disease caused by *S. pneumoniae.*

Therefore, serum samples from mice immunized with ST3-tetrasaccharide detoxified pneumolysin conjugates, expected to contain anti-pneumolysin antibodies, were tested for ability to reduce the pneumolysin cytotoxic effect by *in vitro* human hemolysis assay. To this aim, polyclonal mice sera (collected on day 35) were incubated with native pneumolysin (25 ng) in the presence of human red blood cells. The hemoglobin release measured as absorbance at 540 nm indicates the damage of the red blood cell membrane induced by pneumolysin. Anti-pneumolysin antibodies

contained in collected serum showed strong toxin neutralizing activity, as they completely inhibited the lysis of red blood cells compared to serum withdrawn on day 0. The neutralizing effect was visible up to a serum dilution factor of 80 (**Figure 13a**).

Pneumolysin is a multi-functional membrane protein conserved among all *S. pneumoniae* serotypes. To demonstrate that use of the detoxified pneumolysin as a carrier protein for synthetic carbohydrate vaccines can extend serotype cross-protection by reducing the infection by other serotypes not included in the used vaccine, human red blood cells were incubated with mouse serum collected on day 35 and bacterial lysates of three different *S. pneumoniae* serotypes: 2, 3 and 8. Significant reduction of haemoglobin release was observed for all tested serotypes (**Figure 13b**) with probability values of $p = 0.0065$ for serotype 2, $p = 0.0008$ for serotype 3, and $p = 0.0261$ for serotype 8. A minor haemolysis was still detected in the serum treated samples, probably due to other toxins or lytic components contained in the bacteria cell lysates. The most significant reduction in cytotoxicity was seen with lysates of serotype 3 which might suggest the additional neutralizing effect of specific anti-glycan antibodies.

Example 8. Serum samples from immunized mice prevent pneumolysin toxicity on ***in vitro* alveolar epithelial cells.**

Epithelial cells are one of the targets of *S. pneumoniae.* They contribute to maintain the barrier function in the lung as well as in the brain. The disruption of lung epithelial cell integrity causes acute pneumonia and can lead to brain meningitis. Therefore, the effect of pneumolysin on epithelial barrier function was analyzed in-vitro. Human alveolar basal epithelial cells A549 were exposed to cell lysate of *S. pneumoniae* serotype 2, 3 and 8 and pre-incubated with or without the serum from mice immunized with ST3-tetrasaccharide dPly conjugate (blood collected on day 35). Changes in transcellular electrical resistance of cell monolayers were analyzed by Electric Cell-substrate Impedance Sensing (ECIS). Serum samples, expected to contain anti-pneumolysin antibodies, significantly inhibited the effect of pneumolysin contained in the bacterial lysates (ST2, ST3, ST8), on A549 monolayer permeability (**Figure 14**) indicated by a lower decrease in electrical resistance (R) (p-value < 0.0001 for each tested serotype).

The above results indicate that mice immunized with ST3-tetrasaccharide dPly conjugates produce high levels of pneumolysin-neutralizing antibodies, which may block acute lung injury in the early course of pneumococcal pneumonia and following respiratory failure. Therefore, antibodies against pneumolysin produced after immunization with the ST3-tetrasaccharide dPly conjugate, can have additional beneficial effects in the prevention and treatment of severe pneumococcal pneumonia.

**Example 9. Pneumolysin neutralization activity of serum samples from immunized pigs**

Serum samples from pigs immunized with the ST3-tetrasaccharide dPly conjugate showed pneumolysin toxin neutralization activity. Indeed, the serum samples were able to inhibit the hemolysis of red blood cells induced *in vitro* by pneumolysin (**Figure 15**). Nevertheless, the group that was immunized with ST3-tetrasaccharide dPly conjugate showed a statistically significant decrease of hemoglobin release compared to alum injected groups (p = 0.0185).

## Claims

1. A conjugate of a synthetic *Streptococcus pneumoniae* oligosaccharide with a carrier protein selected from detoxified pneumolysin or an immunogenic fragment thereof, and pneumococcal surface protein A, or an immunogenic fragment thereof.

2. The conjugate according to claim 1, wherein the synthetic *Streptococcus pneumoniae* oligosaccharide is selected from the group of serotypes comprising consisting of serotype 1, 2, 3, 4, 5, and 8.

3. The conjugate according to claim 1, wherein the synthetic *Streptococcus pneumoniae* oligosaccharide is of *Streptococcus pneumoniae* serotype 3.

4. The conjugate according to claim 3, wherein the conjugate has the general formula (**I**)

$$\mathbf{[H\text{-}(C)_{c3}\text{-}(D\text{-}C)_{c2}\text{-}(D)_{c1}\text{-}O\text{-}L^3\text{-}NH\text{-}W^3]_{m3}\text{-}carrier\ protein} \qquad (I)$$

wherein

C represents: 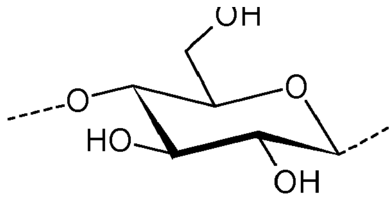

D represents: 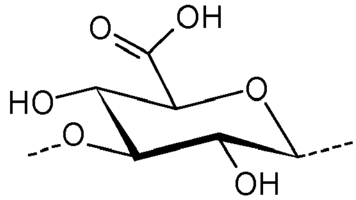

c2 is an integer selected from 1, 2, 3, 4, 5, 6, 7, 8, 9 and 10;
c1 and c3 represent independently of each other an integer which is 0 or 1;
$L^3$ represents a linker;
m3 is comprised between about 2 and about 18;
$-W^3-$ is selected from:

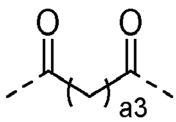 , 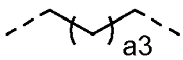 , and 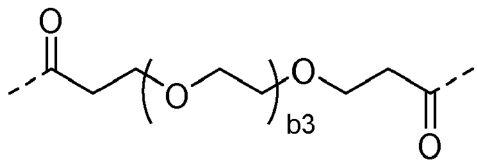 ;

a3 represents an integer from 1 to 10;
b3 represents an integer from 1 to 4; and
carrier protein has the meaning as defined in claim 1.

**5.** The conjugate according to claim 1, wherein the synthetic *Streptococcus pneumoniae* oligosaccharide is of *Streptococcus pneumoniae* serotype 2.

**6.** The conjugate according to claim 5, wherein the conjugate has the general formula (**II**)

$$\mathbf{[B^*\text{-}A_{y+3}\text{-}A_{y+2}\text{-}A_{y+1}\text{-}A_y\text{-}O\text{-}L^2\text{-}NH\text{-}W^2]_{m2}\text{-carrier protein}} \qquad (\mathbf{II})$$

wherein y is an integer selected from 1, 2, 3 and 4,

$A_1 = A_5 =$ 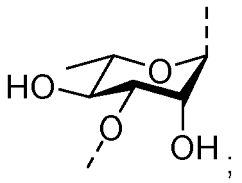 ; $A_2 = A_6 =$ 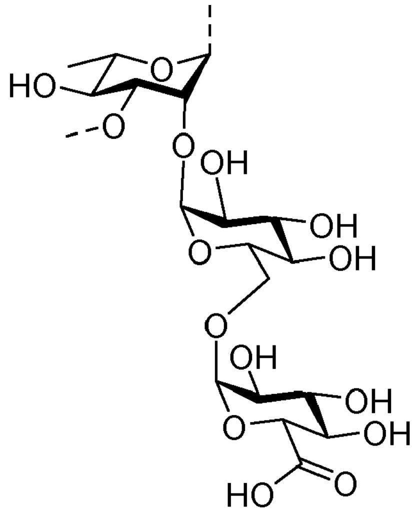 ,

$A_3 = A_7 =$ 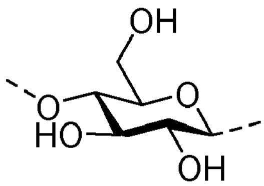 ; $A_4 =$ 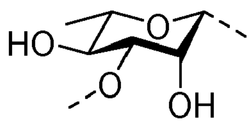 ;

B*- represents H-, H-$A_y$-, H-$A_{y+i}$-$A_y$-, H-$A_{y+2}$-$A_{y+1}$-$A_y$- or H-$A_{y+3}$-$A_{y+2}$-$A_{y+1}$-$A_y$-;
$L^2$ represents a linker;
m2 is comprised between about 2 and about 18,
-$W^2$- is selected from:

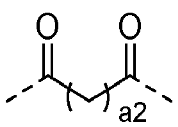 , 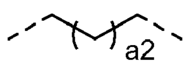 , and 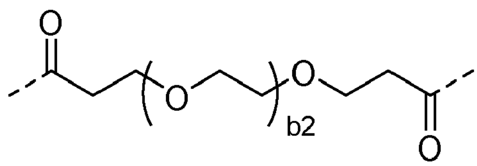 ;

a2 represents an integer from 1 to 10;
b2 represents an integer from 1 to 4; and
carrier protein has the meaning as defined in claim 1.

**7.** An immunogenic composition comprising one or more conjugates according to any one of the claims 1 - 6.

**8.** The immunogenic composition according to claim 7 comprising (i) a conjugate of a synthetic *Streptococcus pneumoniae* oligosaccharide with detoxified pneumolysin or an immunogenic fragment thereof and (ii) a conjugate of a synthetic *Streptococcus pneumoniae* oligosaccharide with pneumococcal surface protein A or an immunogenic fragment thereof.

**9.** The immunogenic composition according to claim 7 comprising a conjugate according to claim 3 or 4, and / a mixture consisting of capsular polysaccharides from *Streptococcus pneumoniae* serotypes 1, 4, 5, 6A, 6B, 7F, 9V, 14, 18C, 19A, 19F and 23F individually conjugated to $CRM_{197}$ carrier protein.

**10.** The immunogenic composition according to any one of the claims 7 - 9, wherein the one or more conjugates are selected from a conjugate of a synthetic *Streptococcus pneumoniae* serotype 1 oligosaccharide, a conjugate of a synthetic *Streptococcus pneumoniae* serotype 2 oligosaccharide, a conjugate of a synthetic *Streptococcus pneumoniae* serotype 3 oligosaccharide, a conjugate of a synthetic *Streptococcus pneumoniae* serotype 4 oligosaccharide, a conjugate of a synthetic *Streptococcus pneumoniae* serotype 5 oligosaccharide, a conjugate of a synthetic

*Streptococcus pneumoniae* serotype 8 oligosaccharide.

**11.** The immunogenic composition according to any one of the claims 7 - 10, further comprising at least one unconjugated *Streptococcus pneumoniae* protein selected from detoxified pneumolysin or an immunogenic fragment thereof, and pneumococcal surface protein A, or an immunogenic fragment thereof.

**12.** The conjugate according to any one of the claims 1 - 6 for use or the immunogenic composition according to any one of the claims 7 - 11 for use in raising a protective immune response in a human and/or animal host.

**13.** The conjugate according to any one of the claims 1 - 6 for use or the immunogenic composition according to any one of the claims 7 - 11 for use in the prevention and/or treatment of a disease caused by *Streptococcus pneumoniae*.

**14.** The conjugate according to any one of the claims 1 - 6 for use or the immunogenic composition according to any one of the claims 7 - 11 for use in the prevention and/or treatment of a disease caused by *Streptococcus pneumoniae* serotype 2 and/or serotype 3.

**15.** A vaccine comprising the conjugate according to any one of the claims 1 - 6 or the immunogenic composition according to any one of the claims 7 - 11 together with at least one pharmaceutically acceptable adjuvant and/or excipient.

# Figure 1

a)

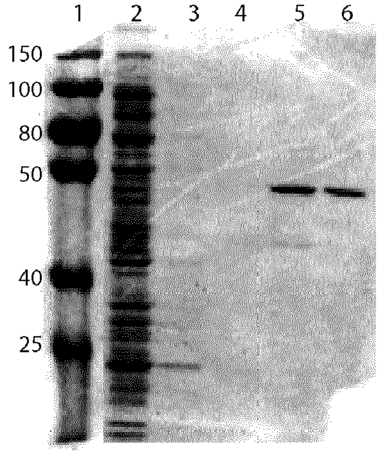


1. Protein Ladder
2. Flow-through
3. Wash 1
4. Wash 2
5. Elution 1
6. Elution 2

b)

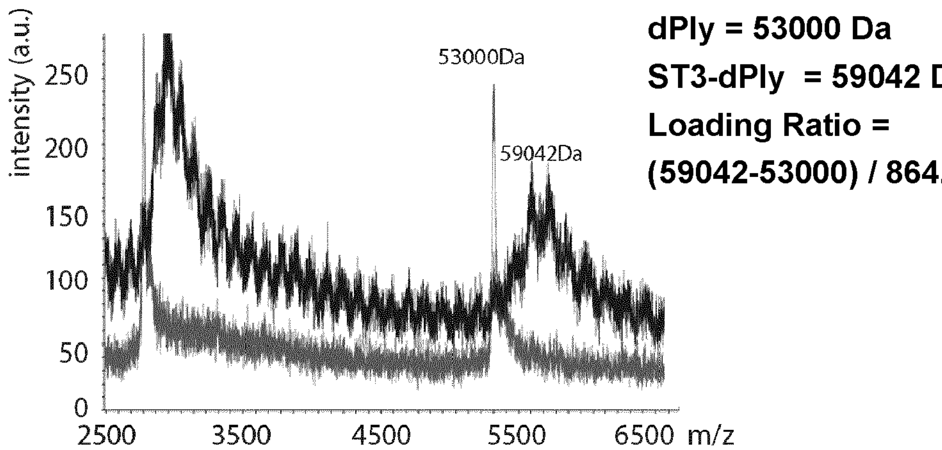


dPly = 53000 Da

ST3-dPly = 59042 Da

Loading Ratio =

(59042-53000) / 864.75 = 6.9

c)

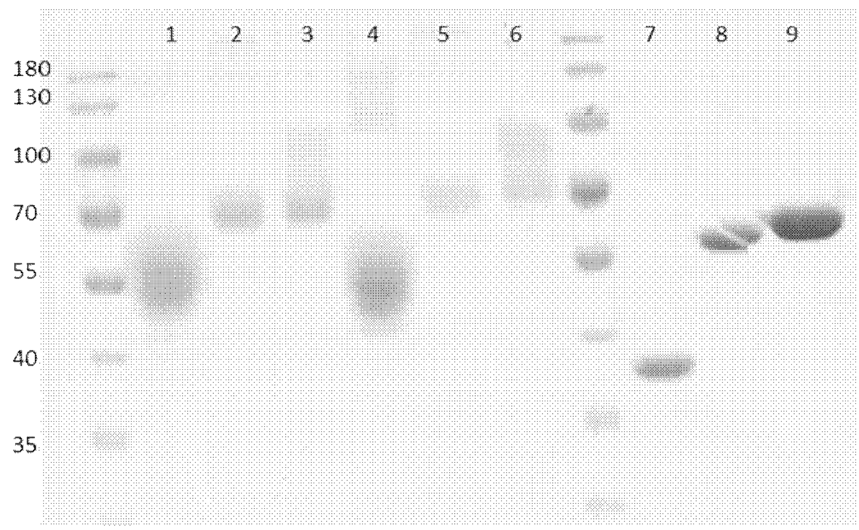

Figure 2

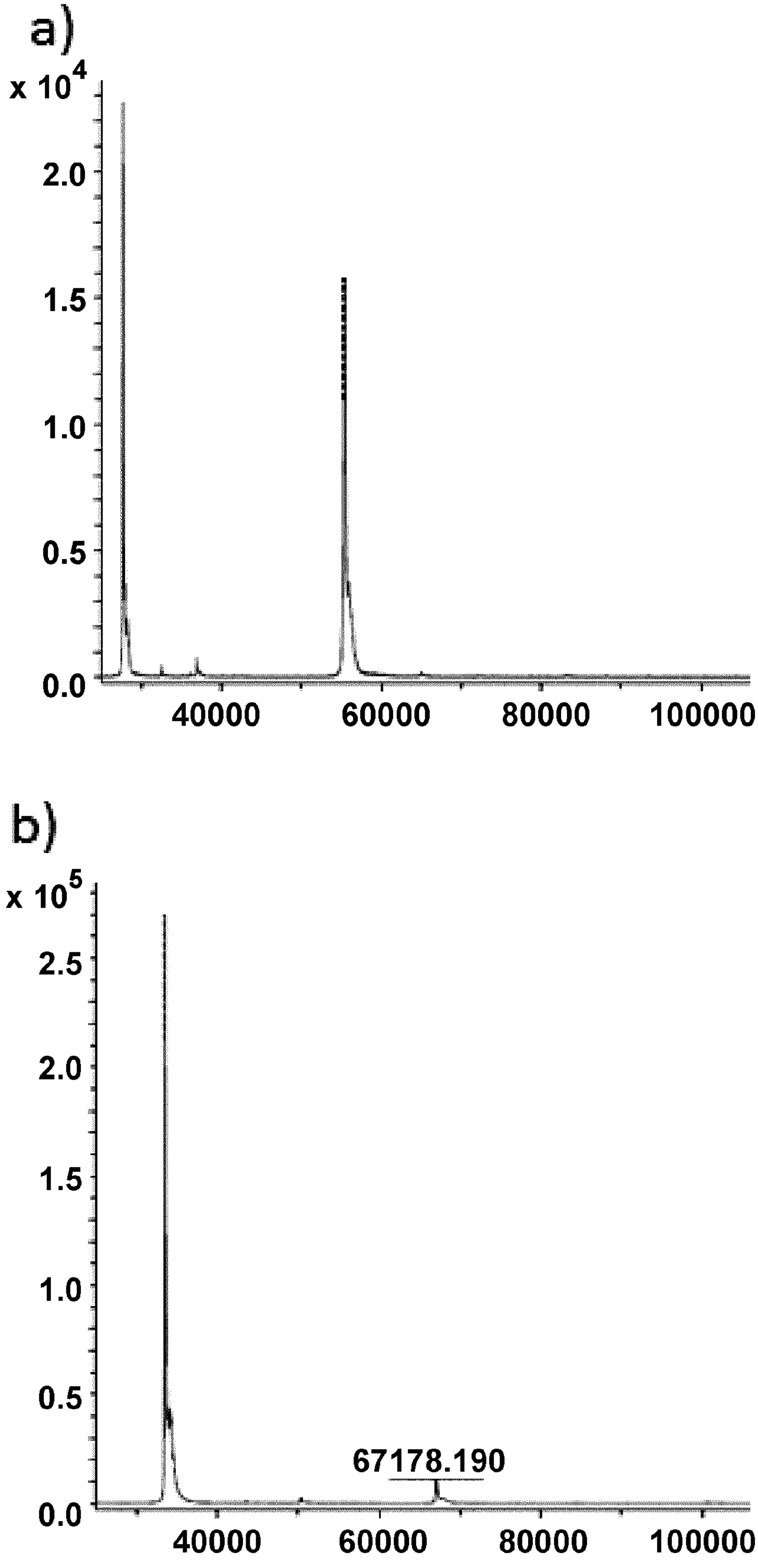
a)
x 10^4
2.0
1.5
1.0
0.5
0.0
40000
60000
80000
100000
b)
x 10^5
2.5
2.0
1.5
1.0
0.5
0.0
67178.190
40000
60000
80000
100000

Figure 2 (*continue*)

c)

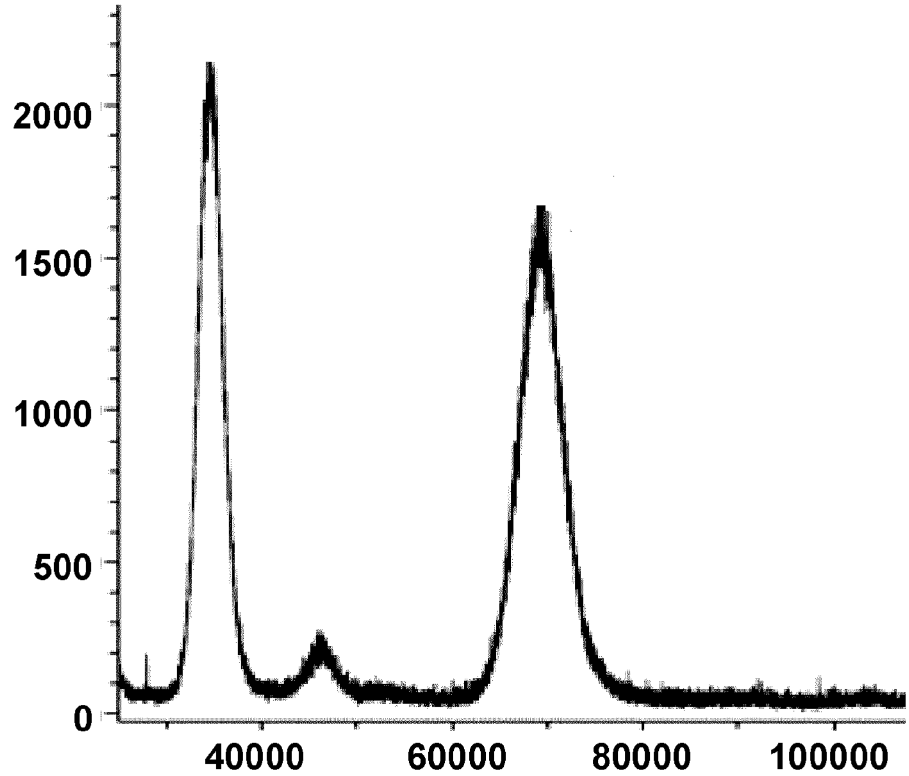


d)

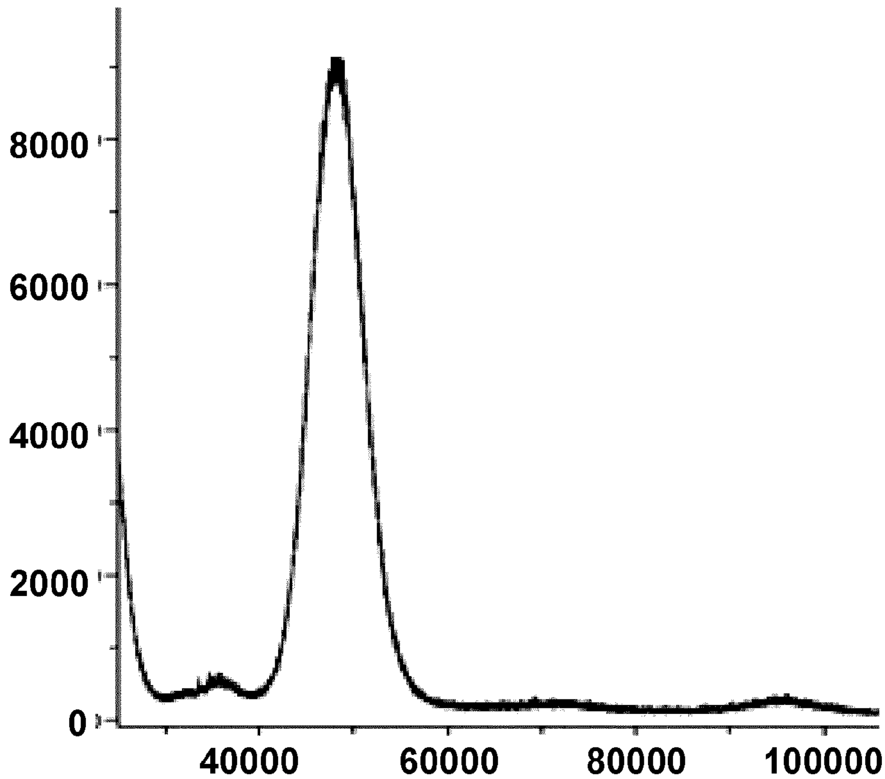

Figure 2 (*continue*)

e)

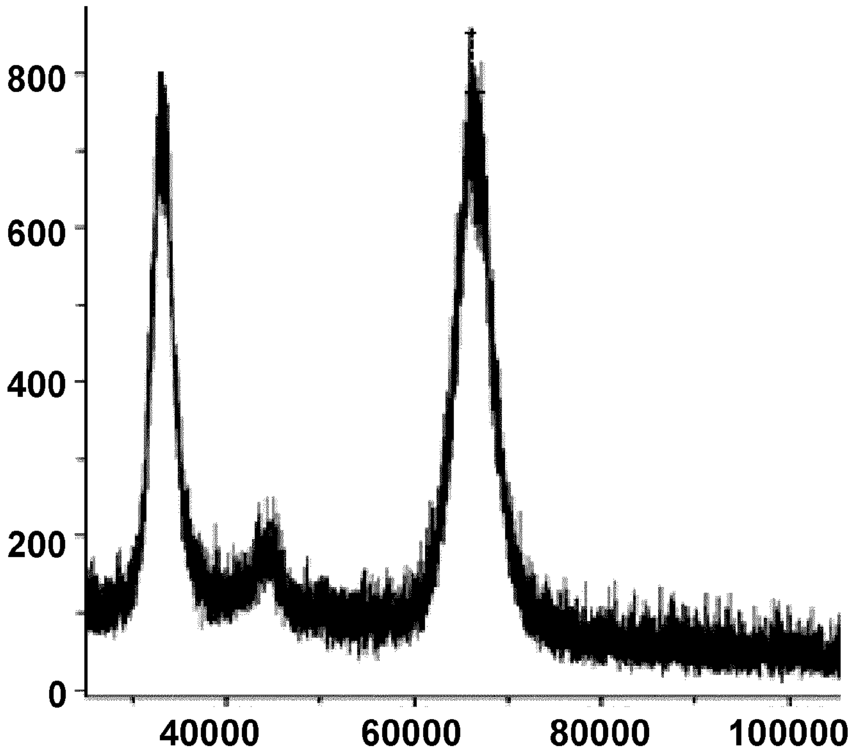


f)

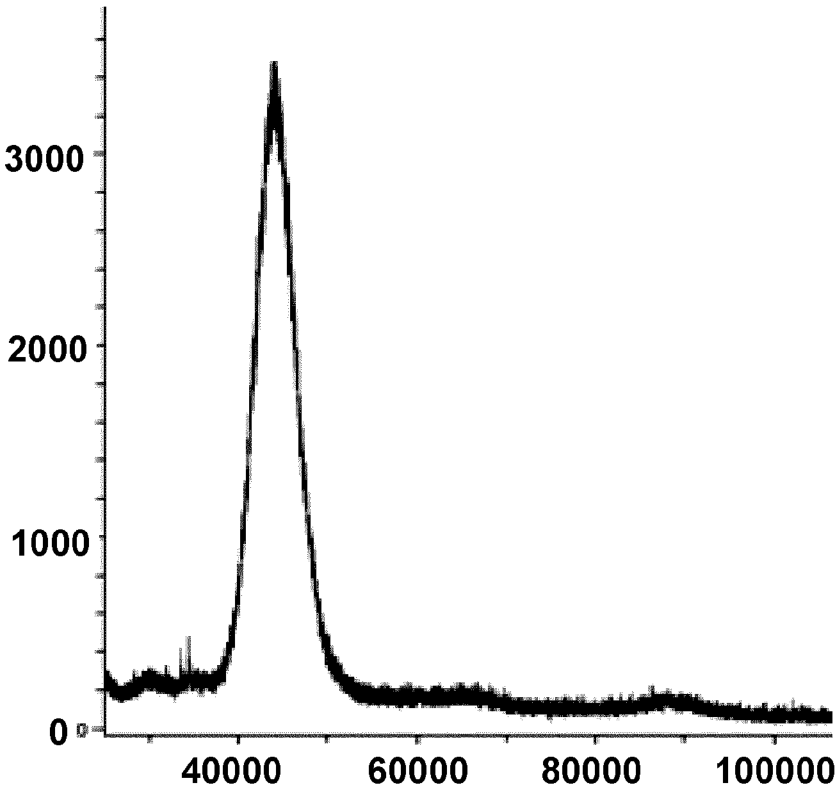

Figure 3

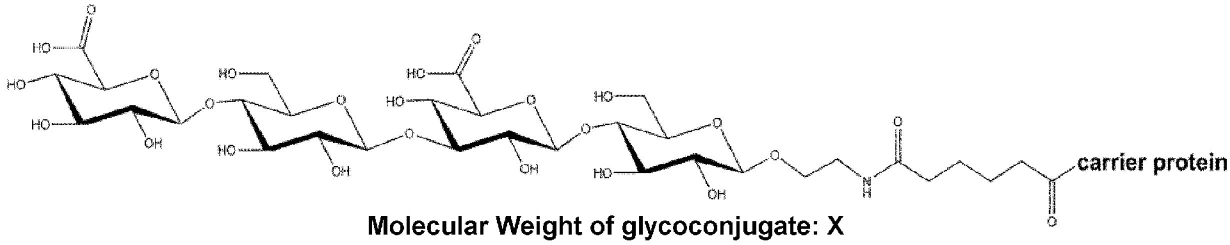

Molecular Weight of glycoconjugate: X

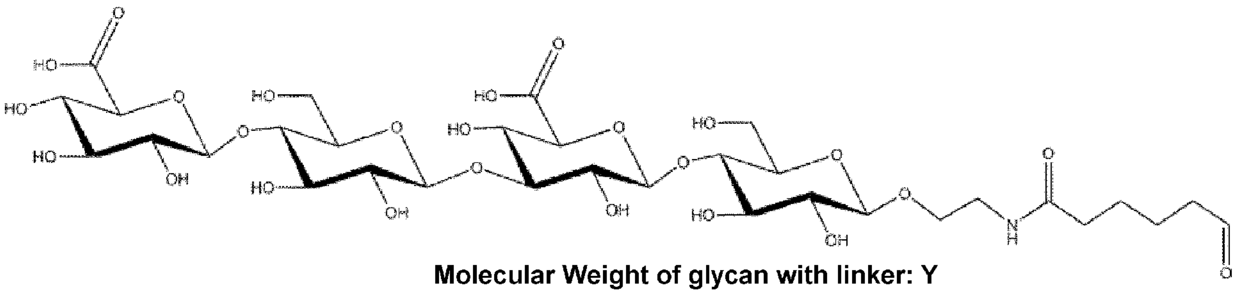

Molecular Weight of glycan with linker: Y

Figure 3 (*continue*)

Carrier protein: dPly, PspA, or CRM197

Molecular Weight of carrier protein: Z

Loading ration =
number of glycan per one protein

$$(X - Z) /Y$$

Figure 4

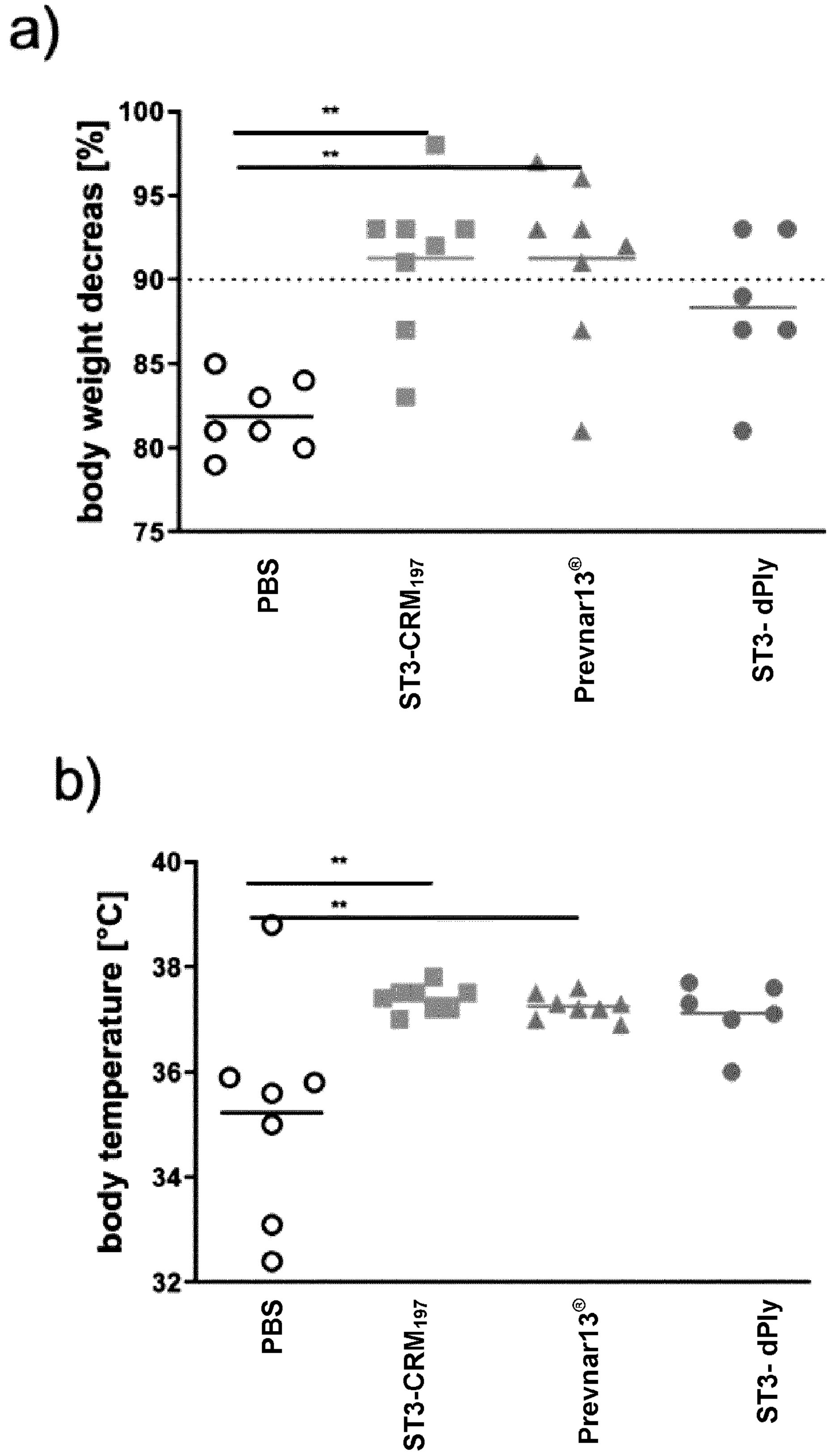
a)
body weight decreas [%]
100
95
90
85
80
75
**
**
PBS
ST3-CRM197
Prevnar13®
ST3- dPly
b)
body temperature [°C]
40
38
36
34
32
**
**
PBS
ST3-CRM197
Prevnar13®
ST3- dPly

Figure 4 (*continue*)

c)

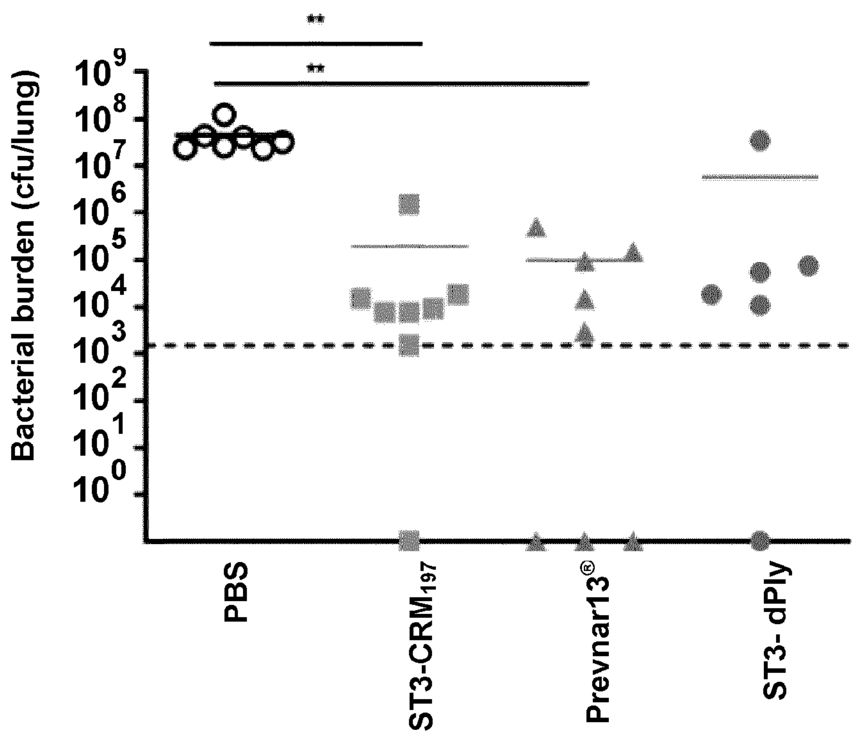


d)

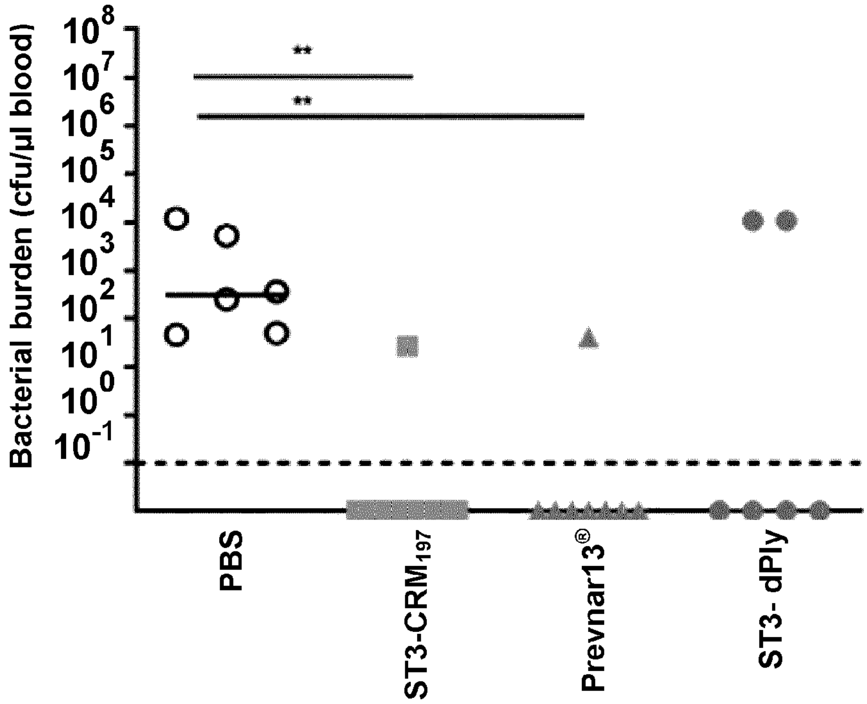

Figure 5

a)

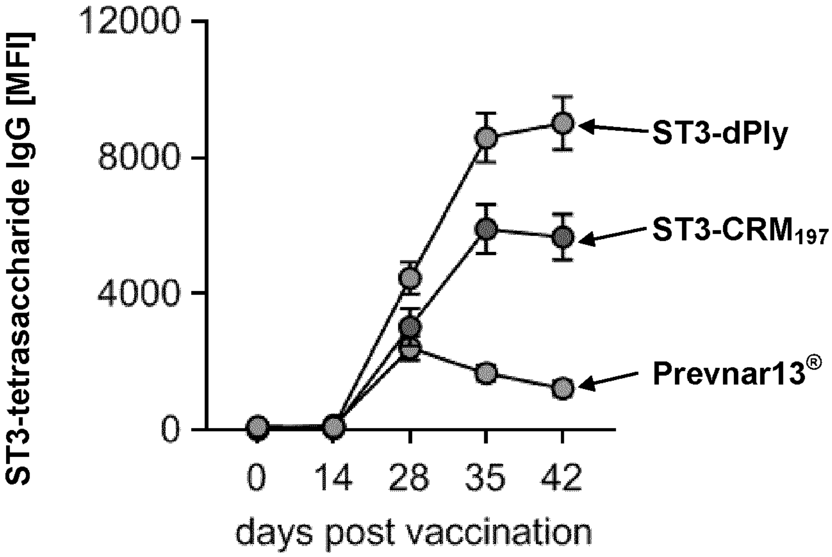
12000
8000
4000
0
ST3-tetrasaccharide IgG [MFI]
0
14
28
35
42
days post vaccination
ST3-dPly
ST3-CRM197
Prevnar13®

b)

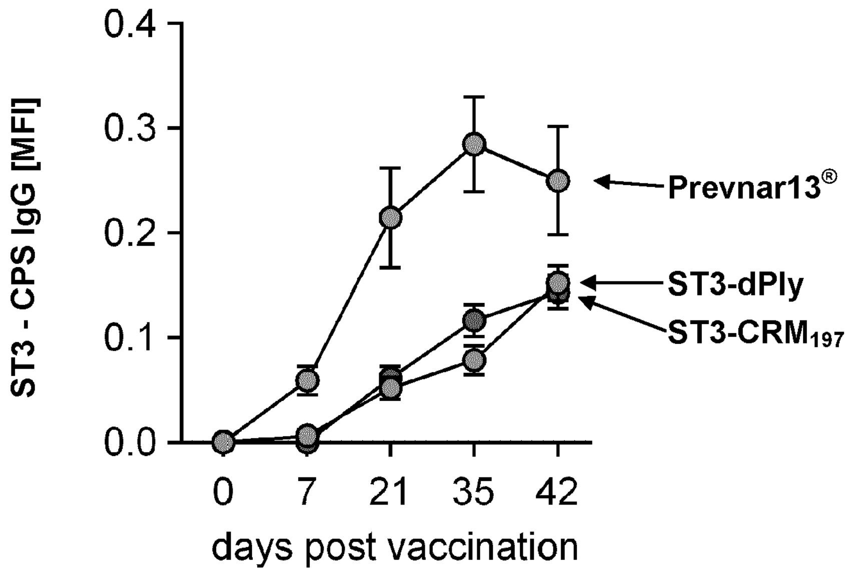
0.4
0.3
0.2
0.1
0.0
ST3 - CPS IgG [MFI]
0
7
21
35
42
days post vaccination
Prevnar13®
ST3-dPly
ST3-CRM197

Figure 6

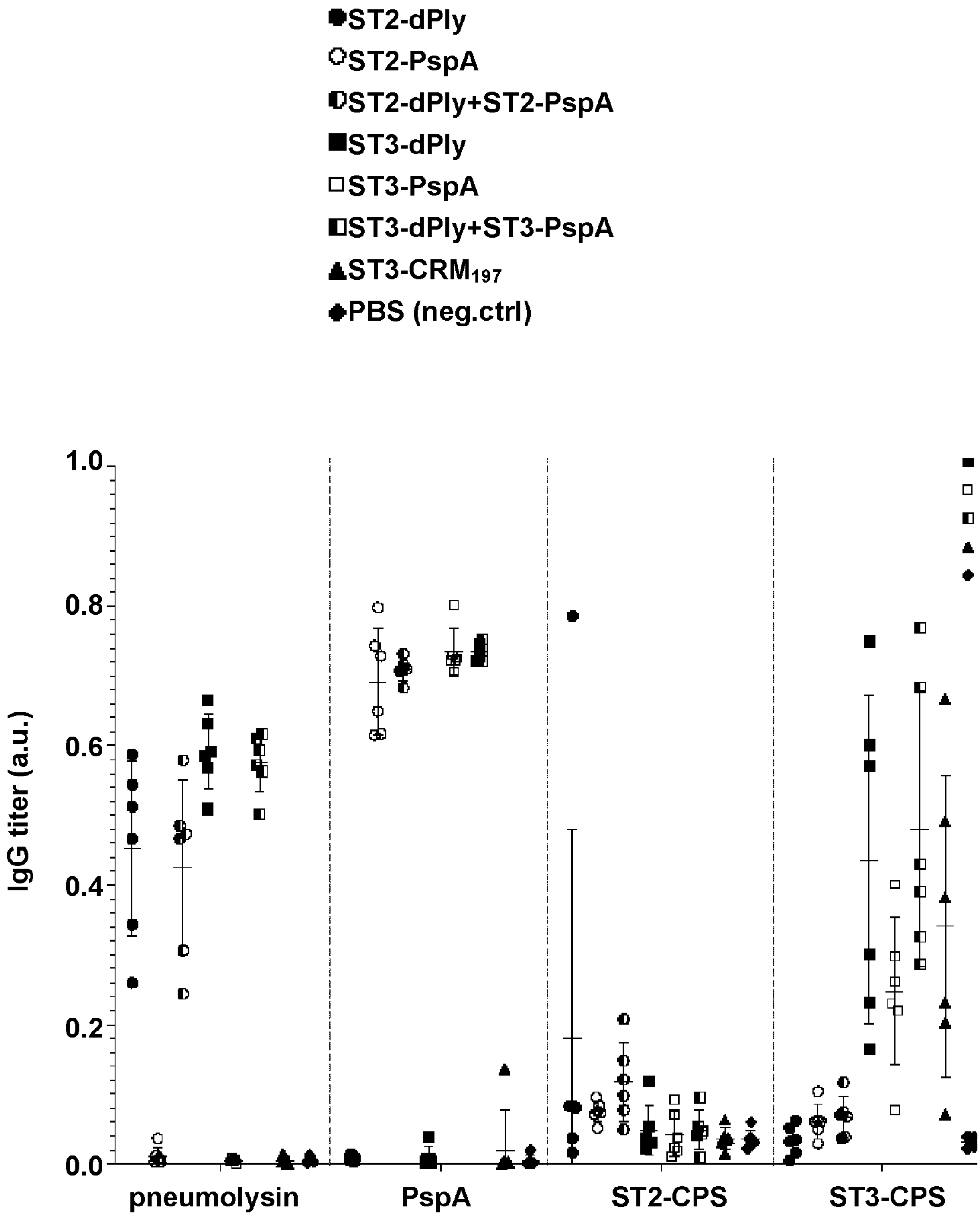
ST2-dPly
ST2-PspA
ST2-dPly+ST2-PspA
ST3-dPly
ST3-PspA
ST3-dPly+ST3-PspA
ST3-CRM197
PBS (neg.ctrl)
1.0
0.8
0.6
0.4
0.2
0.0
IgG titer (a.u.)
pneumolysin
PspA
ST2-CPS
ST3-CPS

Figure 7

a)

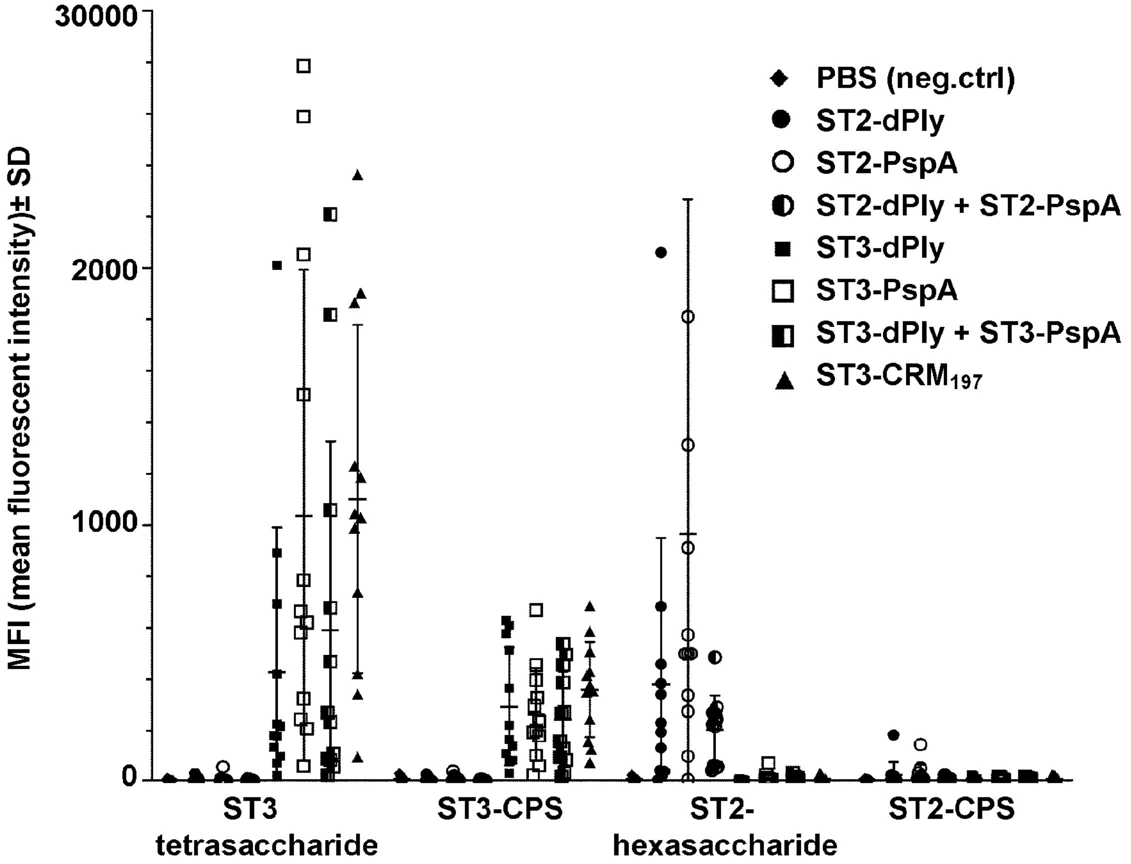
30000
2000
1000
0
MFI (mean fluorescent intensity)± SD
PBS (neg.ctrl)
ST2-dPly
ST2-PspA
ST2-dPly + ST2-PspA
ST3-dPly
ST3-PspA
ST3-dPly + ST3-PspA
ST3-CRM197
ST3 tetrasaccharide
ST3-CPS
ST2-hexasaccharide
ST2-CPS

b)

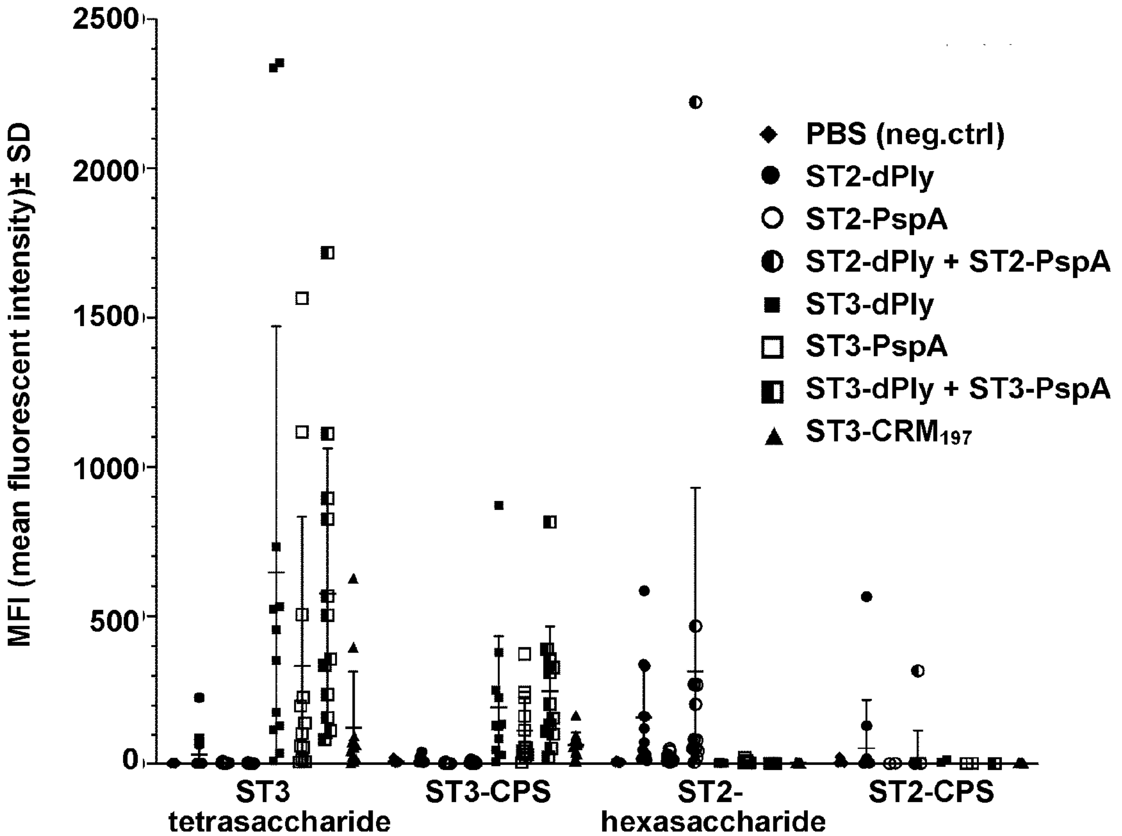
2500
2000
1500
1000
500
0
MFI (mean fluorescent intensity)± SD
PBS (neg.ctrl)
ST2-dPly
ST2-PspA
ST2-dPly + ST2-PspA
ST3-dPly
ST3-PspA
ST3-dPly + ST3-PspA
ST3-CRM197
ST3 tetrasaccharide
ST3-CPS
ST2-hexasaccharide
ST2-CPS

Figure 8

a)

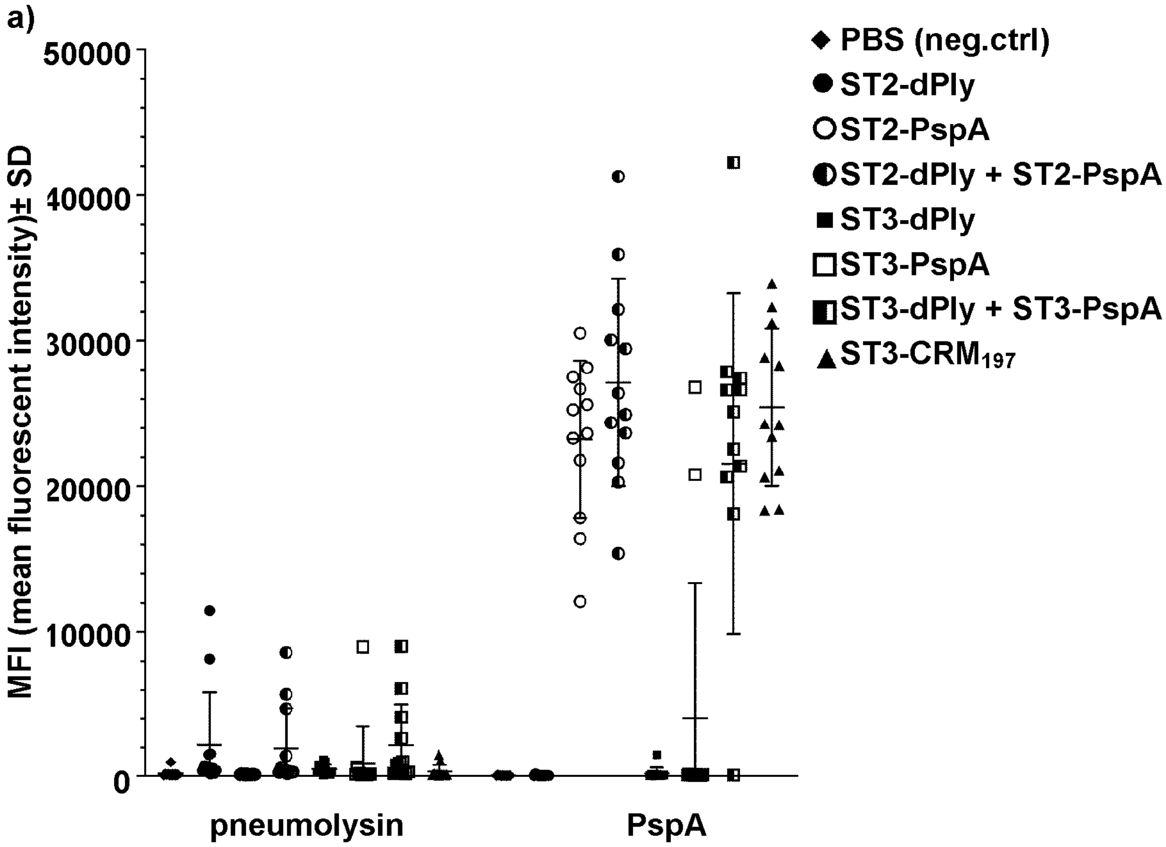
50000
40000
30000
20000
10000
0
MFI (mean fluorescent intensity)± SD
pneumolysin
PspA
PBS (neg.ctrl)
ST2-dPly
ST2-PspA
ST2-dPly + ST2-PspA
ST3-dPly
ST3-PspA
ST3-dPly + ST3-PspA
ST3-CRM197

b)

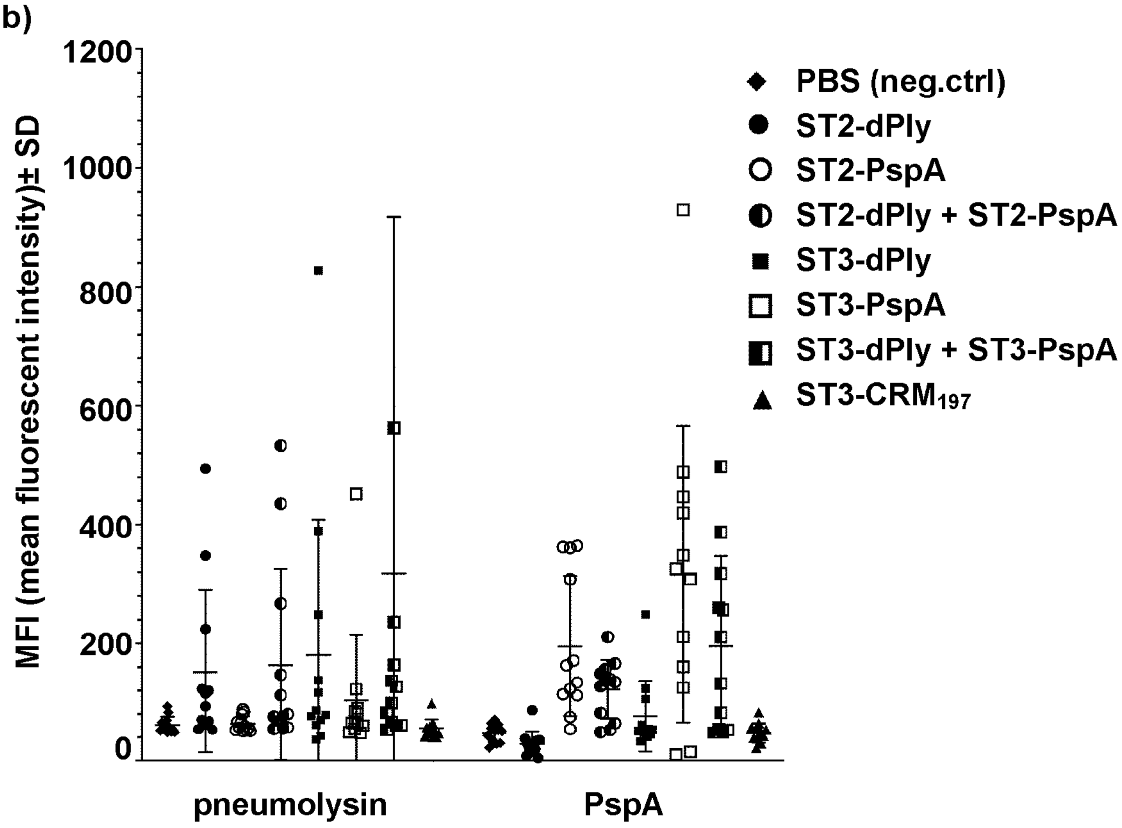
1200
1000
800
600
400
200
0
MFI (mean fluorescent intensity)± SD
pneumolysin
PspA
PBS (neg.ctrl)
ST2-dPly
ST2-PspA
ST2-dPly + ST2-PspA
ST3-dPly
ST3-PspA
ST3-dPly + ST3-PspA
ST3-CRM197

Figure 9

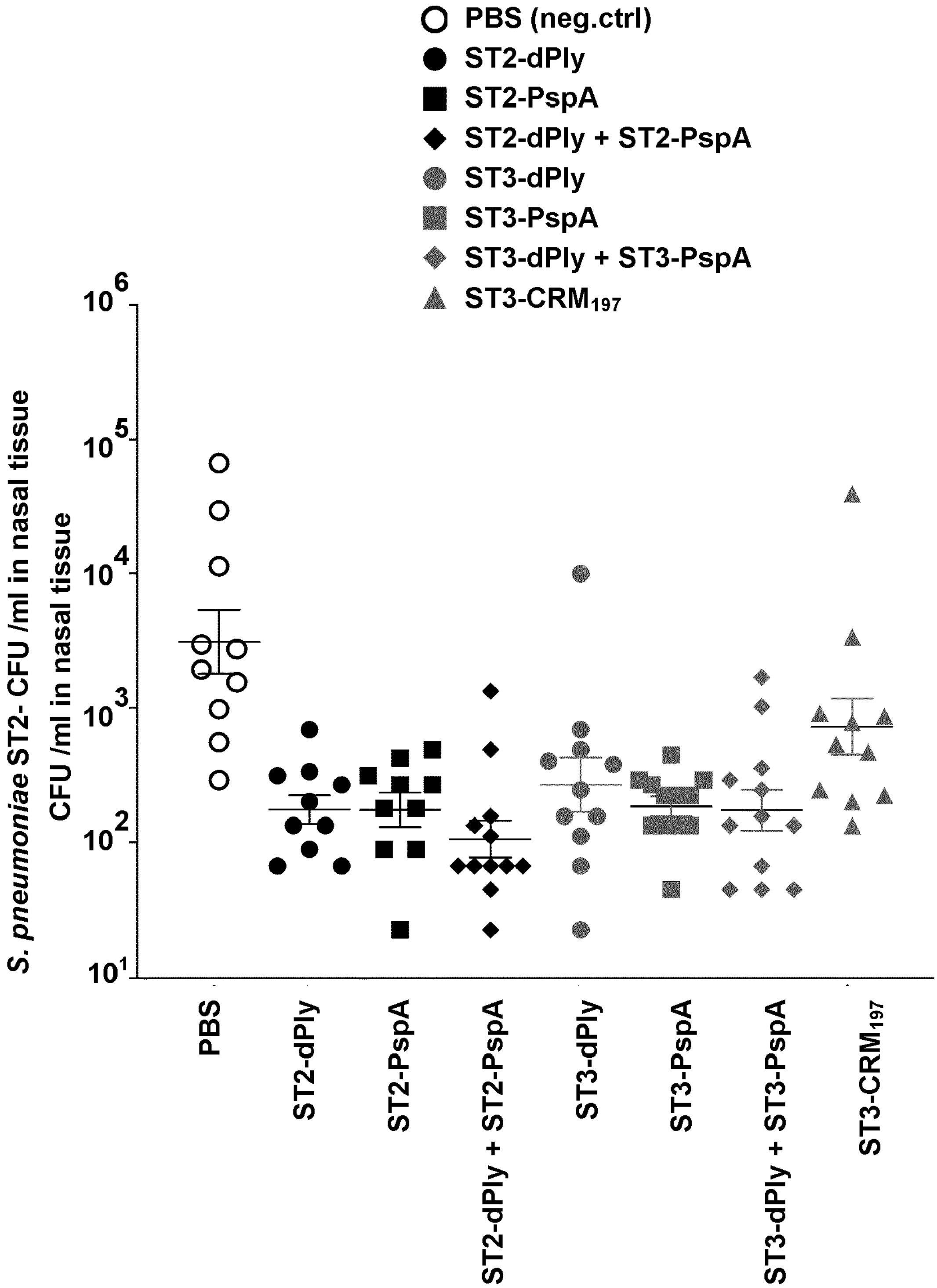
PBS (neg.ctrl)
ST2-dPly
ST2-PspA
ST2-dPly + ST2-PspA
ST3-dPly
ST3-PspA
ST3-dPly + ST3-PspA
ST3-CRM197
S. pneumoniae ST2- CFU /ml in nasal tissue
CFU /ml in nasal tissue
10^6
10^5
10^4
10^3
10^2
10^1
PBS
ST2-dPly
ST2-PspA
ST2-dPly + ST2-PspA
ST3-dPly
ST3-PspA
ST3-dPly + ST3-PspA
ST3-CRM197

**Figure 10**

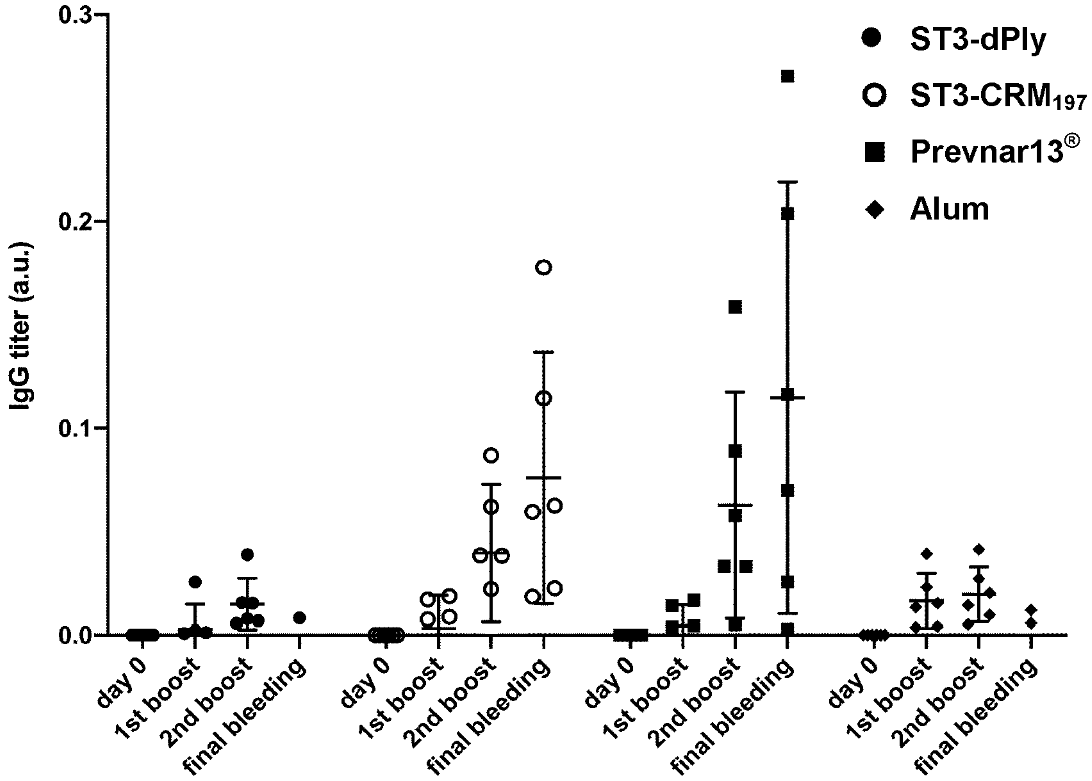

0.3
0.2
0.1
0.0
IgG titer (a.u.)
ST3-dPly
ST3-CRM197
Prevnar13®
Alum
day 0
1st boost
2nd boost
final bleeding


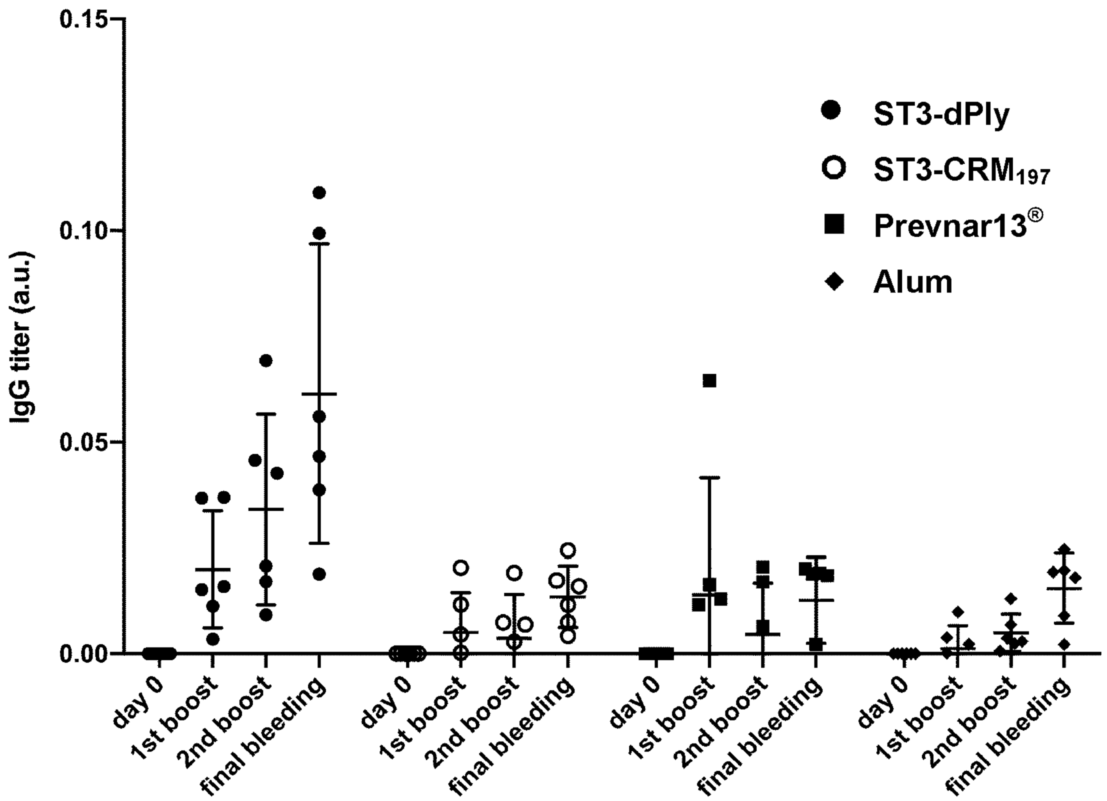

0.15
0.10
0.05
0.00
IgG titer (a.u.)
ST3-dPly
ST3-CRM197
Prevnar13®
Alum
day 0
1st boost
2nd boost
final bleeding

## Figure 11

a)

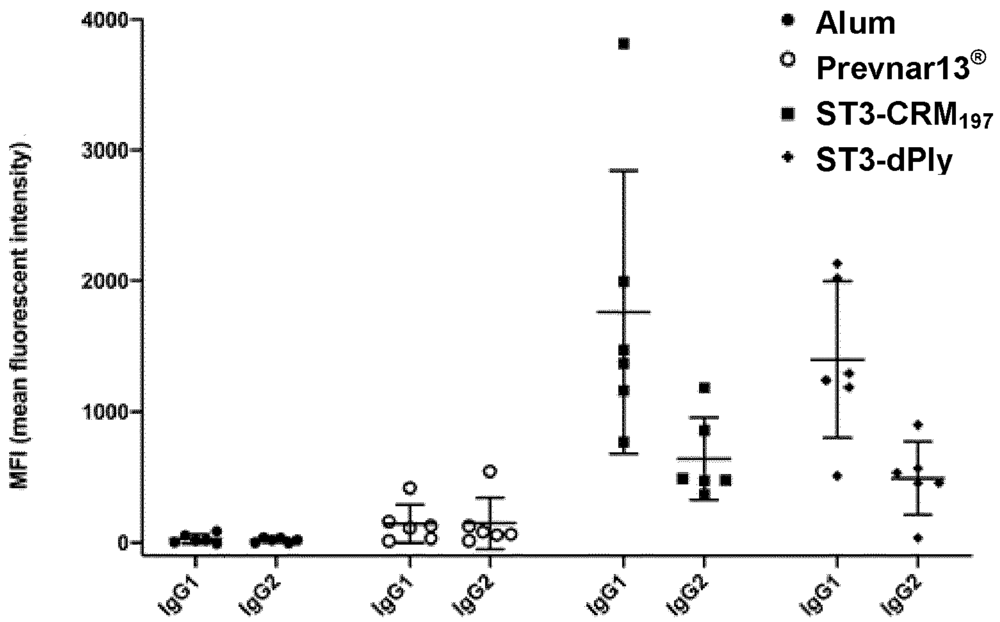
MFI (mean fluorescent intensity)
4000
3000
2000
1000
0
IgG1
IgG2
IgG1
IgG2
IgG1
IgG2
IgG1
IgG2
Alum
Prevnar13®
ST3-CRM197
ST3-dPly

b)

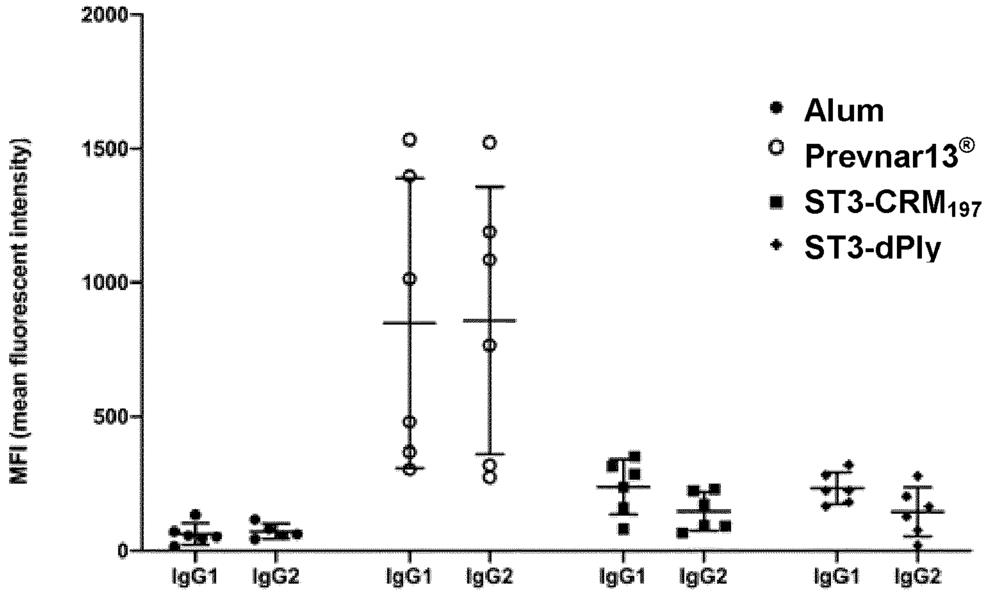
MFI (mean fluorescent intensity)
2000
1500
1000
500
0
IgG1
IgG2
IgG1
IgG2
IgG1
IgG2
IgG1
IgG2
Alum
Prevnar13®
ST3-CRM197
ST3-dPly

**Figure 11 (*continued*)**

c)

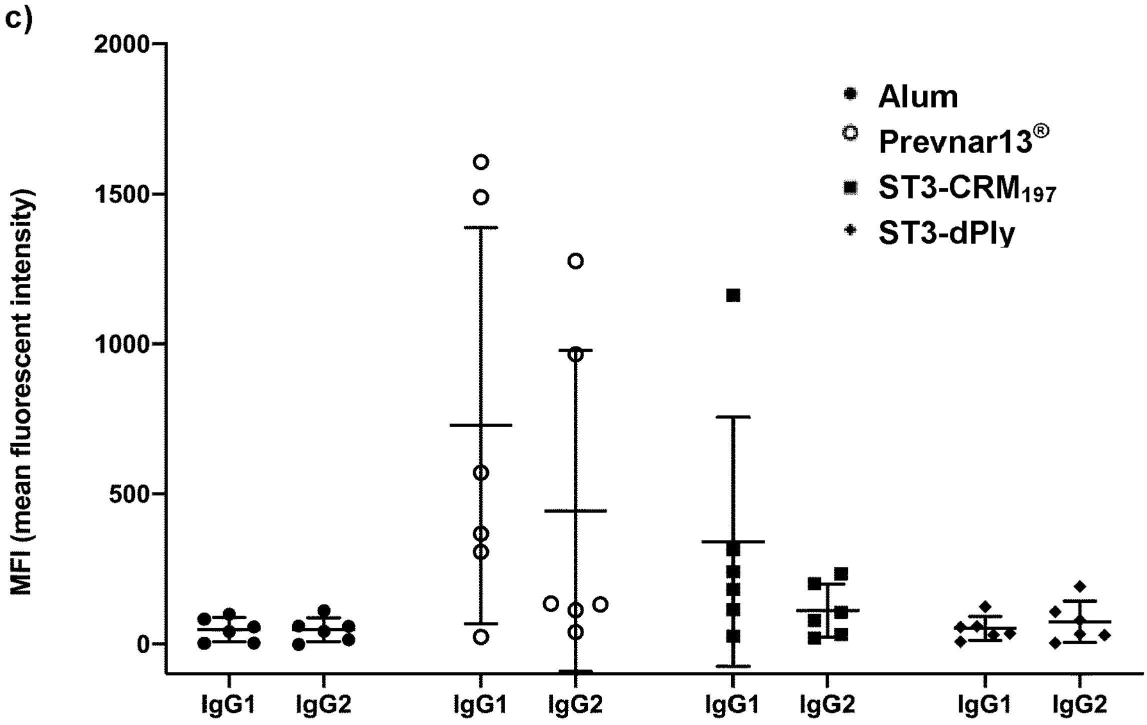


d)

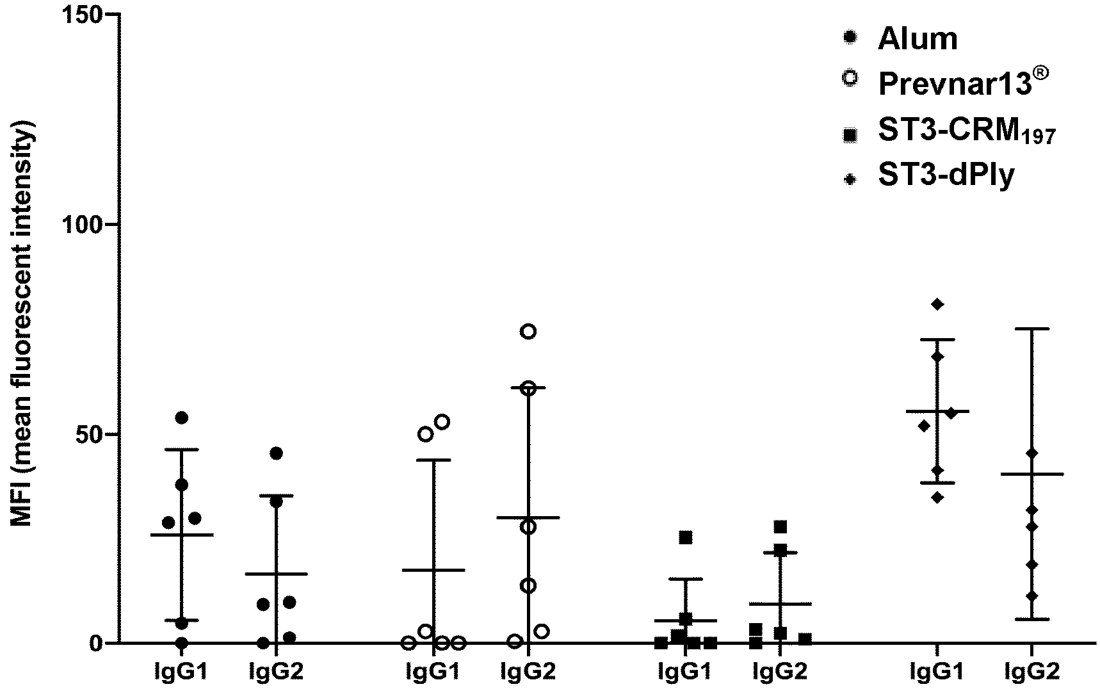

Figure 12

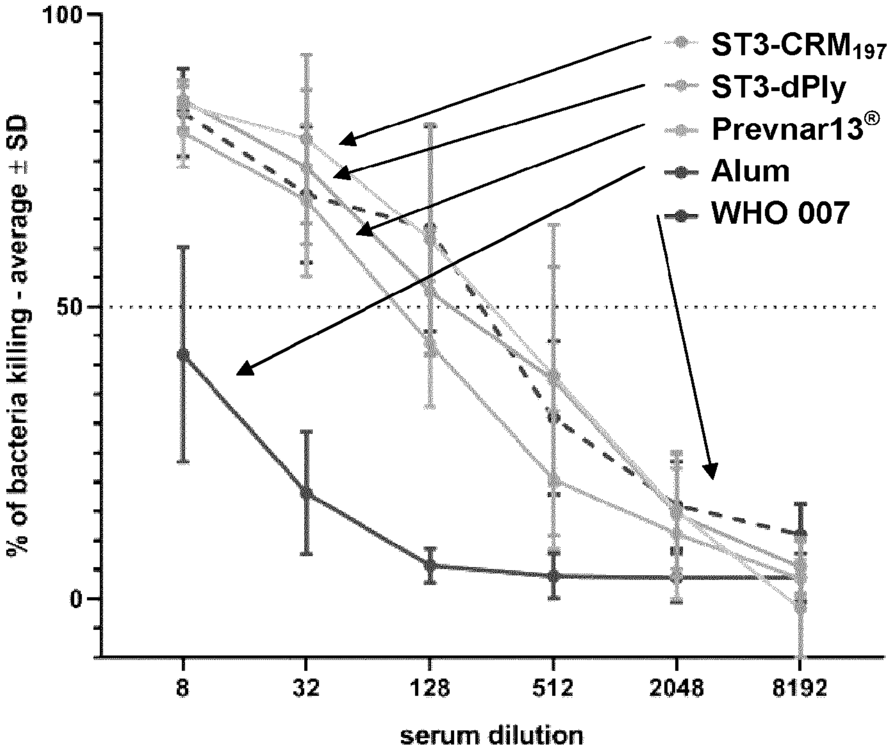
100
50
0
% of bacteria killing - average ± SD
8
32
128
512
2048
8192
serum dilution
ST3-CRM197
ST3-dPly
Prevnar13®
Alum
WHO 007

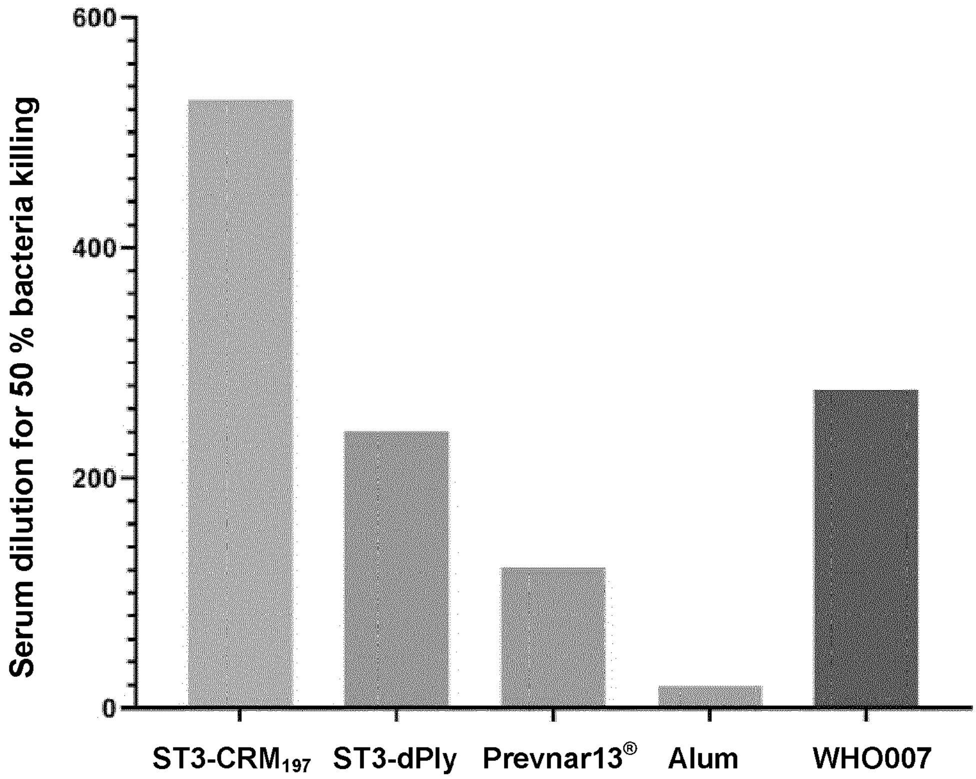
600
400
200
0
Serum dilution for 50 % bacteria killing
ST3-CRM197
ST3-dPly
Prevnar13®
Alum
WHO007

**Figure 13**

a)

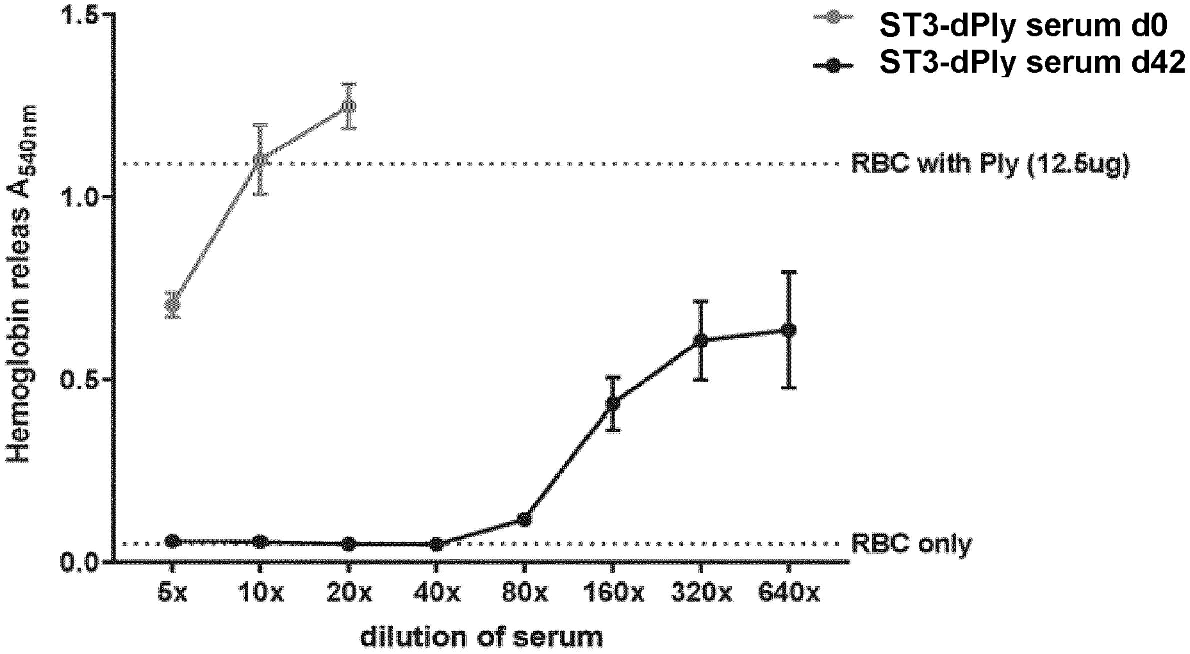
ST3-dPly serum d0
ST3-dPly serum d42
RBC with Ply (12.5ug)
RBC only
Hemoglobin releas $A_{540nm}$
1.5
1.0
0.5
0.0
5x
10x
20x
40x
80x
160x
320x
640x
dilution of serum

b)

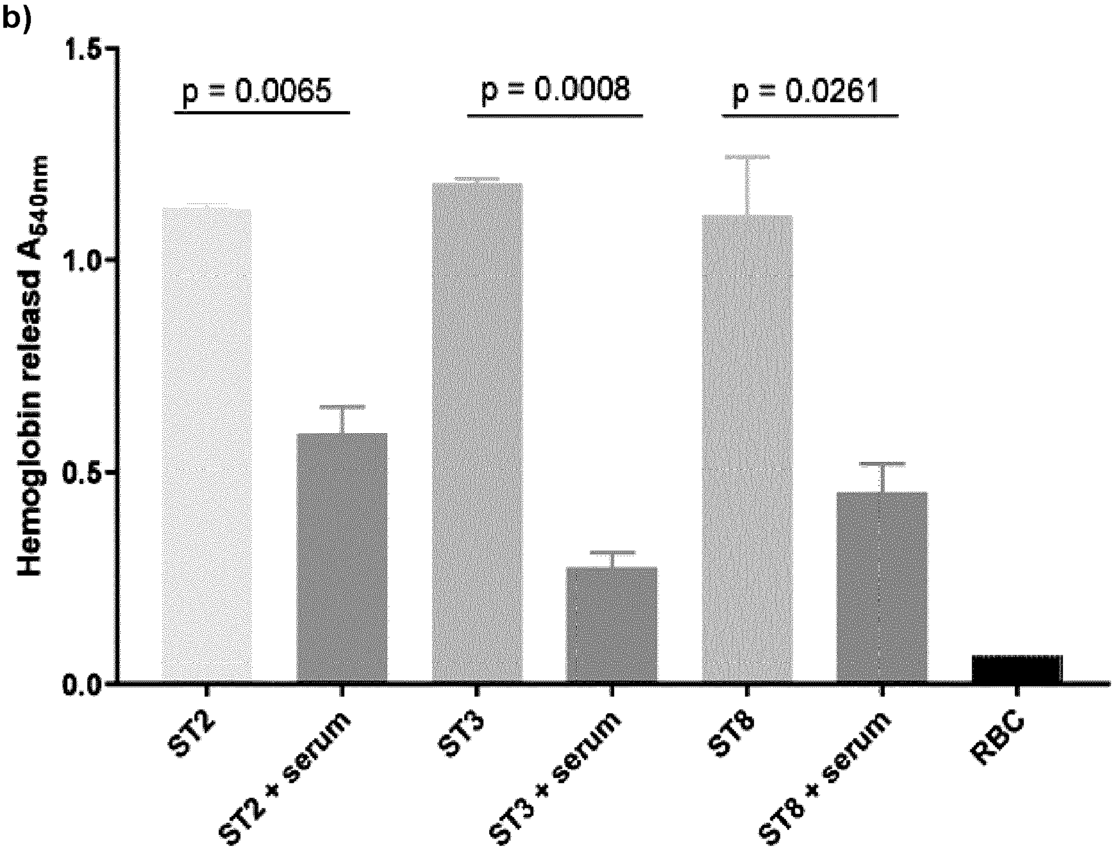
Hemoglobin releasd $A_{540nm}$
1.5
1.0
0.5
0.0
p = 0.0065
p = 0.0008
p = 0.0261
ST2
ST2 + serum
ST3
ST3 + serum
ST8
ST8 + serum
RBC

## Figure 14

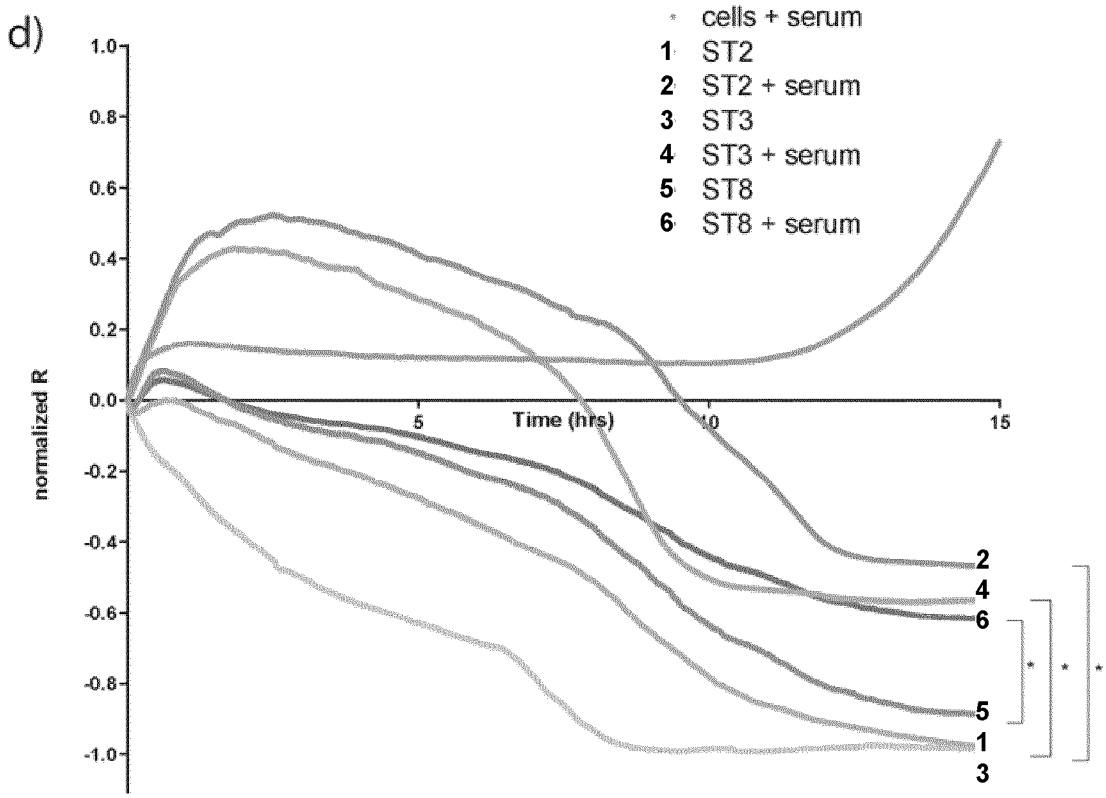


## Figure 15

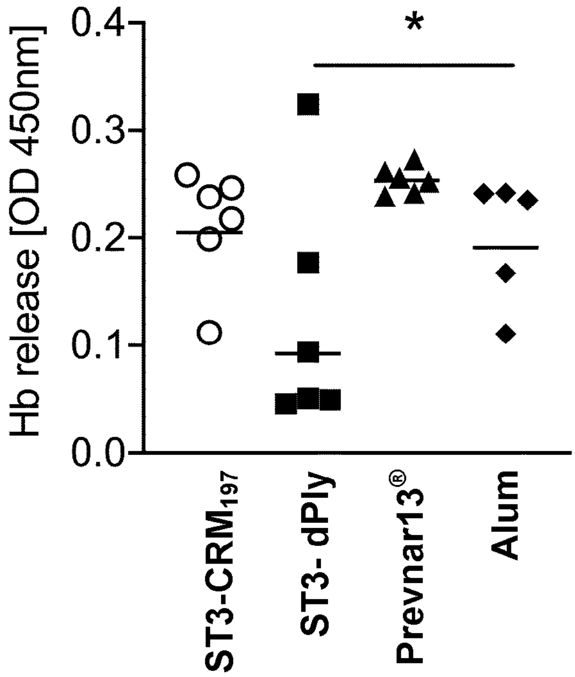

Europäisches Patentamt
European Patent Office
Office européen des brevets

# EUROPEAN SEARCH REPORT

Application Number
EP 20 17 0605

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2019/240308 A1 (MATUR RAMESH VENKAT [IN] ET AL) 8 August 2019 (2019-08-08)<br>*** para. 40, 58, claims 1, 2 *** | 1-4,7, 12-15<br>5,6,8-11 | INV.<br>A61K39/09<br>A61P11/00<br>A61P31/04<br>C07K14/315 |
| X<br>Y | US 2011/091506 A1 (GIBSON PETER [AU] ET AL) 21 April 2011 (2011-04-21)<br>*** Para. 8, 65 *** | 1-4,7,15<br>5,6,8-14 | |
| X<br>Y | US 5 565 204 A (KUO JOSEPH S-C [US] ET AL) 15 October 1996 (1996-10-15)<br>*** col. 1, 2 *** | 1,7, 12-15<br>2-6,8-11 | |
| Y | PAULINA KAPLONEK ET AL: "Improving vaccines against Streptococcus pneumoniae using synthetic glycans", PROCEEDINGS OF THE NATIONAL ACADEMY OF SCIENCES, vol. 115, no. 52, 7 December 2018 (2018-12-07), pages 13353-13358, XP055726119, ISSN: 0027-8424, DOI: 10.1073/pnas.1811862115<br>*** Figure 1; page 13355 *** | 1-15 | |
| Y | MADHU EMMADI ET AL: "A Streptococcus pneumoniae Type 2 Oligosaccharide Glycoconjugate Elicits Opsonic Antibodies and Is Protective in an Animal Model of Invasive Pneumococcal Disease", JOURNAL OF THE AMERICAN CHEMICAL SOCIETY, vol. 139, no. 41, 4 October 2017 (2017-10-04), pages 14783-14791, XP055726122, US ISSN: 0002-7863, DOI: 10.1021/jacs.7b07836<br>*** abstract; Figures 1, 2 *** | 1-15 | TECHNICAL FIELDS SEARCHED (IPC)<br>A61K<br>C12R<br>A61P<br>C07K |

The present search report has been drawn up for all claims

2

| Place of search | Date of completion of the search | Examiner |
|---|---|---|
| Munich | 1 September 2020 | Heder, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 17 0605

This annex lists the patent family members relating to the patent documents cited in the above-mentioned European search report. The members are as contained in the European Patent Office EDP file on
The European Patent Office is in no way liable for these particulars which are merely given for the purpose of information.

01-09-2020

| Patent document cited in search report | Publication date | Patent family member(s) | Publication date |
|---|---|---|---|
| US 2019240308 A1 | 08-08-2019 | AU 2016285017 A1 | 04-01-2018 |
| | | BR 112017028130 A2 | 28-08-2018 |
| | | CA 2989230 A1 | 29-12-2016 |
| | | CN 107810010 A | 16-03-2018 |
| | | EA 201890431 A1 | 29-06-2018 |
| | | EP 3313436 A2 | 02-05-2018 |
| | | JP 2018518512 A | 12-07-2018 |
| | | US 2019240308 A1 | 08-08-2019 |
| | | WO 2016207905 A2 | 29-12-2016 |
| | | ZA 201708713 B | 28-08-2019 |
| US 2011091506 A1 | 21-04-2011 | AU 2009208403 A1 | 06-08-2009 |
| | | EP 2252326 A1 | 24-11-2010 |
| | | US 2011091506 A1 | 21-04-2011 |
| | | WO 2009094730 A1 | 06-08-2009 |
| US 5565204 A | 15-10-1996 | AT 198051 T | 15-12-2000 |
| | | AU 704450 B2 | 22-04-1999 |
| | | CA 2198251 A1 | 29-02-1996 |
| | | DE 69519634 T2 | 13-06-2001 |
| | | DK 0778781 T3 | 02-01-2001 |
| | | EP 0778781 A1 | 18-06-1997 |
| | | ES 2152421 T3 | 01-02-2001 |
| | | GR 3035081 T3 | 30-03-2001 |
| | | IL 115047 A | 31-08-2005 |
| | | JP 3927233 B2 | 06-06-2007 |
| | | JP H10504717 A | 12-05-1998 |
| | | KR 970705410 A | 09-10-1997 |
| | | PT 778781 E | 30-03-2001 |
| | | US 5565204 A | 15-10-1996 |
| | | WO 9605859 A1 | 29-02-1996 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5614382 A
- WO 1990006951 A1
- WO 9315760 A
- WO 9508348 A
- WO 9629094 A
- WO 9842721 A
- US 4673574 A
- EP 0161188 A
- EP 208375 A
- EP 0477508 A
- US 200710184072 A, Hausdorff
- US 4365170 A, Jennings
- WO 9014837 A
- US 4912094 A
- US 6113918 A
- US 6207646 B
- WO 0018434 A
- WO 02098368 A
- WO 02098369 A
- WO 9313302 A
- WO 9219265 A
- WO 2015040140 A1


### Non-patent literature cited in the description


- **BENAISSA-TROUW et al.** *Infection and Immunity,* 2001, vol. 69, 4698-4701
- **MOORE MR et al.** *The Lancet. Infectious diseases,* 2015, vol. 15 (3), 301-309
- **PARAMESWARAPPA SG et al.** *Cell chemical biology,* 2016, vol. 23 (11), 1407-1416
- **JAISWAL, N. et al.** Distribution of Serotypes, Vaccine Coverage, and Antimicrobial Susceptibility Pattern of Streptococcus Pneumoniae in Children Living in SAARC Countries: A Systematic Review. *PLOS ONE,* 2014, 9
- **ASIA, LE C. et al.** Current Trend in Pneumococcal Serotype Distribution in. *J Vaccines Vaccin,* 2011
- **TAYLOR et al.** *PlosOne,* 2013, vol. 8 (4), e61300
- **BUNDLE.** *Chem. Eur. J.,* 2010, vol. 16, 3476
- **CHU C. et al.** *Infect. Immunity,* 1983, 245-256
- **E. W. MARTIN.** Remington's Pharmaceutical Sciences. Mack Publishing Co,
- *Sichuan Province in China,* 2005
- Remington's Pharmaceutical Sciences. Mack Publishing Co,
- **BUNDLE D. R. et al.** *ACS Chem. Biol.,* 2012, vol. 7, 1754
- *Angew. Chem. Int. Ed.,* 2013, vol. 52, 5858
- **HAMMERSCHMIDT.** *Infect Immun,* 1999, vol. 67 (4), 1683-1687